# EUROPEAN PATENT APPLICATION

(11) **EP 3 777 846 A1**
(43) Date of publication of application: **17.02.2021**
(21) Application number: 20193435.3
(22) Date of filing: 07.12.2016
(51) Int. Cl.: A61K 31/137, A61K 31/198, A61K 31/437, A61K 31/501, A61K 31/5377, A61K 31/573, A61P 29/00, A61P 25/02

(54) **VAP-1 INHIBITORS FOR TREATING PAIN**

(30) Priority: 07.12.2015 GB 201521547; 25.10.2016 GB 201618031
(62) Divisional of application: 16816335.0
(71) Applicant: BenevolentAI Cambridge Limited, London W1T 5HD (GB)
(72) Inventor: PRITCHARD, Martyn, London, W1T 5HD (GB); RICHARDSON, Peter, London, W1T 5HD (GB)
(74) Representative: Gill Jennings & Every LLP

(57) **Abstract**

This invention relates to the use of inhibitors of VAP-1/SSAO activity, and pharmaceutical compositions comprising the same, for the treatment of pain; and to a combined preparation comprising an inhibitor of VAP-1/SSAO activity and a steroid, and the use of the combined preparation in medicine, particularly for treatment of pain.

## Description

### FIELD OF THE INVENTION

This invention relates to the use of inhibitors of VAP-1/SSAO activity, and pharmaceutical compositions comprising the same, for the treatment of pain. The invention relates also to combined preparations comprising an inhibitor of VAP-1/SSAO activity and a steroid, and the use of the combined preparations in medicine, particularly for treatment of pain.

### BACKGROUND ART

Semicarbazide-sensitive amine oxidase (SSAO), otherwise known as Vascular Adhesion Protein-1 (VAP-1) or Amine Oxidase, Copper Containing 3 (AOC3), belongs to the copper-containing amine oxidase family of enzymes (EC.1.4.3.6). Members of this enzyme family are sensitive to inhibition by semicarbazide and utilize cupric ion and protein-derived topa quinone (TPQ) cofactor in the oxidative deamination of primary amines to aldehydes, hydrogen peroxide, and ammonia according to the following reaction:

R-CH₂-NH₂ + O₂ → R-CHO + H₂O₂ + NH₃

Known substrates for human SSAO include endogenous methylamine and aminoacetone as well as some xenobiotic amines such as benzylamine [Lyles, Int. J. Biochem. Cell Biol. 1996, 28, 259-274; Klinman, Biochim. Biophys. Acta 2003, 1647(1-2), 131-137; Matyus et al., Curr. Med. Chem. 2004, 11(10), 1285-1298; O'Sullivan et al., Neurotoxicology 2004, 25(1-2), 303-315]. In analogy with other copper-containing amine oxidases, DNA-sequence analysis and structure determination suggest that the tissue-bound human SSAO is a homodimeric glycoprotein consisting of two 90-100 kDa subunits anchored to the plasma membrane by a single N-terminal membrane spanning domain [Morris et al., J. Biol. Chem. 1997, 272, 9388-9392; Smith et al., J. Exp. Med. 1998, 188, 17-27; Airenne et al., Protein Science 2005, 14, 1964-1974; Jakobsson et al., Acta Crystallogr. D Biol. Crystallogr. 2005, 61(Pt 11), 1550-1562].

SSAO activity has been found in a variety of tissues including vascular and non-vascular smooth muscle tissue, endothelium, and adipose tissue [Lewinsohn, Braz. J. Med. Biol. Res. 1984, 17, 223-256; Nakos & Gossrau, Folia Histochem. Cytobiol. 1994, 32, 3-10; Yu et al., Biochem. Pharmacol. 1994, 47, 1055-1059; Castillo et al., Neurochem. Int. 1998, 33, 415-423; Lyles & Pino, J. Neural. Transm. Suppl. 1998, 52, 239-250; Jaakkola et al., Am. J. Pathol. 1999, 155, 1953-1965; Morin et al., J. Pharmacol. Exp. Ther. 2001, 297, 563-572; Salmi & Jalkanen, Trends Immunol. 2001, 22, 211-216]. In addition, SSAO protein is found in blood plasma and this soluble form appears to have similar properties as the tissue-bound form [Yu et al., Biochem. Pharmacol. 1994, 47, 1055-1059; Kurkijärvi et al., J. Immunol. 1998, 161, 1549-1557]. It has recently been shown that circulating human and rodent SSAO originates from the tissue-bound form [Göktürk et al., Am. J. Pathol. 2003, 163(5), 1921-1928; Abella et al., Diabetologia 2004, 47(3), 429-438; Stolen et al., Circ. Res. 2004, 95(1), 50-57], whereas in other mammals the plasma/serum SSAO is also encoded by a separate gene called AOC4 [Schwelberger, J. Neural. Transm. 2007, 114(6), 757-762].

The precise physiological role of this abundant enzyme has yet to be fully determined, but it appears that SSAO and its reaction products may have several functions in cell signalling and regulation. For example, recent findings suggest that SSAO plays a role in both GLUT4-mediated glucose uptake [Enrique-Tarancon et al., J. Biol. Chem. 1998, 273, 8025-8032; Morin et al., J. Pharmacol. Exp. Ther. 2001, 297, 563-572] and adipocyte differentiation [Fontana et al., Biochem. J. 2001, 356, 769-777; Mercier et al., Biochem. J. 2001, 358, 335-342]. In addition, SSAO has been shown to be involved in inflammatory processes where it acts as an adhesion protein for leukocytes [Salmi & Jalkanen, Trends Immunol. 2001, 22, 211-216; Salmi & Jalkanen, in "Adhesion Molecules: Functions and Inhibition" K. Ley (Ed.), 2007, pp. 237-251], and might also play a role in connective tissue matrix development and maintenance [Langford et al., Cardiovasc. Toxicol. 2002, 2(2), 141-150; Göktürk et al., Am. J. Pathol. 2003, 163(5), 1921-1928]. Moreover, a link between SSAO and angiogenesis has recently been discovered [Noda et al., FASEB J. 2008, 22(8), 2928-2935], and based on this link it is expected that inhibitors of SSAO have an anti-angiogenic effect..

Several studies in humans have demonstrated that SSAO activity in blood plasma is elevated in conditions such as congestive heart failure, diabetes mellitus, Alzheimer's disease, and inflammation [Lewinsohn, Braz. J. Med. Biol. Res. 1984, 17, 223-256; Boomsma et al., Cardiovasc. Res. 1997, 33, 387-391; Ekblom, Pharmacol. Res. 1998, 37, 87-92; Kurkijärvi et al., J. Immunol. 1998, 161, 1549-1557; Boomsma et al., Diabetologia 1999, 42, 233-237; Meszaros et al., Eur. J. Drug Metab. Pharmacokinet. 1999, 24, 299-302; Yu et al., Biochim. Biophys. Acta 2003, 1647(1-2), 193-199; Mátyus et al., Curr. Med. Chem. 2004, 11(10), 1285-1298; O'Sullivan et al., Neurotoxicology 2004, 25(1-2), 303-315; del Mar Hernandez et al., Neurosci. Lett. 2005, 384(1-2), 183-187]. The mechanisms underlying these alterations of enzyme activity are not clear. It has been suggested that reactive aldehydes and hydrogen peroxide produced by endogenous amine oxidases contribute to the progression of cardiovascular diseases, diabetic complications and Alzheimer's disease [Callingham et al., Prog. Brain Res. 1995, 106, 305-321; Ekblom, Pharmacol. Res. 1998, 37, 87-92; Yu et al., Biochim. Biophys. Acta 2003, 1647(1-2), 193-199; Jiang et al., Neuropathol Appl Neurobiol. 2008, 34(2), 194-204]. Furthermore, the enzymatic activity of SSAO is involved in the leukocyte extravasation process at sites of inflammation where SSAO has been shown to be strongly expressed on the vascular endothelium [Salmi et al., Immunity 2001, 14(3), 265-276; Salmi & Jalkanen, in "Adhesion Molecules: Functions and Inhibition" K. Ley (Ed.), 2007, pp. 237-251]. Accordingly, inhibition of SSAO has been suggested to have a therapeutic value in the prevention of diabetic complications and in inflammatory diseases [Ekblom, Pharmacol. Res. 1998, 37, 87-92; Salmi et al., Immunity 2001, 14(3), 265-276; Salter-Cid et al., J. Pharmacol. Exp. Ther. 2005, 315(2), 553-562].

WO2007/146188 teaches that blocking SSAO activity inhibits leucocyte recruitment, reduces the inflammatory response, and is expected to be beneficial in prevention and treatment of seizures, for example, in epilepsy.

O'Rourke et al (J Neural Transm. 2007;114(6):845-9) examined the potential of SSAO inhibitors in neurological diseases, having previously demonstrated the efficacy of SSAO inhibition in a rat model of stroke. An SSAO inhibitor is tested on relapsing-remitting experimental autoimmune encephalomyelitis (EAE), a mouse model that shares many characteristics with human multiple sclerosis. The data demonstrates the potential clinical benefit of small molecule anti-SSAO therapy in this model and therefore in treatment of human multiple sclerosis.

SSAO knockout animals are phenotypically overtly normal but exhibit a marked decrease in the inflammatory responses evoked in response to various inflammatory stimuli [Stolen et al., Immunity 2005, 22(1), 105-115]. In addition, antagonism of its function in wild type animals in multiple animal models of human disease (e.g. carrageenan-induced paw inflammation, oxazolone-induced colitis, lipopolysaccharide-induced lung inflammation, collagen-induced arthritis, endotoxin-induced uveitis) by the use of antibodies and/or small molecules has been shown to be protective in decreasing the leukocyte infiltration, reducing the severity of the disease phenotype and reducing levels of inflammatory cytokines and chemokines [Kirton et al., Eur. J. Immunol. 2005, 35(11), 3119-3130; Salter-Cid et al., J. Pharmacol. Exp. Ther. 2005, 315(2), 553-562; McDonald et al., Annual Reports in Medicinal Chemistry 2007, 42, 229-243; Salmi & Jalkanen, in "Adhesion Molecules: Functions and Inhibition" K. Ley (Ed.), 2007, pp. 237-251; Noda et al., FASEB J. 2008 22(4), 1094-1103; Noda et al., FASEB J. 2008, 22(8), 2928-2935]. This anti-inflammatory protection seems to be afforded across a wide range of inflammatory models all with independent causative mechanisms, rather than being restricted to one particular disease or disease model. This would suggest that SSAO may be a key nodal point for the regulation of the inflammatory response, and it seems therefore likely that SSAO inhibitors may be effective anti-inflammatory drugs in a wide range of human diseases.

Fibrosis can result from chronic tissue inflammation when the resolution of the inflammation is partly abrogated by the chronic nature of the inflammatory stimulus. The result can be inappropriate repair of the tissue with excessive extracellular matrix deposition (including collagen) with tissue scarring. This is a consequence of myofibroblast activation by stimuli including fibronectin and reactive oxygen species as well as growth factors such as transforming growth factor-β-1 (TGFβ-1), insulin-like growth factor-I (IGF-I), platelet-derived growth factor (PDGF) and connective tissue growth factor (CTGF) resulting in increased production of collagen, elastin, hyaluronan, glycoproteins and proteoglycans. In addition the activity of invading macrophages plays a crucial part in regulating the repair and fibrotic processes.

VAP-1 has also been implicated in the progression and maintenance of fibrotic diseases especially in the liver. Weston and Adams (J Neural Transm. 2011, 118(7), 1055-64) have summarised the experimental data implicating VAP-1 in liver fibrosis. Weston et al (EASL Poster 2010) showed highly increased expression of VAP-1 in human fibrotic liver, particularly associated with the activated myofibroblasts and collagen fibrils. This anatomical association with fibrosis was consistent with the observation that blockade of VAP-1 accelerated the resolution of carbon tetrachloride induced fibrosis, and suggested a role for the VAP-1/SSAO enzyme product H2O2 in the activation of the myofibroblasts. The same authors also showed that the pro-fibrotic growth factor TGFβ increased the expression of VAP-1 in liver cells by approximately 50-fold. In addition VAP-1 has been implicated in inflammation of the lung (e.g. Singh et al., 2003, Virchows Arch 442:491-495) suggesting that VAP-1 blockers would reduce lung inflammation and thus be of benefit to the treatment of cystic fibrosis by treating both the pro-fibrotic and pro-inflammatory aspects of the disease.

SSAO (VAP-1) is up regulated in gastric cancer and has been identified in the tumour vasculature of human melanoma, hepatoma and head and neck tumours (Yoong KF, McNab G, Hubscher SG, Adams DH. (1998), J Immunol 160, 3978-88.; Irjala H, Salmi M, Alanen K, Gre'nman R, Jalkanen S (2001), Immunol. 166, 6937-6943; Forster-Horvath C, Dome B, Paku S, et al. (2004), Melanoma Res. 14, 135-40.). One report (Marttila-Ichihara F, Castermans K, Auvinen K, Oude Egbrink MG, Jalkanen S, Griffioen AW, Salmi M. (2010), Jlmmunol. 184, 3164-3173.) has shown that mice bearing enzymically inactive VAP-1 grow melanomas more slowly, and have reduced tumour blood vessel number and diameter. The reduced growth of these tumours was also reflected in the reduced (by 60-70%) infiltration of myeloid suppressor cells. Encouragingly VAP-1 deficiency had no effect on vessel or lymph formation in normal tissue.

For the above reasons, it is expected that inhibition of SSAO will reduce the levels of pro-inflammatory enzyme products (aldehydes, hydrogen peroxide and ammonia) whilst also decreasing the adhesive capacity of immune cells and correspondingly their activation and final extra-vasation. Diseases where such an activity is expected to be therapeutically beneficial include all diseases where immune cells play a prominent role in the initiation, maintenance or resolution of the pathology, such inflammatory diseases and immune/autoimmune diseases. Examples of such diseases include multiple sclerosis, arthritis and vasculitis.

### SUMMARY OF THE INVENTION

According to a first aspect of the invention, the applicants have found that compounds having VAP-1 inhibitory activity are surprisingly effective in the treatment of pain, including inflammatory and neuropathic pain. Surprisingly, the applicants have found that compounds having VAP-1 inhibitory activity are effective in treating pain even when there is no detectible reduction in inflammation. In other words, compounds having VAP-1 inhibitory activity are effective in treating pain without necessarily reducing inflammation. Alternatively, compounds having VAP-1 inhibitory activity provide treatment of pain, or relief from pain, for a treated patient on a timescale that is much shorter than the time typically required for a measurable or perceptible reduction of inflammation

Co-pending UK patent application number GB1507048.5, the content of which is hereby incorporated by reference in its entirety, already claims the use of the VAP-1 inhibitor (3S)-Tetrahydrofuran-3-yl (4S)-4-isopropyl-1,4,6,7-tetrahydro-5H-imidazo[4,5-c]pyridine-5-carboxylate and hydrates and pharmaceutically acceptable salts thereof for the treatment of pain. Therefore the use of (3S)-Tetrahydrofuran-3-yl (4S)-4-isopropyl-1,4,6,7-tetrahydro-5H-imidazo[4,5-c]pyridine-5-carboxylate and hydrates and pharmaceutically acceptable salts thereof in the treatment of pain may be excluded from the scope of claims directed to the first aspect of the present invention. However, in the broadest sense, the present invention includes the use of compounds having VAP-1 inhibitory activity in the treatment of pain, including inflammatory pain and neuropathic pain.

According to a second aspect of the invention, the applicants have found that a combined preparation of a VAP-1 inhibitor and a steroid is surprisingly effective for the treatment of pain, in particular inflammatory and neuropathic pain.

According to a third aspect of the invention, the applicants have made available a combined preparation comprising the VAP-1 inhibitor (3*S*)-Tetrahydrofuran-3-yl (4*S*)-4-isopropyl-1,4,6,7-tetrahydro-5H-imidazo[4,5-c]pyridine-5-carboxylate and hydrates and pharmaceutically acceptable salts thereof and a steroid. This combined preparation is expected to be surprisingly effective as a medicament, particularly for the treatment of pain, including inflammatory and neuropathic pain.

### BRIEF DESCRIPTION OF THE FIGURES

Embodiments of the invention are described below, with reference to the accompanying drawings in which:

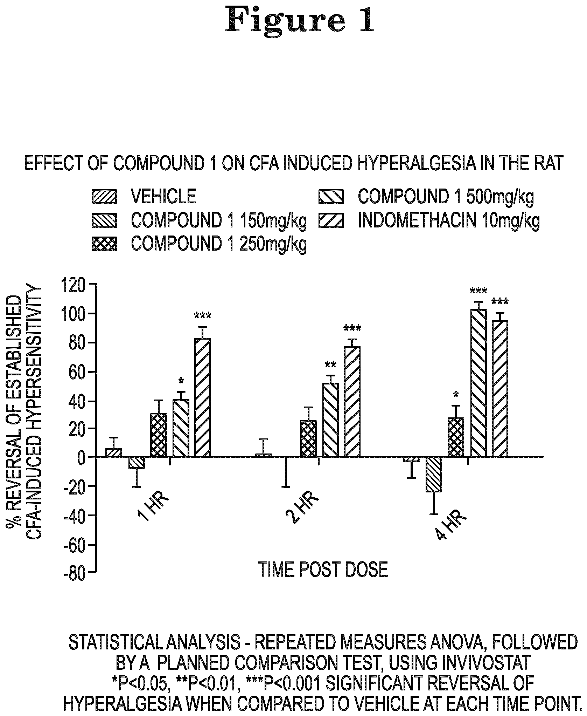

**Figure 1** shows the effects of (3*S*)-Tetrahydrofuran-3-yl (4*S*)-4-isopropyl-1,4,6,7-tetrahydro-5H-imidazo[4,5-c]pyridine-5-carboxylate (referred to as Compound 1) in the CFA induced thermal hyperalgesia (pain) model (left to right - vehicle; 150mg/kg; 250mg/kg; 500mg/kg; 10mg/kg indomethacin;

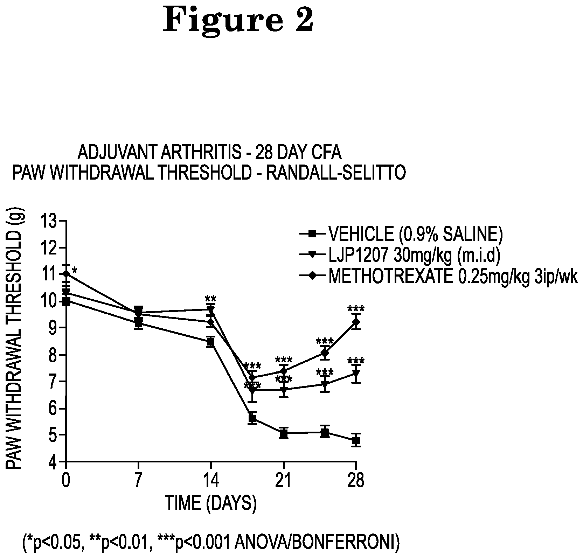

**Figure 2** shows the effects of LJP1207 on a CFA-induced arthritis model, which is a well-established pain model;

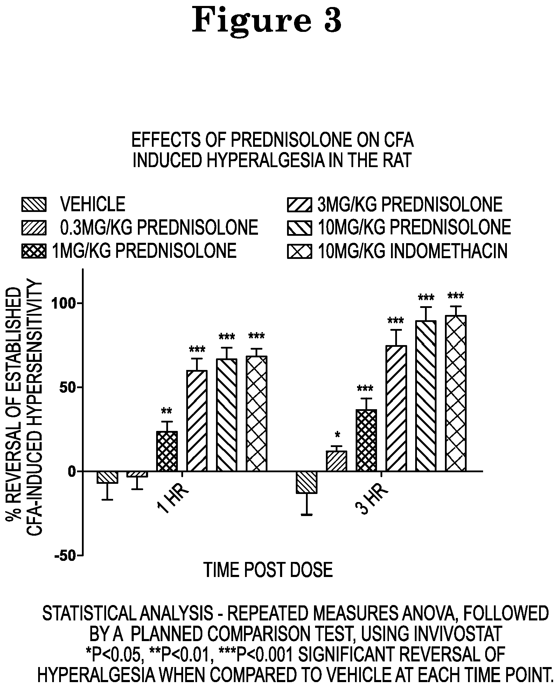

**Figure 3** shows the effects of prednisolone on CFA induced hyperalgesia in the rat at 1 hour and three hours post dose (left to right - vehicle; 0.3mg/kg prednisolone; 1mg/kg prednisolone; 3mg/kg prednisolone; 10mg/kg prednisolone; 10mg/kg indomethacin);

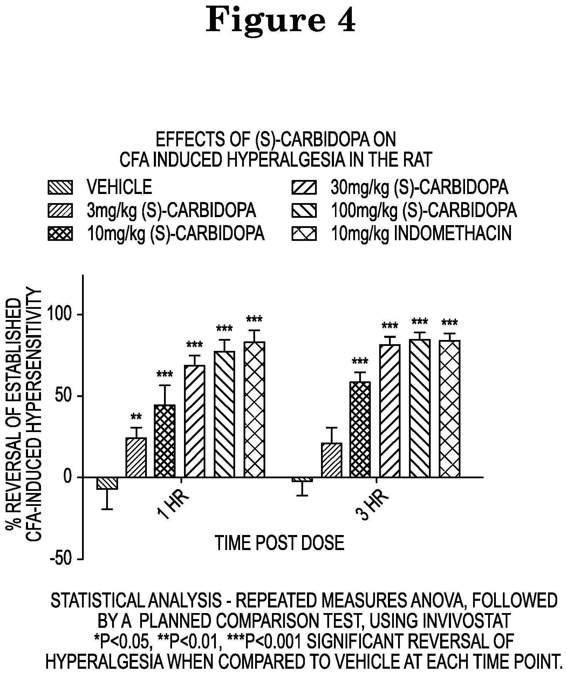

**Figure 4** shows the effects of (*S*)-carbidopa on CFA induced hyperalgesia in the rat at one hour and three hours post dose (left to right - vehicle; 3mg/kg (*S*)-carbidopa; 10mg/kg (*S*)-carbidopa; 30mg/kg (*S*)-carbidopa; 100mg/kg (*S*)-carbidopa; 10mg/kg indomethacin); and

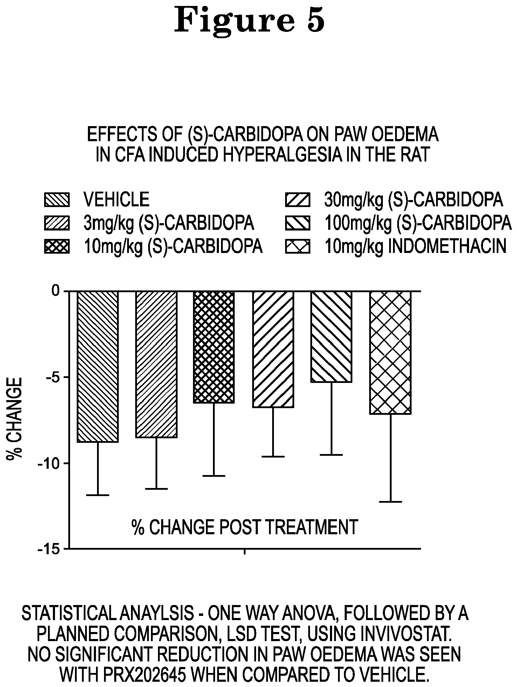

**Figure 5** shows the effects of (*S*)-carbidopa on paw oedema in CFA-induced hyperalgesia in the rat at 3 hours hour post dose (left to right - vehicle/vehicle; 3mg/kg (*S*)-carbidopa/vehicle; 10mg/kg (*S*)-carbidopa/vehicle; 30mg/kg (*S*)-carbidopa/vehicle; 100mg/kg (*S*)-carbidopa/vehicle; 10mg/kg (*S*)-indomethacin/vehicle,).

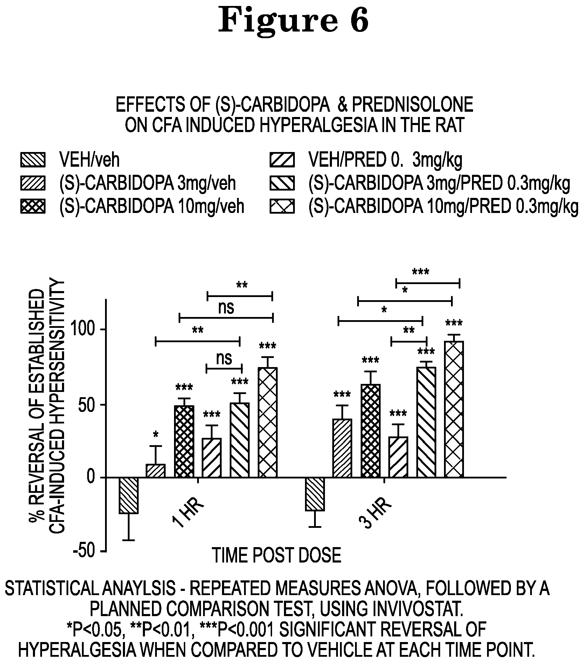

**Figure 6** shows the effects of (*S*)-carbidopa and prednisolone on CFA-induced hyperalgesia in the rat at one hour and three hours post dose (left to right - vehicle/vehicle; 3mg/kg (*S*)-carbidopa/vehicle; 10mg/kg (*S*)-carbidopa/vehicle; vehicle/0.3mg/kg prednisolone; 3mg/kg (*S)*-carbidopa/0.3mg/kg prednisolone, 10mg/kg (*S*)-carbidopa/0.3mg/kg prednisolone).

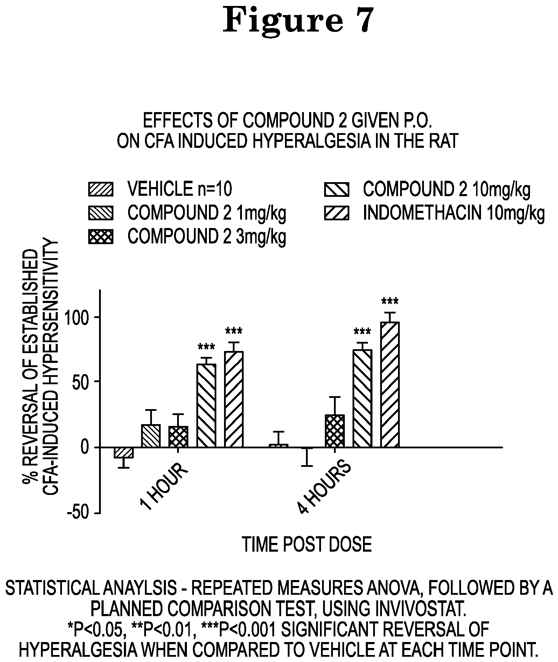

**Figure 7** shows the effect of 1-(4-{5-[3-(4-Fluorophenyl)-3H-imidazo[4,5-c]pyridin-2-yl]pyridin-2-yl}piperazin-1-yl)ethan-1-one (referred to as Compound 2) on CFA-induced hyperalgesia in the rat at one hour and four hours post dose (left to right - vehicle/vehicle; 1 mg/kg Compound 2/vehicle; 3 mg/kg Compound 2/vehicle; 10 mg/kg Compound 2/vehicle; 10 mg/kg Indomethacin/vehicle).

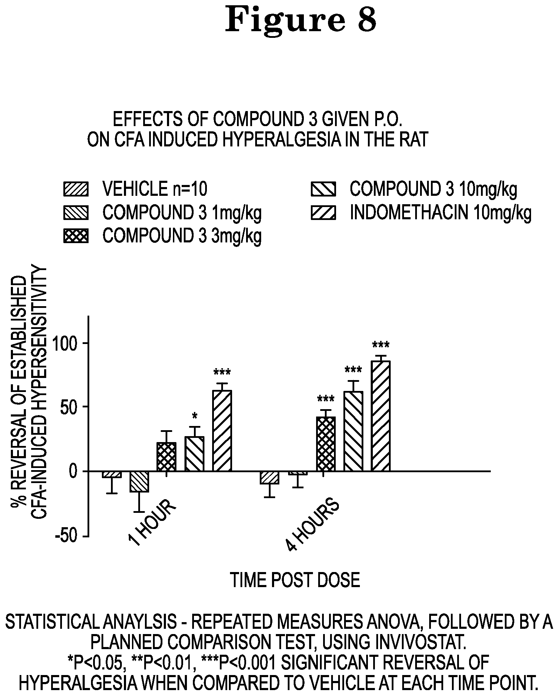

**Figure 8** shows the effect of 1-{5-[3-(4-Fluorophenyl)-3H-imidazo[4,5-c]pyridin-2-yl]pyridin-2-yl}-4-methanesulfonylpiperazine (referred to as Compound 3) on CFA-induced hyperalgesia in the rat at one hour and four hours post dose (left to right - vehicle/vehicle; 1 mg/kg Compound 3/vehicle; 3 mg/kg Compound 3/vehicle; 10 mg/kg Compound 3/vehicle; 10 mg/kg Indomethacin/vehicle).

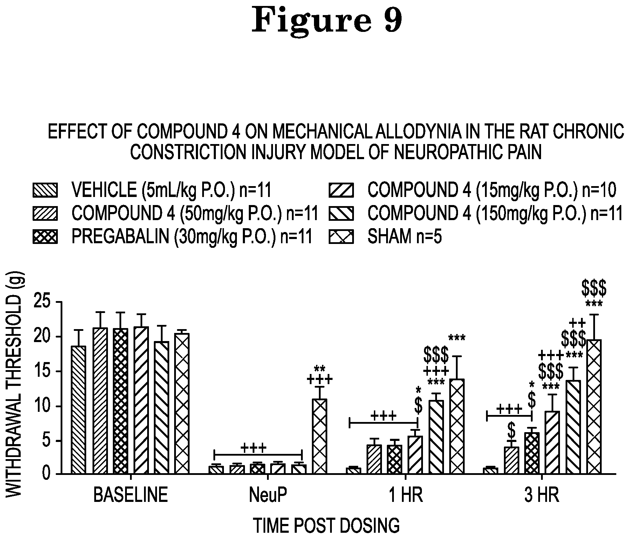

**Figure 9** shows the effect of 4-{5-[3-(4-Fluorophenyl)-3H-imidazo[4,5-c]pyridin-2-yl]pyridin-2-yl}morpholine (referred to as Compound 4) on mechanical allodynia in the rat chronic constriction injury (CCI) model of neuropathic pain (left to right - vehicle/vehicle; 15 mg/kg Compound 4/vehicle; 50 mg/kg Compound 4/vehicle; 150 mg/kg Compound 4/vehicle; 30 mg/kg Pregabalin/vehicle; sham).

### DETAILED DESCRIPTION OF THE INVENTION

### Definitions

As used herein, the terms "treatment," "treating," "treat" and the like, refer to obtaining a desired pharmacologic and/or physiologic effect. In the case of the treatment of pain, the effect can be prophylactic in terms of completely or partially preventing pain or a symptom thereof and/or can be therapeutic in terms of a partial or complete cure for pain and/or an adverse effect attributable to the disease. "Treatment," as used herein, covers any treatment of pain in a mammal, particularly in a human, and includes: (a) preventing the disease from occurring in a subject which can be predisposed to the disease but has not yet been diagnosed as having it; (b) inhibiting the disease, i.e., arresting its development; and (c) relieving the disease, i.e., causing regression of the disease.

An "effective amount" of a VAP-1 inhibitor and/or steroid refers to the amount of a VAP-1 inhibitor and/or steroid that, when administered to a mammal or other subject for treating a disease or condition, is sufficient to effect such treatment for the disease or condition. The "effective amount" will vary depending on the VAP-1 inhibitor and/or steroid, the disease and its severity and the age, weight, etc., of the subject to be treated. The therapeutic effect may be objective (i.e., measurable by some test or marker) or subjective (i.e., subject gives an indication of or feels an effect).

The term "VAP-1 inhibitor" or "VAP-1 inhibitor compound" includes both non-biological small molecule inhibitors of VAP-1 and biological inhibitors of VAP-1, including but not limited to RNA, antibodies, polypeptidic or proteinaceous inhibitors of VAP-1.

For present purposes, a "VAP-1 inhibitor" or "VAP-1 inhibitor compound" is one which has an IC50 value of less than 1000nM in the VAP-1 Assay described below.

"Pharmaceutically acceptable" means being useful in preparing a pharmaceutical composition that is generally safe, non-toxic and neither biologically nor otherwise undesirable and includes being useful for veterinary use as well as human pharmaceutical use. Suitable pharmaceutically acceptable salts include, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates. For a review on salts, see Handbook of Pharmaceutical Salts: Properties, Selection, and Use by Stahl and Wermuth (Wiley-VCH, Weinheim, Germany, 2002). Pharmaceutically acceptable salts may also be formed with bases. Such salts include salts derived from inorganic or organic bases, for example alkali metal salts such as magnesium or calcium salts, and organic amine salts such as morpholine, piperidine, dimethylamine or diethylamine salts.

The term "pain" as used herein includes inflammatory pain and neuropathic pain. In an embodiment, the pain is inflammatory pain. In an embodiment, the term "pain" excludes neuropathic pain.

### VAP-1 Inhibitors

In one aspect of the invention, a suitable VAP-1 inhibitor is a non-biological small molecule inhibitor of VAP-1. Small molecules of different structural classes have previously been disclosed as VAP-1 inhibitors, for example in WO 02/38153 (tetrahydroimidazo[4,5-c]pyridine derivatives), in WO 03/006003 (2-indanylhydrazine derivatives), in WO 2005/014530 (allylhydrazine and hydroxylamine (aminooxy) compounds) and in WO 2007/120528 (allylamino compounds), WO2011034078 (N-[3-(heterocyclyl or phenyl)benzyl]-2-aminoglycinamides), and WO2012120195 (Pyridazinones), and WO2012124696 (Guanidines), and Bioorganic & Medicinal Chemistry (2013), 21(13), 3873-3881 (1H-imidazol-2-amine derivatives), and Bioorganic & Medicinal Chemistry (2013), 21(5), 1219-1233 (Thiazoles).

Many further small molecule VAP-1 inhibitors are known, for example, haloallyl amines of WO2009066152; imidazopyridines of WO2010064020; dihydralazine (WO2010015870); pyrazolo[4,3-c]pyridines of WO2010031791; 4,5,6,7-tetrahydroimidazo[4,5-c]pyridines of US2002198189, WO0238153 and WO2010031789; oximes of WO2010029379; allyl hydrazine, hydroxylamine and other compounds of US2005096360, WO2006094201 and WO2005014530; amine, amide and allylamino compounds of WO2007120528, US2007078157, WO2005082343 and WO2009055002; hydroxamic acids of WO2006013209; vitamin B1, vitamin B1 derivatives and vitamin B1 precursors of WO2008025870; 2,3,4,6,8-pentamethoxyl-dibenzofuran (CN100486971); compounds of US2007066646; aminoglycosides of WO2005063261; carbocyclic hydrazino compounds of WO03006003; hydrazono compounds of US2004106654 and WO0202090; haloallylamines such as MDL72161A, MDL72274A and MDL72964A (mofegiline, (E)-4-fluoro-beta-fluoromethylene benzene butanamine hydrochloride, (E)-2-(4-fluorophenethyl)-3-fluoroallylamine hydrochloride) as in WO9323023, Lyles et al, Biochem. Pharmacol., 1987, 2847 and McDonald et al, J. Med. Chem., 1985, 186; thiazoles of WO2004087138, WO2004067521, WO2005089755, WO2006011631 and WO2006028269; semicarbazide and hydrazines (e.g. phenylhydrazine, phenelzine, carbazine and hydrazaline) as in McDonald et al, Annual reports in medicinal chemistry, 42, 229-243, 2007; hydrazines of WO2004104191 and WO2002002541; 1,3,4-oxadiazine compounds of WO200202541; hydrazino alcohol derivatives of WO2005080319; propargylamines of Sayre et al, Biochem., Biophys., Res. Commun, 2003, 788, Sayre et al, Bioorg. Med. Chem., 2006, 1444 and Sayre et al, Eur. J. Biochem., 2002, 3645; peptides as in Yegutkin, Eur. J. Immunol., 2004, 2276; dihydropyrroles of US20060025438 and Sayre et al, J. Am. Chem. Soc., 2002, 12135; proline amide derivatives of Sayre et al, Bioorg. Med. Chem., 2007, 1868; benzene and thiophene derivatives of WO2009145360 and WO 2009096609; thiazoles of US20040259923; and also includes molecules such as 5-hydroxytryptamine, 3-bromopropylamine, N-(phenyl-allyl)-hydrazine HCI (LJP-1207), 2-hydrazinopyridine, MDL-72274 ((E)-2-phenyl-3-chloroallylamine hydrochloride), MDL-72214 (2-phenylallylamine), MDL-72145, MDL-72161, mexiletine, isoniazid, imipramine, maprotiline, zimeldine, nomifensine, azoprocarbazine, monomethylhydrazine, dl-alphamethyltryptamine, dl-alphamethylbenzylamine, MD780236 (Dostert et al, J. Pharmacy & Pharmacol., 1984, 782), Z-3-Fluoro-2-(4-methoxybenzyl)allylamine hydrochloride (LJP-1586) (O'Rourke et al, JPET, 2008, 867), 2-(dimethyl(2-phenylethyl)silyl)methanamine, cuprozine, alkylamino derivatives of 4-aminomethylpyridine (Bertini et al, J. Med. Chem., 2005, 664), (1S,2S)-2-(1-methylhydrazino)-1-indanol (BTT-2052) (Marttila-Ichihara et al, JI, 2010, 2741), RTU-1096, kynuramine, carbidopa, compounds of WO2013163675, compounds of WO2009051223, ASP8232 and PXS-4728A.

In another aspect of the invention, the VAP-1 inhibitor is a biological inhibitor of VAP-1. Biological inhibitors of VAP-1 include but are not limited to antibodies to VAP-1, RNAi, siRNA (examples of siRNAs suitable for targeting VAP-1 are described, for example, in WO2006134203), anti-sense oligonucleotides, anti-sense peptidyl nucleic acids, and aptamers. Examples of VAP-1 antibodies include but are not limited to anti-VAP-1 neutralizing antibody (available, for example, from R&D systems, Minneapolis, MN, catalogue numbers. AF3957, MAB39571 and MAB3957; Everest Biotech , Oxford, UK, catalogue number EB07582; and antibodies identified in WO2008129124, WO2003093319 and Koskinen et al, Blood, 2004, 3388, Arvilommi et al, Eur. J. Immunol., 1996, 825, Salmi et al, J. Exp. Med., 1993, 2255 and Kirten et al, Eur. J. Immunol., 2005, 3119. A further example of a VAP-1 antibody is BTT1023 (Biotie Therapies), a fully human anti-VAP-1 antibody.

The VAP-1 inhibitors disclosed specifically or generically in the above publications are expected to have utility in the treatment of pain according to the first aspect of the present invention. The VAP-1 inhibitors disclosed specifically or generically in the above publications are expected to have utility in a combined preparation with a steroid in the treatment of pain according to the second aspect of the present invention. In the practice of the present invention, a combination of two or more VAP-1 inhibitors may also be employed.

Provided below are further specific Examples of VAP-1 inhibitor compounds suitable for use in the first and second aspects of the present invention. Any pharmaceutically acceptable salt form of the Examples is suitable for use in the present invention. Specific examples of inhibitors of VAP-1 include the compounds speficially disclosed as Examples in WO 2010/031789, namely:

| | |
|---|---|
| 2,2,2-Trichloroethyl 4-isopropyl-1,4,6,7-tetrahydro-5H-imidazo[4,5-c]pyridine-5-carboxylate | 3-Chlorobenzyl 4-isopropyl-1,4,6,7-tetrahydro-5H-imidazo[4,5-c]pyridine-5-carboxylate |
| 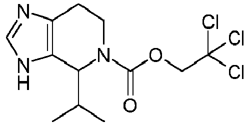 | 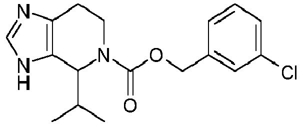 |
| | 4-Chlorobenzyl 4-isopropyl-1,4,6,7-tetrahydro-5H-imidazo[4,5-c]pyridine-5-carboxylate |
| 2-Chloro-2,2-difluoroethyl 4-isopropyl-1,4,6,7-tetrahydro-5H-imidazo[4,5-c]pyridine-5-carboxylate | 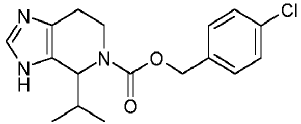 |
| 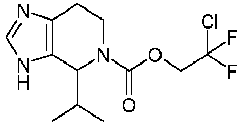 | Pyridin-2-ylmethyl 4-isopropyl-1,4,6,7-tetrahydro-5H-imidazo[4,5-c]pyridine-5-carboxylate |
| Benzyl 4-isopropyl-1,4,6,7-tetrahydro-5H-imidazo[4,5-c]pyridine-5-carboxylate | 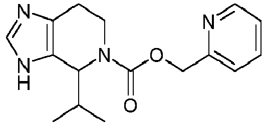 |
| 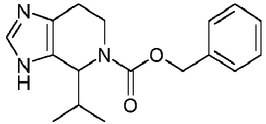 | |
| Pyridin-3-ylmethyl 4-isopropyl-1,4,6,7-tetrahydro-5H-imidazo[4,5-c]pyridine-5-carboxylate | 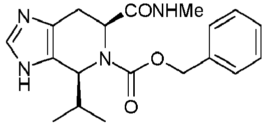 |
| 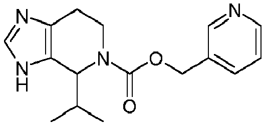 | 5-Benzyl 6-methyl (4S,6S)-4-isopropyl-1,4,6,7-tetrahydro-5H-imidazo[4,5-c]pyridine-5,6-dicarboxylate |
| Pyridin-4-ylmethyl 4-isopropyl-1,4,6,7-tetrahydro-5H-imidazo[4,5-c]pyridine-5-carboxylate | 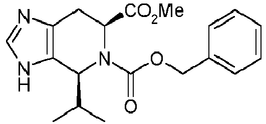 |
| 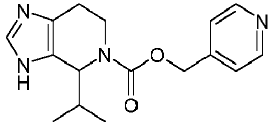 | 2-Phenoxyethyl 4-isopropyl-1,4,6,7-tetrahydro-5H-imidazo[4,5-c]pyridine-5-carboxylate |
| | 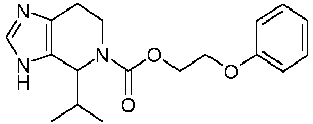 |
| (5-Chloropyridin-2-yl)methyl 4-isopropyl-1,4,6,7-tetrahydro-5H-imidazo[4,5-c]pyridine-5-carboxylate | 2-(4-Chlorophenoxy)ethyl 4-isopropyl-1,4,6,7-tetrahydro-5H-imidazo[4,5-c]pyrid ine-5-carboxylate |
| 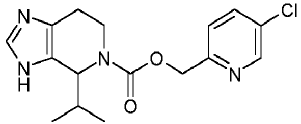 | 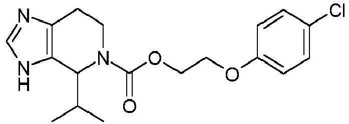 |
| Pyrazin-2-ylmethyl 4-isopropyl-1,4,6,7-tetrahydro-5H-imidazo[4,5-c]pyridine-5-carboxylate | (3S)-Tetrahydrofuran-3-yl (4S)-4-isopropyl-1,4,6,7-tetrahydro-5H-imidazo[4,5-c]pyridine-5-carboxylate |
| 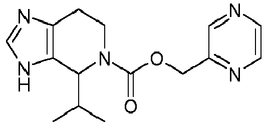 | 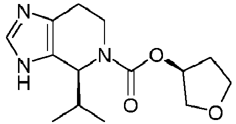 |
| Benzyl (4S,6S)-6-(aminocarbonyl)-4-isopropyl-1,4,6,7-tetrahydro-5H-imidazo[4,5-c]pyridine-5-carboxylate | Tetrahydrofuran-3-ylmethyl 4-isopropyl-1,4,6,7-tetrahydro-5H-imidazo[4,5-c]pyridine-5-carboxylate |
| 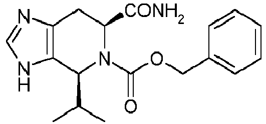 | 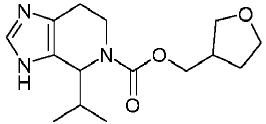 |
| Benzyl (4S,6S)-4-isopropyl-6-[(methylamino)carbonyl]-1,4,6,7-tetrahydro-5H-imidazo[4,5-c]pyridine-5-carboxylate | (3-Methyloxetan-3-yl)methyl 4-isopropyl-1,4,6,7-tetrahydro-5H-imidazo[4,5-c]pyridine-5-carboxylate |
| 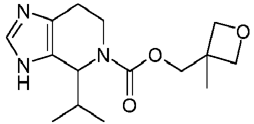 | 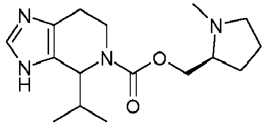 |
| 2-(Dimethylamino)ethyl 4-isopropyl-1,4,6,7-tetrahydro-5H-imidazo[4,5-c]pyridine-5-carboxylate | (3R)-1-methylpyrrolidin-3-yl 4-isopropyl-1,4,6,7-tetrahydro-5H-imidazo[4,5-c]pyridine-5-carboxylate |
| 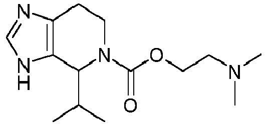 | 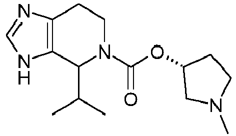 |
| (2R)-Tetrahydrofuran-2-ylmethyl 4-isopropyl-1,4,6,7-tetrahydro-5H-imidazo[4,5-c]pyridine-5-carboxylate | Oxetan-2-ylmethyl 4-isopropyl-1,4,6,7-tetrahydro-5H-imidazo[4,5-c]pyridine-5-carboxylate |
| 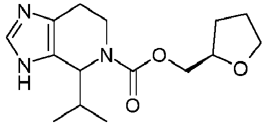 | 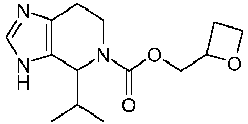 |
| 1,3-Thiazol-2-ylmethyl 4-isopropyl-1,4,6,7-tetrahydro-5H-imidazo[4,5-c]pyridine-5-carboxylate | 2-(Pyridin-3-yloxy)ethyl 4-isopropyl-1,4,6,7-tetrahydro-5H-imidazo[4,5-c]pyridine-5-carboxylate |
| 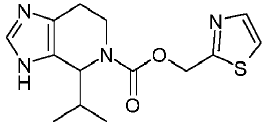 | 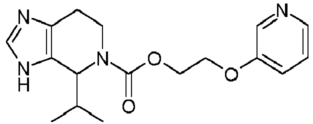 |
| (5-Methylisoxazol-3-yl)methyl 4-isopropyl-1,4,6,7-tetrahydro-5H-imidazo[4,5-c]pyridine-5-carboxylate | 2-(2,2,2-Trifluoroethoxy)ethyl 4-isopropyl-1,4,6,7-tetrahydro-5H-imidazo[4,5-c]pyridine-5-carboxylate |
| 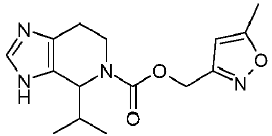 | 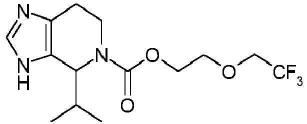 |
| [(2*S*)-1-Methylpyrrolidin-2-yl]methyl 4-isopropyl-1,4,6,7-tetrahydro-5H-imidazo[4,5-c]pyridine-5-carboxylate | |

Other specific examples of inhibitors of VAP-1 include the following, which are Examples from WO2011/113798:

| | |
|---|---|
| 3-(4-Chlorophenyl)-1-(oxolan-3-ylmethyl)-1 *H-*pyrrolo[3,2-*c*]pyridine | |
| 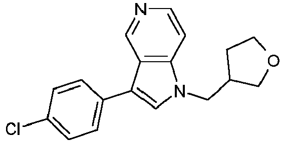 | |
| *tert*-Butyl 4-[3-(4-chlorophenyl)-1 *H*-pyrrolo[3,2-c]pyridin-1-yl]piperidine-1-carboxylate | |
| 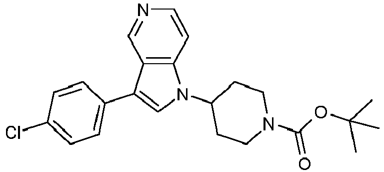 | |
| 3-(4-Chlorophenyl)-1-(oxolan-3-yl)-1*H-*pyrrolo[3,2-*c*]pyridine | 4-[1-(4-Chlorophenyl)-1 *H*-pyrrolo[2,3-c]pyridin-3-yl] |
| 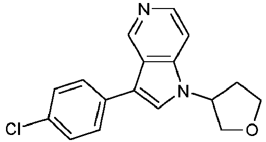 | 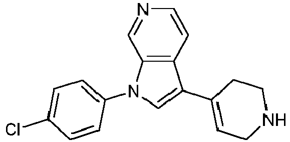 |
| 3-(4-Chlorophenyl)-1-(oxan-4-yl)-1 *H-*pyrrolo[3,2-*c*]pyridine | *tert*-Butyl 4-[1-(4-chlorophenyl)-1 *H*-pyrrolo[2,3-c]pyridin-3-yl]piperidine-1-carboxylate |
| 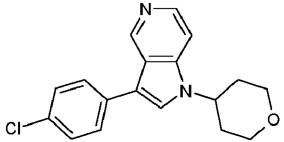 | 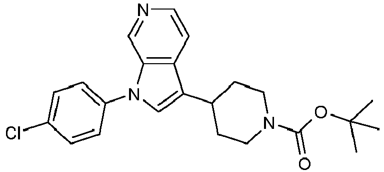 |
| 3-(4-Chlorophenyl)-1-piperidin-4-yl-1 *H-*pyrrolo[3,2-*c*]pyridine | 1-(4-Chlorophenyl)-3-piperidin-4-yl-1 *H-*pyrrolo[2,3-c]pyridine |
| 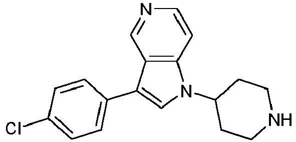 | 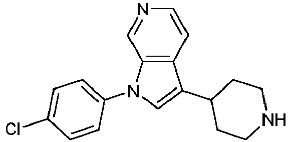 |
| 4-[3-(3,4-Dichlorophenyl)-1 *H*-pyrrolo[3,2-*c*]pyridin-1-yl]piperidine | *tert*-Butyl *N*-{4-[1-(4-chlorophenyl)-1*H-*pyrrolo[2,3-*c*]pyridin-3-yl]cyclohexyl}carbamate |
| 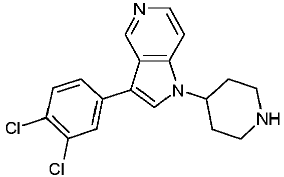 | 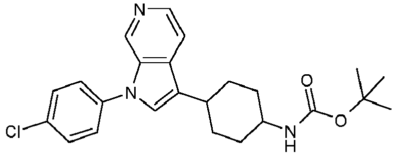 |
| 1-{4-[3-(3,4-Dichlorophenyl)-1*H*-pyrrolo[3,2-*c*]pyridin-1-yl]piperidin-1-yl}-2-hydroxyethan-1-one | 4-[1-(4-Chlorophenyl)-1*H*-pyrrolo[2,3-*c*]pyridin-3-yl]cyclohexan-1-amine |
| 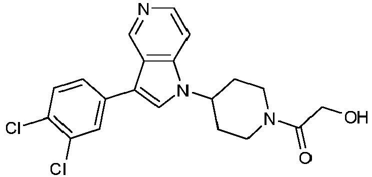 | 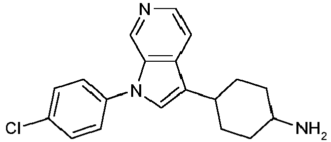 |
| 4-[1-(4-Chloro-2-methylphenyl)-1 *H*-pyrrolo[2,3-*c*]pyridin-3-yl]cyclohexan-1-amine | 3-Amino-1-{4-[1-(4-chlorophenyl)-1 *H-*pyrrolo[2,3-*c*]pyridin-3-yl]piperidin-1-yl}propan-1-one hydrochloride |
| 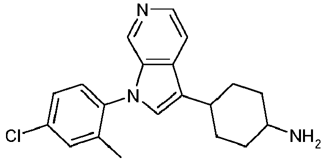 | 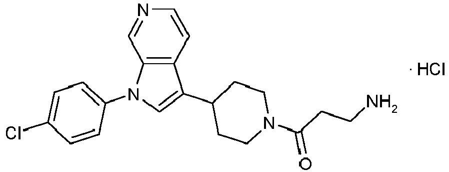 |
| 1-{4-[1-(4-Chlorophenyl)-1 *H*-pyrrolo[2,3-*c*]pyridin-3-yl]piperidin-1-yl}-2-(dimethyl-amino)ethan-1-one | 2-{4-[1-(4-Chlorophenyl)-1*H*-pyrrolo[2,3-*c*]pyridin-3-yl]piperidin-1-yl}ethan-1-ol |
| 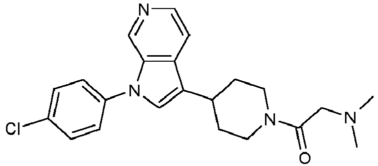 | 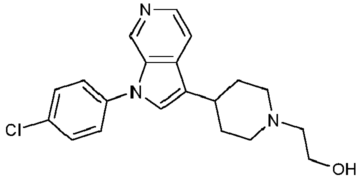 |
| 1-{4-[1-(4-Chlorophenyl)-1 *H*-pyrrolo[2,3-*c*]pyridine-3-yl]piperidin-1-yl}-2-hydroxyethan-1-one | 4-[1-(4-Chlorophenyl)-1*H*-pyrrolo[2,3-*c*]pyridin-3-yl]-1-(1*H*-pyrazol-3-ylmethyl)piperidine |
| 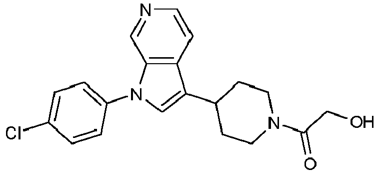 | 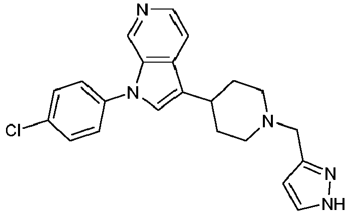 |
| 2-Amino-1-{4-[1-(4-chlorophenyl)-1 *H-*pyrrolo[2,3-c]pyridin-3-yl]piperidin-1-yl}ethan-1-one hydrochloride | 4-[1-(4-Chlorophenyl)-1 *H*-pyrrolo[2,3-c]pyridin-3-yl]-1-[(1-methyl-1 *H*-pyrazol-4-yl)-methyl]piperidine |
| 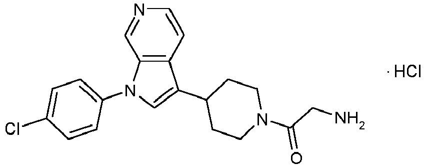 | 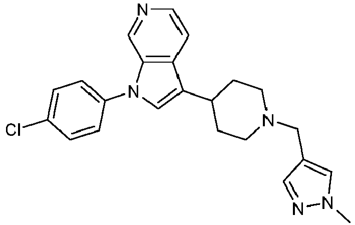 |
| 3-{4-[1-(4-Chlorophenyl)-1 *H*-pyrrolo[2,3-c]pyridine-3-yl]piperidin-1-yl}propanenitrile hydrochloride | 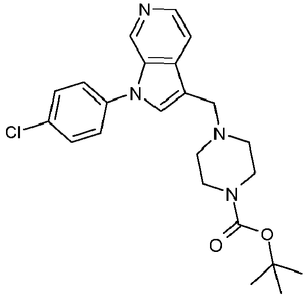 |
| 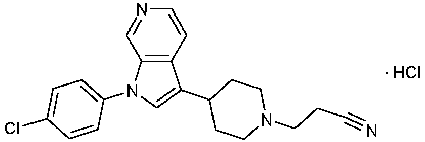 | |
| 4-{4-[1-(4-Chlorophenyl)-1 *H*-pyrrolo[2,3-*c*]pyridin-3-yl]piperidin-1-yl}butanenitrile hydrochloride | |
| 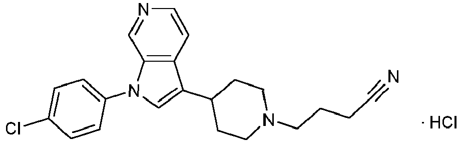 | 1-{[1-(4-Chlorophenyl)-1 *H*-pyrrolo[2,3-*c*]pyridin-3-yl]methyl}piperazine |
| [1-(4-Chlorophenyl)-1 *H*-pyrrolo[2,3-*c*]pyridin-3-yl]methanol | 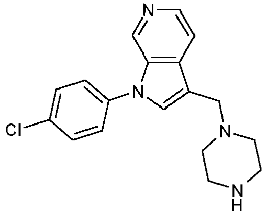 |
| 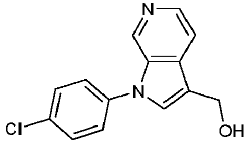 | 2-(1-{[1-(4-Chlorophenyl)-1 *H*-pyrrolo[2,3-*c*]pyridin-3-yl]methyl}piperidin-4-yl)ethan-1-ol |
| 1-{[1-(4-Chlorophenyl)-1 *H*-pyrrolo[2,3-*c*]pyridin-3-yl]methyl}-4-methylpiperazine | 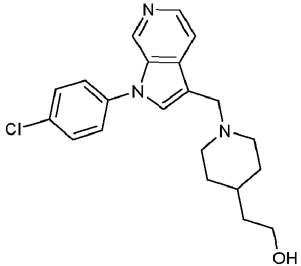 |
| 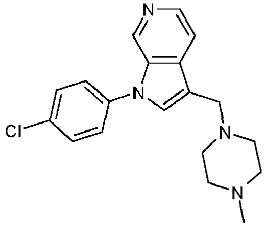 | |
| *tert*-Butyl 4-{[1-(4-chlorophenyl)-1*H-*pyrrolo[2,3-*c*]pyridin-3-yl] | (1-{[1-(4-Chlorophenyl)-1 *H*-pyrrolo[2,3-*c*]pyridin-3-yl]methyl}piperidin-4-yl)methanol |
| | 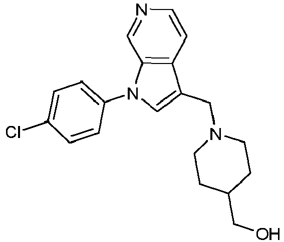 |
| 4-{[1-(4-Chlorophenyl)-1*H*-pyrrolo[2,3-*c*]pyridin-3-yl]methyl}morpholine | 3-(4-Chlorophenyl)-N-(2-methoxyethyl)-N-methylimidazo[1,5-a]pyrazin-1 -amine |
| 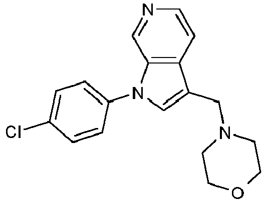 | 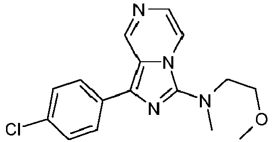 |
| 1-{[1-(4-Chlorophenyl)-1 *H*-pyrrolo[2,3-*c*]pyridin-3-yl]methyl}piperidin-4-ol | 3-(4-Chlorophenyl)-N,N-dimethylimidazo[1,5-a]pyrazin-1-amine |
| 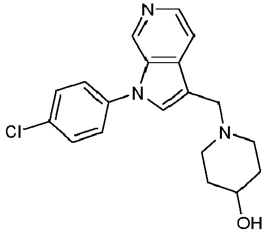 | 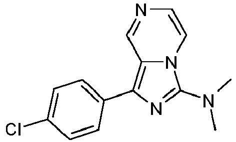 |
| | 3-(4-Chlorophenyl)-1-(oxan-4-yl)imidazo[1,5-a]pyrazine |
| 2-({[1-(4-Chlorophenyl)-1 *H*-pyrrolo[2,3-*c*]pyridin-3-yl]methyl}amino)ethan-1-ol | 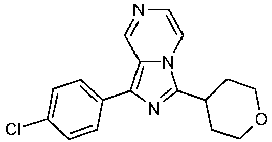 |
| 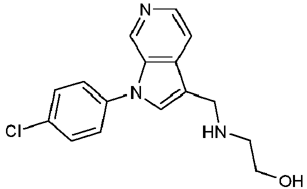 | |
| | 3-(4-Chlorophenyl)-1-(oxan-4-ylmethyl)imidazo[1,5-a]pyrazine |
| 4-[3-(4-Methylphenyl)imidazo[1,5-a]pyrazin-1-yl]morpholine | 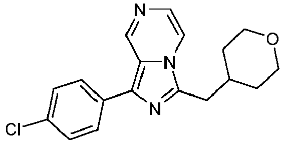 |
| 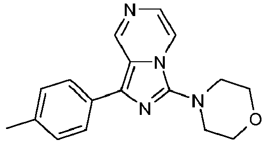 | 3-(4-Chlorophenyl)-1-(oxolan-3-yl)imidazo[1,5-a]pyrazine |
| 4-[3-(4-Chlorophenyl)imidazo[1,5-a]pyrazin-1-yl]morpholine | 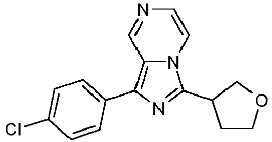 |
| 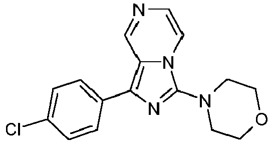 | 3-(4-Chlorophenyl)-1-(4-methoxycyclohexyl)imidazo[1,5-a]pyrazine |
| 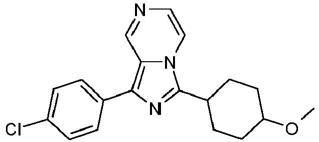 | 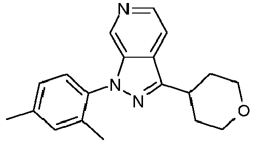 |
| 3-(Oxan-4-yl)-1-phenyl-1 H-pyrazolo[3,4-c]pyridine | 5-Methyl-2-[3-(oxan-4-yl)-1 H-pyrazolo[3,4-c]pyridin-1-yl]pyridine |
| 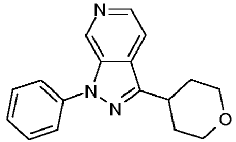 | 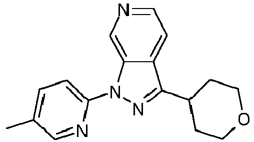 |
| 4-[3-(Oxan-4-yl)-1 H-pyrazolo[3,4-c]pyridin-1-yl]benzonitrile | 2-Methyl-5-[3-(oxan-4-yl)-1 H-pyrazolo[3,4-c]pyridin-1-yl]pyridine |
| 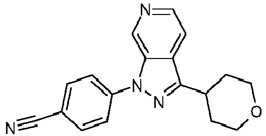 | 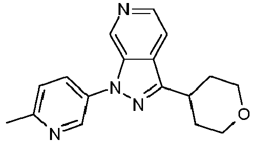 |
| 1-[4-(Difluoromethyl)phenyl]-3-(oxan-4-yl)-1H-pyrazolo[3,4-c]pyridine | 1-[1-(4-Chlorophenyl)-1H-pyrazolo[3,4-c]pyridin-3-yl]-3,3-difluoropyrrolidine |
| 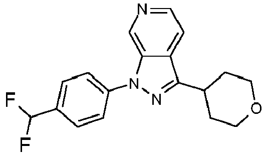 | 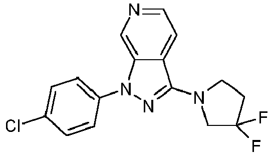 |
| 1-(2-Fluoro-4-methylphenyl)-3-(oxan-4-yl)-1 H-pyrazolo[3,4-c]pyridine | 1-[1-(4-Chlorophenyl)-1H-pyrazolo[3,4-c]pyridin-3-yl]pyrrolidin-3-ol |
| 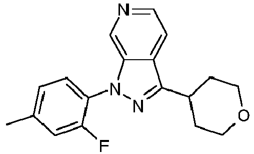 | 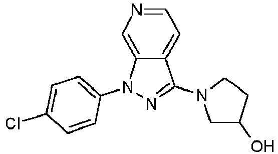 |
| 1-(4-Chloro-2-fluorophenyl)-3-(oxan-4-yl)-1 H-pyrazolo[3,4-c]pyridine | 3-Methoxy-1-[1-(4-methylphenyl)-1H-pyrazolo[3,4-c]pyridin-3-yl]pyrrolidine |
| 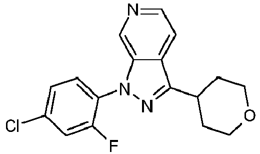 | 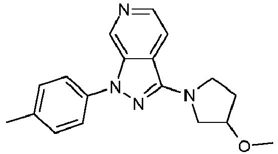 |
| 1-(2,4-Dimethylphenyl)-3-(oxan-4-yl)-1 H-pyrazolo[3,4-c]pyridine | 1-[1-(4-Chlorophenyl)-1H-pyrazolo[3,4-c]pyridin-3-yl]piperidine |
| 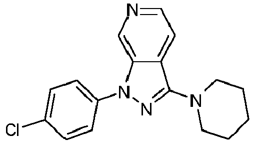 | 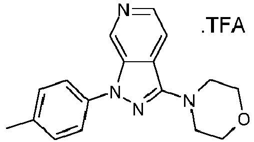 |
| 1-[1-(4-Chlorophenyl)-1 H-pyrazolo[3,4-c]pyridin-3-yl]-4,4-difluoropiperidine | 4-[1-(2-Fluoro-4-methylphenyl)-1 H-pyrazolo[3,4-c]pyridin-3-yl]morpholine |
| 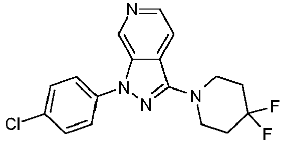 | 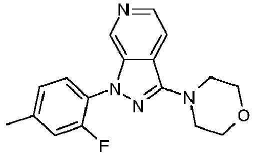 |
| 1-[1-(4-Chlorophenyl)-1H-pyrazolo[3,4-*c*]pyridin-3-yl]piperidin-4-ol | 4-[1-(4-Chlorophenyl)-1H-pyrazolo[3,4-c]pyridin-3-yl]-2-methylmorpholine |
| 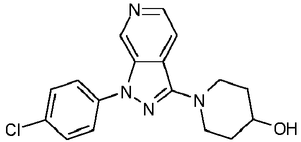 | 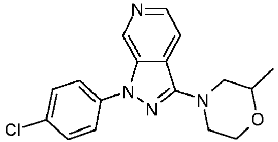 |
| 1-[1-(4-Methylphenyl)-1 H-pyrazolo[3,4-c]pyridin-3-yl]piperidine-4-carboxamide | 4-[1-(4-Chlorophenyl)-1 H-pyrazolo[3,4-c]pyridin-3-yl]-3-methylmorpholine |
| 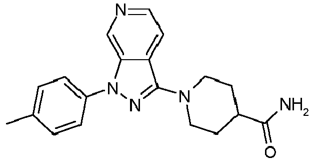 | 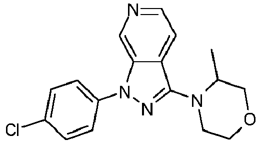 |
| 4-[1-(4-Fluorophenyl)-1 H-pyrazolo[3,4-c]pyridin-3-yl]morpholine | 4-[1-(4-Methylphenyl)-1H-pyrazolo[3,4-c]pyridin-3-yl]-2-(2-methylpropyl)morpholine |
| 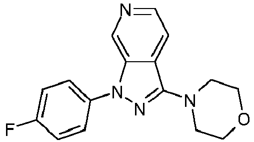 | 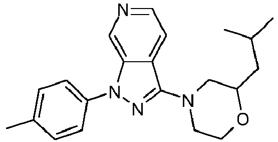 |
| 4-[1-(4-Chlorophenyl)-1 H-pyrazolo[3,4-c]pyridin-3-yl]morpholine | (2S,6R)-4-[1-(4-Chlorophenyl)-1 H-pyrazolo[3,4-c]pyridin-3-yl]-2,6-dimethyl morpho line |
| 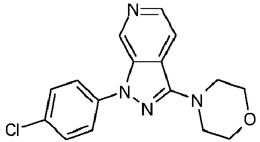 | 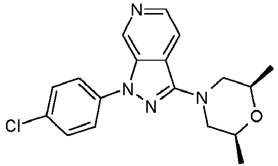 |
| 2,2,2-Trifluoroacetic acid; 4-[1-(4-methylphenyl)-1H-pyrazolo[3,4-c]pyridin-3-yl]morpholine | 3-[1-(4-Methylphenyl)-1 H-pyrazolo[3,4-c]pyridin-3-yl]-8-oxa-3-azabicyclo[3.2.1]octane |
| 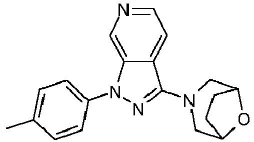 | 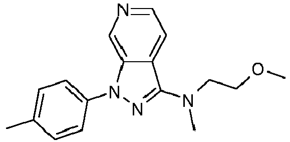 |
| 2,2-Dimethyl-4-[1-(4-methylphenyl)-1 H-pyrazolo[3,4-c]pyridin-3-yl]morpholine | 1-[1-(4-Chlorophenyl)-1 H-pyrazolo[3,4-c]pyridin-3-yl]piperazine |
| 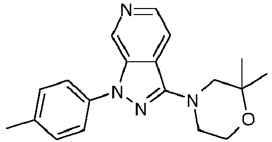 | 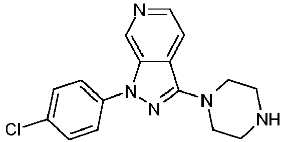 |
| 3,3-Dimethyl-4-[1-(4-methylphenyl)-1 H-pyrazolo[3,4-c]pyridin-3-yl]morpholine | 1-[1-(4-Chlorophenyl)-1H-pyrazolo[3,4-c]pyridin-3-yl]piperidin-4-amine |
| 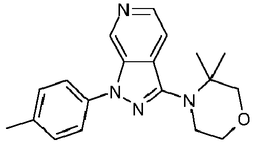 | 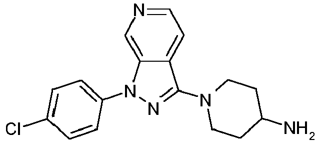 |
| Methyl 4-[1-(4-methylphenyl)-1 H-pyrazolo[3,4-c]pyridin-3-yl] morpholine-3-carboxylate | {4-[1-(4-Methylphenyl)-1 H-pyrazolo[3,4-c]pyridin-3-yl]morpholin-2-yl}methanamine |
| 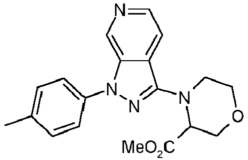 | 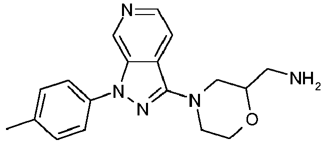 |
| 4-[1-(4-Methylphenyl)-1 H-pyrazolo[3,4-c]pyridin-3-yl]-1,4-oxazepane | *tert*-Butyl N-(2-methoxyethyl)-N-[1-(4-methylphenyl)-1H-pyrazolo[3,4-c]pyridin-3-yl] carbamate |
| 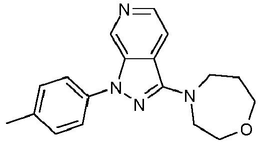 | 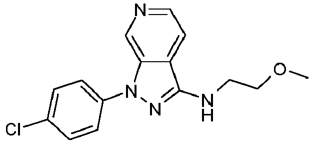 |
| 4-[1-(4-Methylphenyl)-1 H-pyrazolo[3,4-c]pyridin-3-yl]piperazin-2-one | 1-[1-(4-Methylphenyl)-1 H-pyrazolo[3,4-c]pyrid in-3-yl]piperidin-4-ol |
| 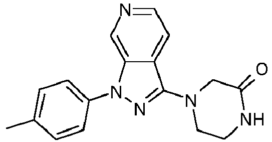 | 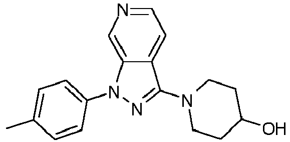 |
| N-(2-Methoxyethyl)-N-methyl-1-(4-methylphenyl)-1H-pyrazolo[3,4-c]pyridin-3-amine | 1-[1-(4-Chlorophenyl)-1 H-pyrazolo[3,4-c]pyridin-3-yl]-4-(1 H-pyrazol-3-ylmethyl)piperazine |
| 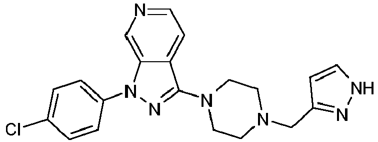 | 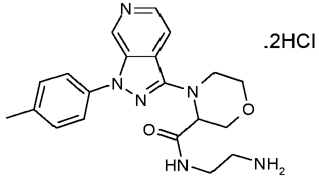 |
| tert-Butyl N-(3-{4-[1-(4-chlorophenyl)-1H-pyrazolo[3,4-c]pyridin-3-yl]piperazin-1-yl}-3-oxopropyl)carbamate | 1-(4-Chlorophenyl)-3-(oxan-4-yl)-1H-pyrazolo[3,4-c]pyridine |
| 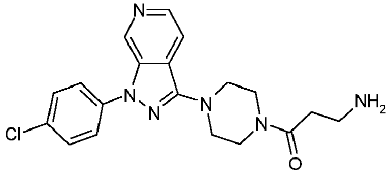 | 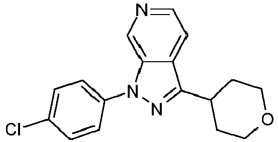 |
| 4-[1-(4-Methylphenyl)-1 H-pyrazolo[3,4-c]pyridin-3-yl]morpholine-3-carboxamide | 1-(4-Methylphenyl)-3-(oxolan-3-yl)-1 H-pyrazolo[3,4-c]pyridine |
| 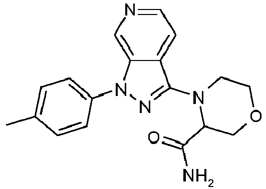 | 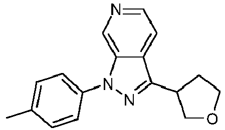 |
| | 1-(4-Methylphenyl)-3-(oxan-4-yl)-1 H-pyrazolo[3,4-c]pyridine |
| 4-[1-(4-Methylphenyl)-1 H-pyrazolo[3,4-c]pyridin-3-yl]-3-[(morpholin-4-yl)carbonyl]morpholine | 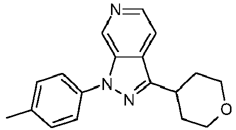 |
| 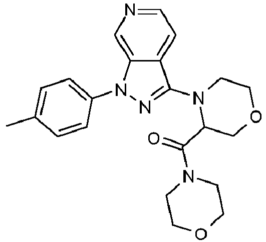 | 1-(4-Fluorophenyl)-3-(oxan-4-yl)-1 H-pyrazolo[3,4-c]pyridine |
| | 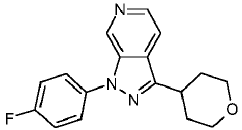 |
| N-(2-Aminoethyl)-4-[1-(4-methylphenyl)-1H-pyrazolo[3,4-c]pyridin-3-yl]morpholine-3-carboxamide dihydrochloride | 4-[1-(4-Chlorophenyl)-1 H-pyrazolo[3,4-c]pyridin-3-yl]piperidine |
| | 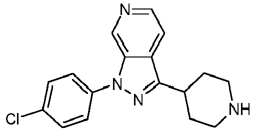 |
| 3-[1-(4-Methylphenyl)-1H-pyrazolo[3,4-c]pyridin-3-yl]morpholine | 4-[1-(4-Chlorophenyl)-1 H-pyrazolo[3,4-c]pyridin-3-yl]-N,N-dimethylpiperidine-1-carboxamide |
| 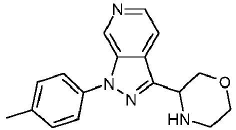 | 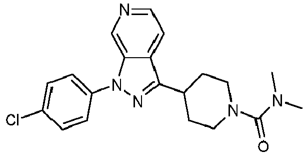 |
| 2-[1-(4-Methylphenyl)-1 H-pyrazolo[3,4-c]pyridin-3-yl]morpholine | |
| 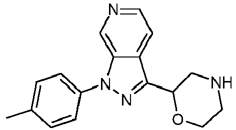 | Ethyl 4-[1-(4-chlorophenyl)-1 H-pyrazolo[3,4-c]pyridin-3-yl]piperidine-1-carboxylate |
| 5-[1-(4-Methylphenyl)-1 H-pyrazolo[3,4-c]pyridin-3-yl]piperidin-2-one | 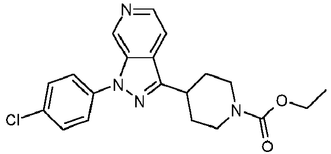 |
| 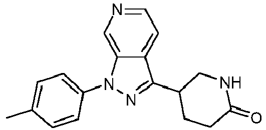 | 3-Amino-1-{4-[1-(4-chlorophenyl)-1 H-pyrazolo[3,4-c]pyridin-3-yl]piperidin-1-yl}propan-1-one dihydrochloride |
| 1-(4-Chlorophenyl)-4-fluoro-3-(oxan-4-yl)-1 H-pyrazolo[3,4-c]pyridine | |
| 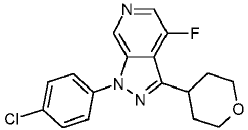 | 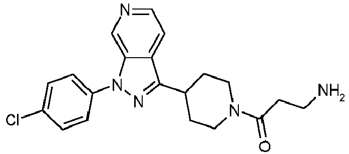 |
| 4-[1-(4-Chlorophenyl)-1H-pyrazolo[3,4-c]pyridin-3-yl]-1-(1H-pyrazol-3-ylmethyl)piperidine | 1-(4-Chlorophenyl)-4-methoxy-3-(oxan-4-yl)-1 H-pyrazolo[3,4-c]pyridine |
| 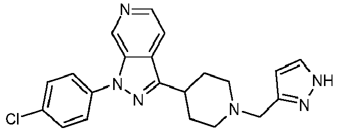 | 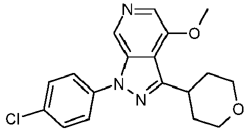 |
| | 1-(4-Chlorophenyl)-3-(oxan-4-yl)-1 H-pyrazolo[3,4-c]pyridin-4-ol |
| 1-Butyl-4-[1-(4-chlorophenyl)-1H-pyrazolo[3,4-c]pyridin-3-yl]piperidine | 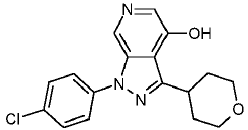 |
| 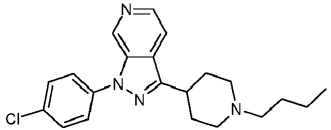 | |
| 1-(4-Chlorophenyl)-5-methoxy-3-(oxan-4-yl)-1 H-pyrazolo[3,4-c]pyridine | 1-(4-Methylphenyl)-3-(oxan-4-yl)-1H-pyrrolo[2,3-c]pyridine |
| 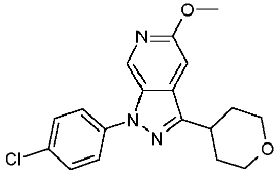 | 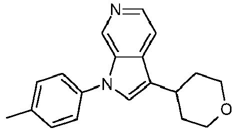 |
| 1-(4-Chlorophenyl)-3-(oxan-4-yl)-1 H,5H,6H-pyrazolo[3,4-c]pyridin-5-one | 5-Chloro-2-[3-(oxan-4-yl)-1H-pyrrolo[2,3-c]pyridin-1-yl]pyridine |
| 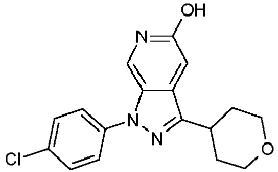 | 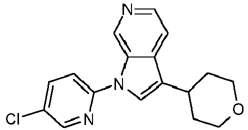 |
| 5-(4-Chlorophenyl)-7-(oxan-4-yl)-5H-pyrrolo[3,2-d]pyrimidine | 4-[1-(4-Chlorophenyl)-1 H-pyrrolo[2,3-c]pyridin-3-yl]morpholine 2,2,2-trifluoroacetic acid |
| 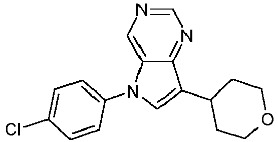 | 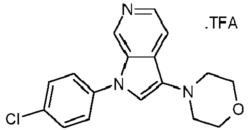 |
| 1-(4-Chlorophenyl)-3-(oxan-4-yl)-1 H-pyrazolo[4,3-d]pyrimidine | 2,2,2-Trifluoroacetic acid; 4-amino-1-{4-[1-(4-chlorophenyl)-1H-pyrrolo[2,3-c]pyridin-3-yl]piperidin-1-yl}butan-1-one |
| 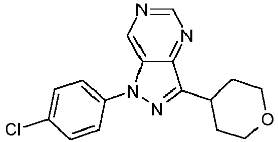 | 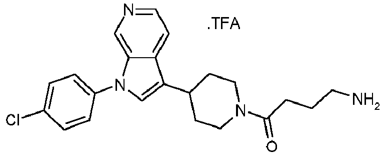 |
| 1-(4-Fluorophenyl)-3-(oxan-4-yl)-1H-pyrrolo[2,3-c]pyridine | 2-Aminoethyl 4-[1-(4-chlorophenyl)-1 H-pyrrolo[2,3-c]pyridin-3-yl]piperidine-1-carboxylate |
| 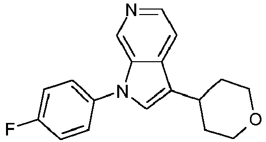 | 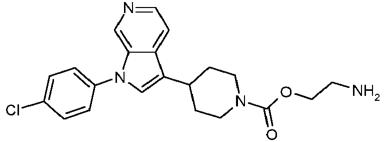 |
| 1-(4-Chlorophenyl)-3-(oxan-4-yl)-1 H-pyrrolo[2,3-c]pyridine | |
| 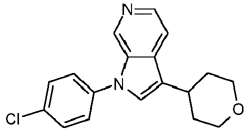 | |
| 3-(3,6-Dihydro-2H-pyran-4-yl)-2-methyl-1-(4-methylphenyl)-1 H-pyrrolo[2,3-c]pyridine | |
| 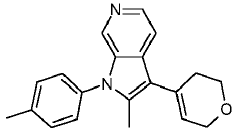 | |

Further specific VAP-1 compounds include the Examples of WO2013/037411, namely:

| | |
|---|---|
| 2,2,2-Trifluoroacetic acid; 2-{4-[1-(4-chlorophenyl)-1 H-pyrrolo[2,3-c]pyridin-3-yl]piperidin-1-yl}ethan-1-amine | 5-Amino-1-{4-[1-(4-chlorophenyl)-1 H-pyrrolo[2,3-c]pyridin-3-yl]piperidin-1-yl}pentan-1 -one |
| 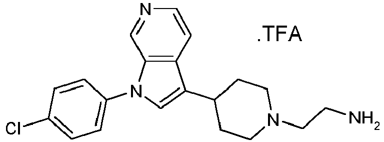 | 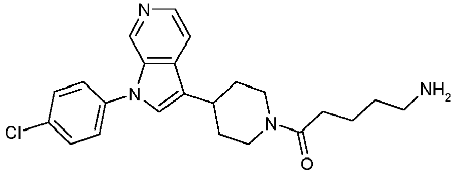 |
| 3-Aminopropyl 4-[1-(4-chlorophenyl)-1 H-pyrrolo[2,3-c]pyridin-3-yl]piperidine-1-carboxylate | N-(2-Aminoethyl)-4-[1-(4-chlorophenyl)-1 H-pyrrolo[2,3-c]pyridin-3-yl]piperidine-1-carboxamide |
| 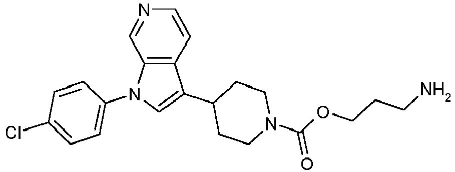 | 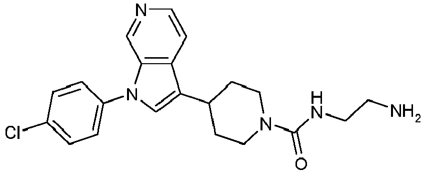 |
| 1-{4-[1-(4-Chlorophenyl)-1 H-pyrrolo[2,3-c]pyridin-3-yl]piperidin-1-yl}-4-(dimethylamino)butan-1-one; 2,2,2-trifluoroacetic acid | N-(3-Aminopropyl)-4-[1-(4-chlorophenyl)-1H-pyrrolo[2,3-c]pyridin-3-yl]piperidine-1-carboxamide |
| 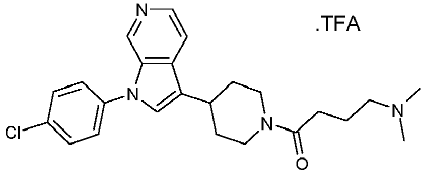 | 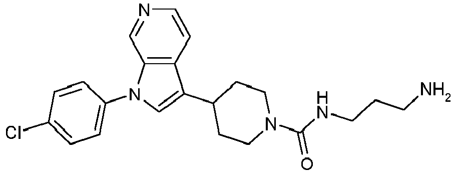 |
| 4-[1-(4-Chlorophenyl)-1 H-pyrrolo[2,3-c]pyridin-3-yl]-N-[3-(dimethylamino)propyl]piperidine-1-carboxamide | Ethyl 1-[1-(4-methylphenyl)-1 H-pyrazolo[3,4-c]pyridin-3-yl]piperidine-4-carboxylate |
| 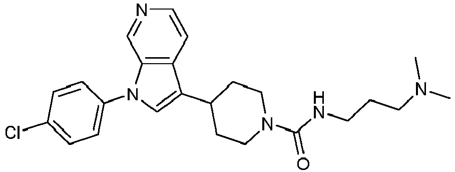 | 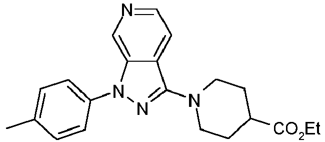 |
| 1-({4-[1-(4-Chlorophenyl)-1 H-pyrrolo[2,3-c]pyridin-3-yl]piperidin-1-yl}carbonyl)piperazine | 1-[1-(4-Chlorophenyl)-1H-pyrazolo[3,4-c]pyridin-3-yl]piperidine-4-carboxylic acid hydrochloride |
| 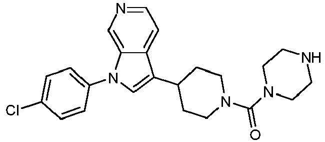 | 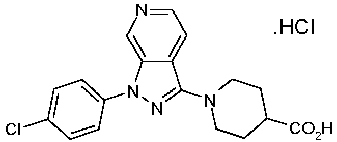 |
| 4-({4-[1-(4-Chlorophenyl)-1H-pyrrolo[2,3-c]pyridin-3-yl]piperidin-1-yl}carbonyl)morpholine | N-(2-Aminoethyl)-1-[1-(4-chlorophenyl)-1H-pyrazolo[3,4-c]pyridin-3-yl]piperidine-4-carboxamide dihydrochloride |
| 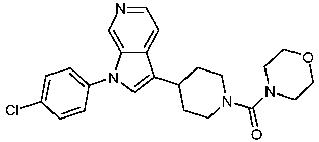 | 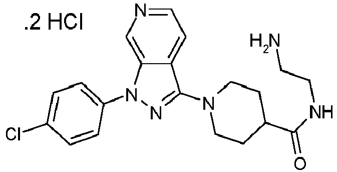 |
| 1-({4-[1-(4-Chlorophenyl)-1 H-pyrrolo[2,3-c]pyridin-3-yl]piperidin-1-yl}carbonyl)-1,4-diazepane | -({1-[1-(4-Chlorophenyl)-1 H-pyrazolo[3,4-c]pyridin-3-yl]piperidin-4-yl}carbonyl) morpholine |
| 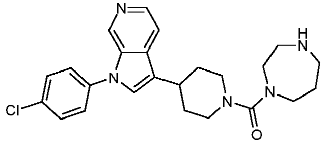 | 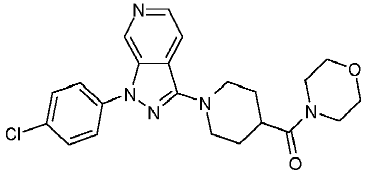 |
| Ethyl 1-[1-(4-chlorophenyl)-1 H-pyrazolo[3,4-c]pyridin-3-yl]piperidine-4-carboxylate | |
| 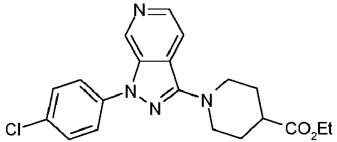 | 1-({1-[1-(4-Chlorophenyl)-1 H-pyrazolo[3,4-c]pyridin-3-yl]piperidin-4-yl}carbonyl)piperazine dihydrochloride |
| 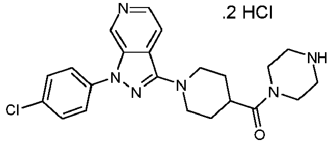 | 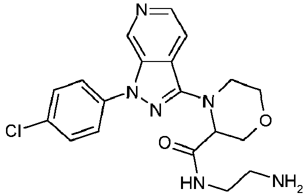 |
| {4-[1-(4-Methylphenyl)-1 H-pyrazolo[3,4-c]pyridin-3-yl]morpholin-3-yl}methanol | 2-{4-[1-(4-Chlorophenyl)-1 H-pyrazolo[3,4-c]pyridin-3-yl]morpholin-3-yl}ethan-1-ol |
| 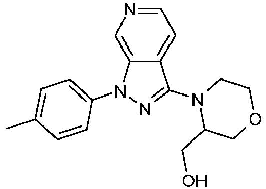 | 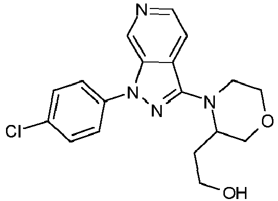 |
| {4-[1-(4-Methylphenyl)-1 H-pyrazolo[3,4-c]pyridin-3-yl]morpholin-2-yl}methanol | Methyl 1-[1-(4-chlorophenyl)-1 H-pyrazolo[3,4-c]pyridin-3-yl]piperidine-2-carboxylate |
| 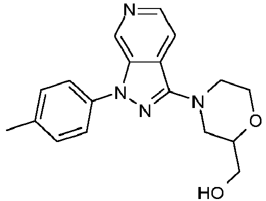 | 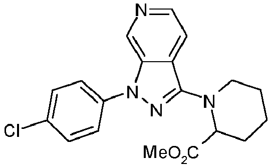 |
| [(3R)-4-[1-(4-Chlorophenyl)-1 H-pyrazolo[3,4-c]pyridin-3-yl]morpholin-3-yl]methanol | |
| 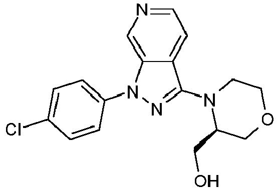 | N-(2-Aminoethyl)-1-[1-(4-chlorophenyl)-1H-pyrazolo[3,4-c]pyridin-3-yl]piperidine-2-carboxamide dihydrochloride |
| Methyl 4-[1-(4-chlorophenyl)-1 H-pyrazolo[3,4-c]pyridin-3-yl]morpholine-3-carboxylate | 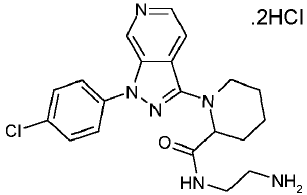 |
| 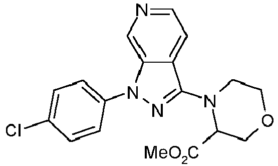 | 1-({1-[1-(4-Chlorophenyl)-1H-pyrazolo[3,4-c]pyridin-3-yl]piperidin-2-yl}carbonyl) piperazine |
| N-(2-Aminoethyl)-4-[1-(4-chlorophenyl)-1H-pyrazolo[3,4-c]pyridin-3-yl]morpholine-3-carboxamide | |
| 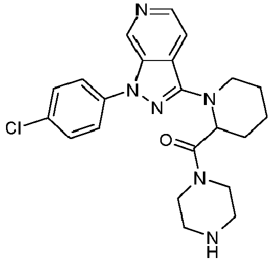 | 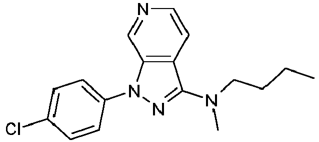 |
| | 1-[4-(Fluoromethyl)phenyl]-3-(oxan-4-yl)-1 H-pyrazolo[3,4-c]pyridine |
| 4-[1-(4-Methylphenyl)-1 H-pyrrolo[2,3-c]pyridin-3-yl]morpholine | 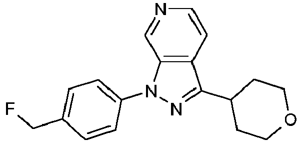 |
| 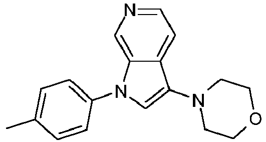 | 3-({4-[1-(4-Chlorophenyl)-1 H-pyrazolo[3,4-c]pyridin-3-yl]piperidin-1-yl}methyl)pyridine |
| 1-(4-Chlorophenyl)-3-(piperidin-4-yl)-1 H-pyrrolo[2,3-c]pyridin-4-ol | 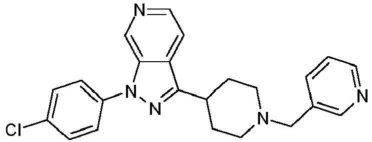 |
| 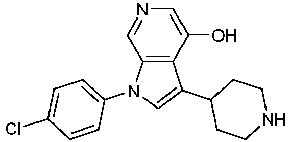 | |
| N-Butyl-1-(4-chlorophenyl)-N-methyl-1H-pyrazolo[3,4-c]pyridin-3-amine | |

Further specific examples of VAP-1 compounds include the Examples of WO2013/038189, namely:

| | |
|---|---|
| 4-[1-(4-Chlorophenyl)-1 H-pyrrolo[2,3-c]pyridin-3-yl]-1-(pyrrolidin-3-yl)piperidine | |
| 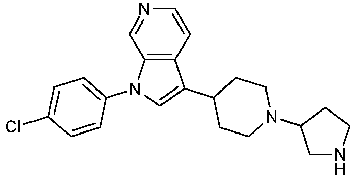 | |
| 4-[1-(4-Chlorophenyl)-1 H-pyrrolo[2,3-c]pyridin-3-yl]-1-(piperidin-4-yl)piperidine | |
| 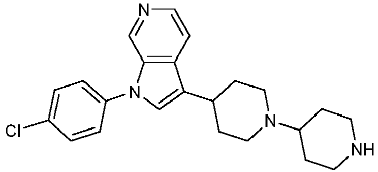 | |
| 4-[1-(4-Chlorophenyl)-1 H-pyrrolo[2,3-c]pyridin-3-yl]-1-(piperidin-4-ylmethyl) piperidine | 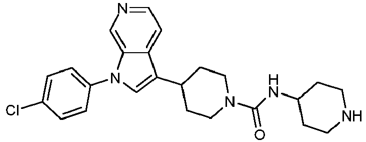 |
| 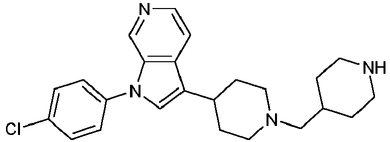 | 4-[1-(4-Chlorophenyl)-1 H-pyrrolo[2,3-c]pyridin-3-yl]-N-(1-methylpiperidin-4-yl)piperidine-1-carboxamide |
| 1-{4-[1-(4-Chlorophenyl)-1 H-pyrrolo[2,3-c]pyridin-3-yl]piperidin-1-yl}-2-(piperidin-4-yl)ethan-1-one | 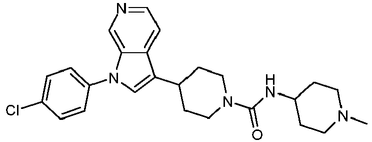 |
| 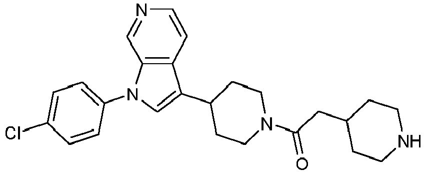 | 4-[1-(4-Chlorophenyl)-1 H-pyrrolo[2,3-c]pyridin-3-yl]-N-[1-(propan-2-yl)piperidin-4-yl]piperidine-1-carboxamide |
| | 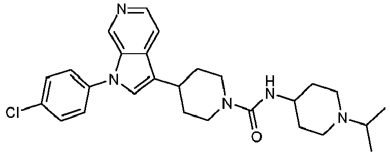 |
| 1-({4-[1-(4-Chlorophenyl)-1 H-pyrrolo[2,3-c]pyridin-3-yl]piperidin-1-yl}carbonyl)-4-methylpiperazine | N-(1-Acetylpiperidin-4-yl)-4-[1-(4-chlorophenyl)-1 H-pyrrolo[2,3-c]pyridin-3-yl]piperidine-1-carboxamide |
| 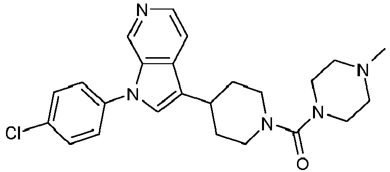 | 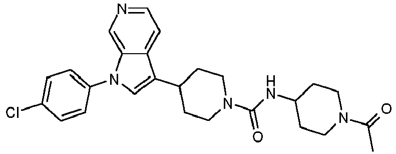 |
| 4-[1-(4-Chlorophenyl)-1 H-pyrrolo[2,3-c]pyridin-3-yl]-N-(piperidin-4-ylmethyl)piperidine-1-carboxamide | 4-[1-(4-Chlorophenyl)-1 H-pyrrolo[2,3-c]pyridin-3-yl]-N-[(1-methylpiperidin-4-yl)methyl]piperidine-1-carboxamide |
| 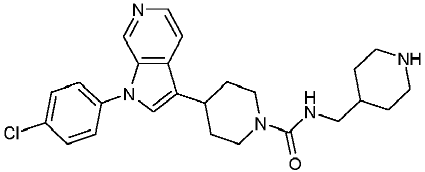 | 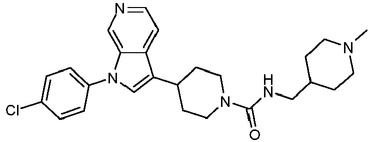 |
| 4-[1-(4-Chlorophenyl)-1 H-pyrrolo[2,3-c]pyridin-3-yl]-N-(piperidin-4-yl)piperidine-1-carboxamide | 4-[1-(4-Chlorophenyl)-1 H-pyrrolo[2,3-c]pyridin-3-yl]-N-[(1-ethylpiperidin-4-yl)methyl]piperidine-1-carboxamide |
| 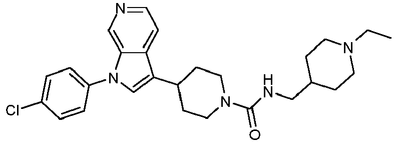 | 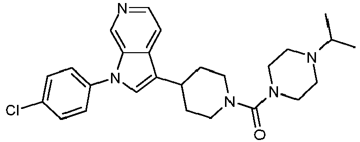 |
| 4-[1-(4-Chlorophenyl)-1 H-pyrrolo[2,3-c]pyridin-3-yl]-N-methyl-N-[(1-methylpiperidin-4-yl)methyl]piperidine-1-carboxamide; formic acid | 1-({4-[1-(4-Chlorophenyl)-1H-pyrrolo[2,3-c]pyridin-3-yl]piperidin-1-yl}carbonyl)-4-(2-methoxyethyl) piperazine |
| 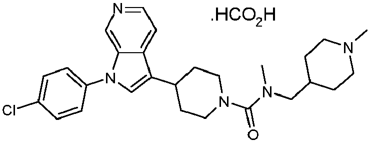 | 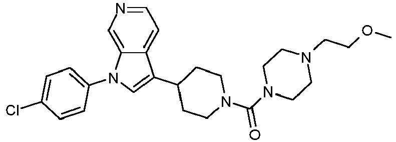 |
| N-{[1-(Carbamoylmethyl)piperidin-4-yl]methyl}-4-[1-(4-chlorophenyl)-1 H-pyrrolo[2,3-c]pyridin-3-yl]piperidine-1-carboxamide; formic acid | (3S)-1-({4-[1-(4-Chlorophenyl)-1 H-pyrrolo[2,3-c]pyridin-3-yl]piperidin-1-yl}carbonyl)-3-(propan-2-yl)piperazine |
| 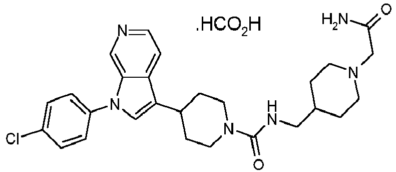 | 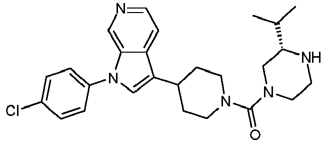 |
| 4-[1-(4-Chlorophenyl)-1 H-pyrrolo[2,3-c]pyridin-3-yl]-N-methyl-N-{[1-(propan-2-yl)piperidin-4-yl]methyl}piperidine-1-carboxamide | 4-[1-(4-Chlorophenyl)-1 H-pyrrolo[2,3-c]pyridin-3-yl]-N-(morpholin-2-ylmethyl)piperidine-1-carboxamide |
| 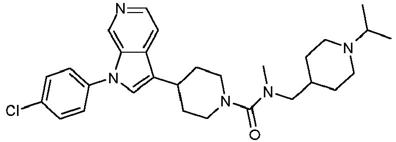 | 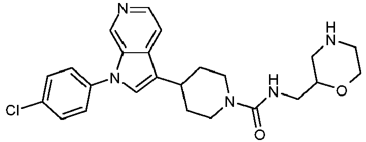 |
| 1-({4-[1-(4-Chlorophenyl)-1 H-pyrrolo[2,3-c]pyridin-3-yl]piperidin-1-yl}carbonyl)-4-cyclopropylpiperazine | 4-[1-(4-Chlorophenyl)-1 H-pyrrolo[2,3-c]pyridin-3-yl]-N-[(1,4-dimethyl piperazin-2-yl)methyl]piperidine-1-carboxamide |
| 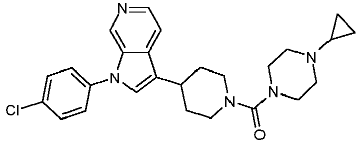 | 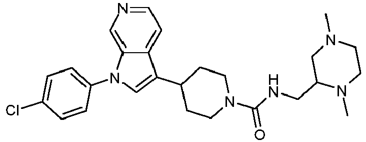 |
| 1-({4-[1-(4-Chlorophenyl)-1H-pyrrolo[2,3-c]pyridin-3-yl]piperidin-1-yl}carbonyl)-4-(propan-2-yl)piperazine | 4-[1-(4-Chlorophenyl)-1 H-pyrrolo[2,3-c]pyridin-3-yl]-N-[2-(morpholin-4-yl)ethyl]piperidine-1-carboxamide |
| | acid |
| 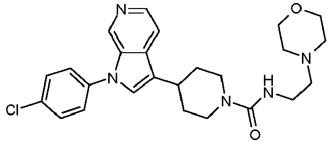 | 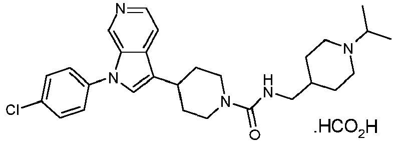 |
| 4-[1-(4-Chlorophenyl)-1 H-pyrrolo[2,3-c]pyridin-3-yl]-N-[2-(piperazin-1-yl)ethyl]piperidine-1-carboxamide | 4-[1-(4-Chlorophenyl)-1 H-pyrrolo[2,3-c]pyridin-3-yl]-N-{[1-(2-methoxyethyl)piperidin-4-yl]methyl}piperidine-1-carboxamide; formic acid |
| 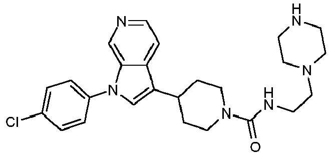 | 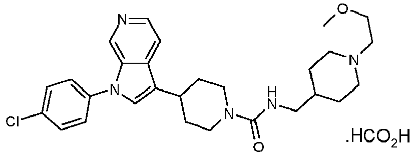 |
| 4-[1-(4-Chlorophenyl)-1 H-pyrrolo[2,3-c]pyridin-3-yl]-N-[2-(1-methylpiperidin-4-yl)ethyl]piperidine-1-carboxamide | N-[3-({4-[1-(4-Chlorophenyl)-1H-pyrrolo[2,3-c]pyridin-3-yl]piperidin-1-yl}carbonylamino)propyl]acetamide |
| 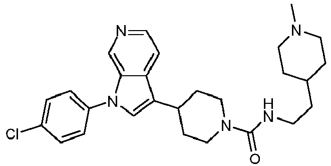 | 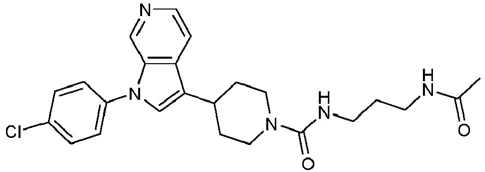 |
| 4-[1-(4-Chlorophenyl)-1 H-pyrrolo[2,3-c]pyridin-3-yl]-N-[2-(4-methylpiperazin-1-yl)ethyl]piperidine-1-carboxamide | Propan-2-yl N-({4-[1-(4-methylphenyl)-1H-pyrazolo[3,4-c]pyridin-3-yl]morpholin-2-yl}methyl)carbamate |
| 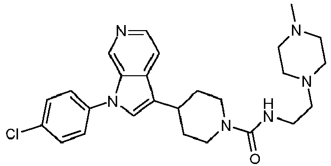 | 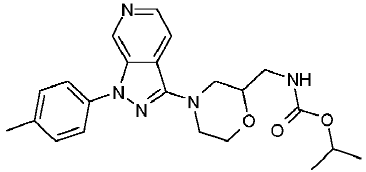 |
| 4-[1-(4-Chlorophenyl)-1 H-pyrrolo[2,3-c]pyridin-3-yl]-N-[3-(morpholin-4-yl)propyl]piperidine-1-carboxamide | 3-Cyclopropyl-1-({4-[1-(4-methylphenyl)-1 H-pyrazolo[3,4-c]pyridin-3-yl]morpholin-2-yl}methyl)urea |
| 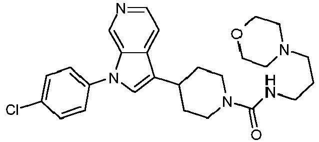 | 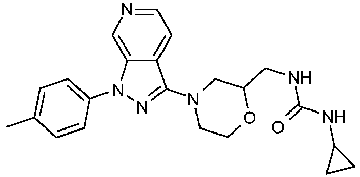 |
| 4-[1-(4-Chlorophenyl)-1 H-pyrrolo[2,3-c]pyridin-3-yl]-N-{[1-(propan-2-yl)piperidin-4-yl]methyl}piperidine-1-carboxamide; formic | |
| 2-({4-[1-(4-Methylphenyl)-1H-pyrazolo[3,4-c]pyridin-3-yl]morpholin-2-yl}methoxy)ethan-1-amine | 3-Aminopropyl 4-({4-[1-(4-chlorophenyl)-1H-pyrazolo[3,4-c]pyridin-3-yl]morpholin-2-yl}methyl)piperazine-1-carboxylate trihydrochloride |
| 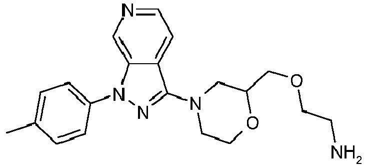 | 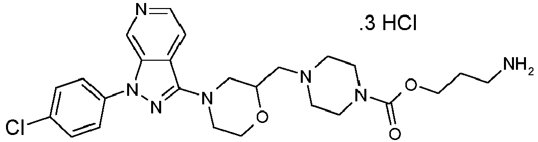 |
| 2-Aminoethyl)({4-[1-(4-methylphenyl)-1 H-pyrazolo[3,4-c]pyridin-3-yl]morpholin-2-yl}methyl)amine trihydrochloride | N-(3-Aminopropyl)-4-({4-[1-(4-chlorophenyl)-1 H-pyrazolo[3,4-c]pyridin-3-yl] morpholin-2-yl}methyl)piperazine-1-carboxamide trihydrochloride |
| 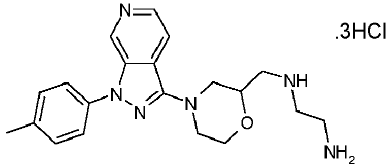 | 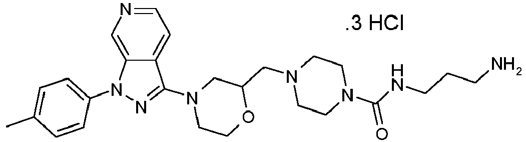 |
| 4-[1-(4-Methylphenyl)-1 H-pyrazolo[3,4-c]pyridin-3-yl]-2-(morpholin-4-ylmethyl)morpholine | 4-({4-[1-(4-Chlorophenyl)-1 H-pyrazolo[3,4-c]pyridin-3-yl]morpholin-2-yl}methyl)-N-ethylpiperazine-1-carboxamide |
| 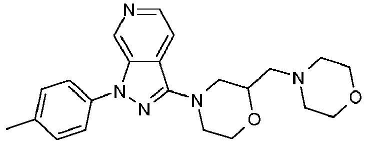 | 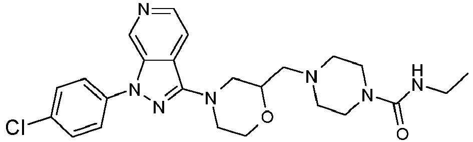 |
| 4-[1-(4-Chlorophenyl)-1 H-pyrazolo[3,4-c]pyridin-3-yl]-2-[(4-methylpiperazin-1-yl)methyl]morpholine | Methyl 2-{4-[1-(4-chlorophenyl)-1 H-pyrazolo[3,4-c]pyridin-3-yl]morpholin-3-yl}acetate |
| 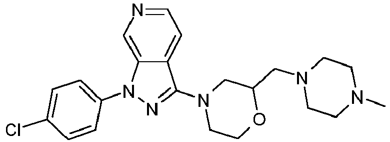 | 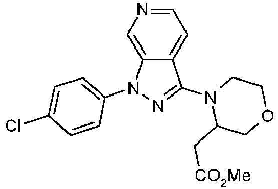 |
| 4-[1-(4-Chlorophenyl)-1 H-pyrazolo[3,4-c]pyridin-3-yl]-2-(piperazin-1-ylmethyl) morpholine trihydrochloride | 4-[1-(4-Chlorophenyl)-1H-pyrazolo[3,4-c]pyridin-3-yl]-3-(morpholin-4-ylmethyl)morpholine |
| 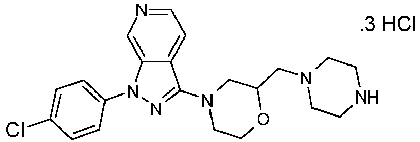 | |
| 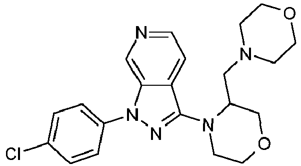 | 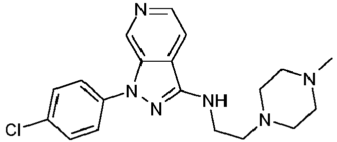 |
| 4-[1-(4-Chlorophenyl)-1H-pyrazolo[3,4-c]pyridin-3-yl]-3-[2-(4-methylpiperazin-1-yl)ethyl]morpholine | 1-[1-(4-Chlorophenyl)-1H-pyrazolo [3,4-c]pyridin-3-yl]-N-(piperidin-4-ylmethyl)piperidine-4-carboxamide dihydrochloride |
| 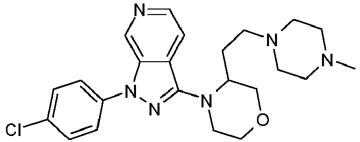 | 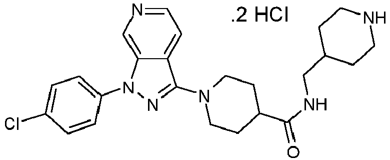 |
| 1-[1-(4-Chlorophenyl)-1H-pyrazolo[3,4-c]pyridin-3-yl]-N-[(1-methylpiperidin-4-yl)methyl]piperidine-2-carboxamide | 4-({1-[1-(4-Chlorophenyl)-1H-pyrazolo[3,4-c]pyridin-3-yl]piperidin-4-yl}methyl)morpholine dihydrochloride |
| 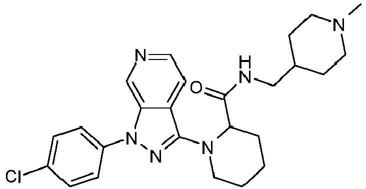 | 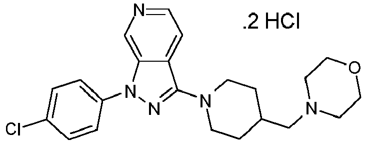 |
| 1-(4-Chlorophenyl)-N-[2-(morpholin-4-yl)ethyl]-1H-pyrazolo[3,4-c]pyridin-3-amine | |
| 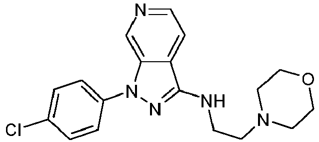 | 1-({1-[1-(4-Chlorophenyl)-1 H-pyrazolo[3,4-c]pyridin-3-yl]piperidin-4-yl}methyl)piperazine |
| 1-(4-Chlorophenyl)-N-[2-(piperazin-1-yl)ethyl]-1 H-pyrazolo[3,4-c]pyridin-3-amine | 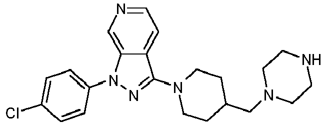 |
| 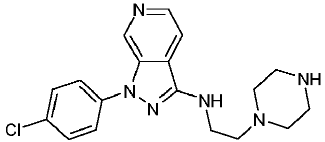 | ({1-[1-(4-Chlorophenyl)-1H-pyrazolo[3,4-c]pyridin-3-yl]piperidin-4-yl}methyl)(piperidin-4-ylmethyl)amine |
| 1-(4-Chlorophenyl)-N-[2-(4-methylpiperazin-1-yl)ethyl]-1H-pyrazolo[3,4-c]pyridin-3-amine | 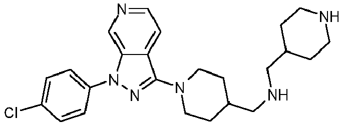 |
| 4-[1-(4-Chlorophenyl)-1H-pyrazolo[3,4-c]pyridin-3-yl]-N-[(1-methylpiperidin-4-yl)methyl]piperazine-1-carboxamide | 2-{4-[1-(4-Chlorophenyl)-1 H-pyrazolo[3,4-c]pyridin-3-yl]morpholin-3-yl}-1-(4-methlierazin-1-yl)ethan-1-one |
| 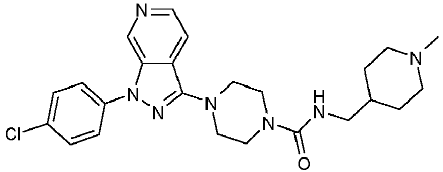 | 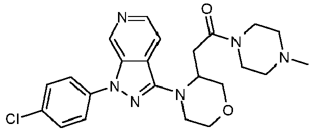 |
| 1-[1-(4-Methylphenyl)-1H-pyrazolo[3,4-c]pyridin-3-yl]piperidin-4-yl acetate | 2-{4-[1-(4-Chlorophenyl)-1 H-pyrazolo[3,4-c]pyridin-3-yl]morpholin-3-yl}-1-[(3S)-3-(dimethylamino)pyrrolidin-1-yl]ethan-1-one |
| 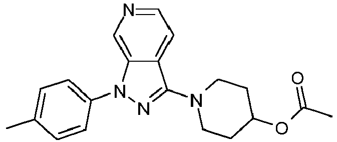 | 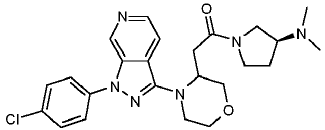 |
| 2-{4-[1-(4-Chlorophenyl)-1 H-pyrazolo[3,4-c]pyridin-3-yl]morpholin-3-yl}acetic acid hydrochloride | 2-{4-[1-(4-Chlorophenyl)-1H-pyrazolo[3,4-c]pyridin-3-yl]morpholin-3-yl}-N-(1-methylpiperidin-4-yl)acetamide |
| 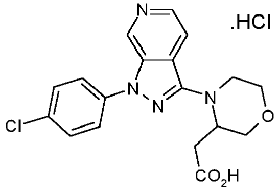 | 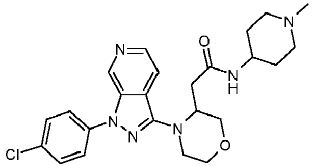 |
| N-(2-Aminoethyl)-2-{4-[1-(4-chlorophenyl)-1H-pyrazolo[3,4-c]pyridin-3-yl]morpholin-3-yl}acetamide dihydrochloride | 2-{4-[1-(4-Chlorophenyl)-1H-pyrazolo[3,4-c]pyridin-3-yl]morpholin-3-yl}-N-[(1-methylpiperidin-4-yl)methyl]acetamide |
| 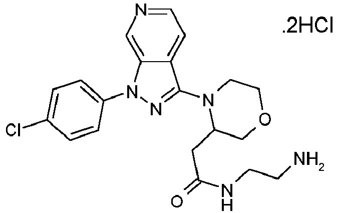 | 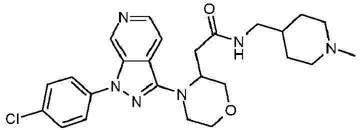 |

Specific examples of inhibitors of VAP-1 include the compounds speficially disclosed as Examples in WO 2010/031791, namely:

| | |
|---|---|
| 3-(4-Fluorophenyl)-1-(tetrahydro-2H-pyran-4-yl)-1H-pyrazolo[4,3-c]pyridine | 3-(4-Chlorophenyl)-1-piperidin-4-yl-1 H-pyrazolo[4,3-c]pyridine |
| 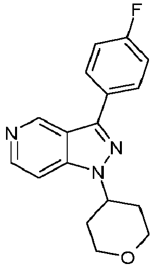 | 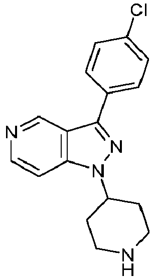 |
| 3-(4-Chlorophenyl)-2-(tetrahydro-2H-pyran-4-yl)-2H-pyrazolo[4,3-c]pyridine | -(4-Chlorophenyl)-1-(1-methylpiperidin-4-yl)-1 H-pyrazolo[4,3-c]pyridine |
| 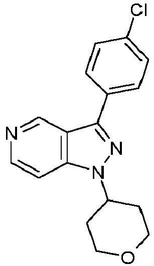 | 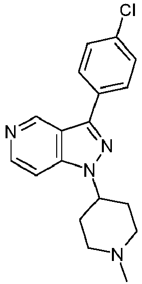 |
| 3-(4-Methylphenyl)-1-(tetrahydro-2H-pyran-4-yl)-1 H-pyrazolo[4,3-c]pyridine | {4-[3-(4-Chlorophenyl)-1 H-pyrazolo[4,3-c]pyridin-1-yl]piperidin-1-yl}acetonitrile |
| 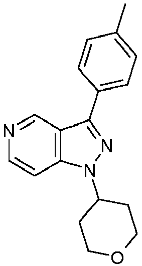 | 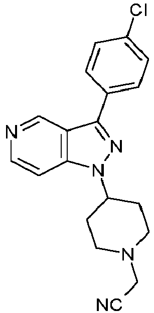 |
| 3-(4-Chlorophenyl)-1-[(3R)-tetrahydrofuran-3-yl]-1 H-pyrazolo[4,3-c]pyridine | |
| 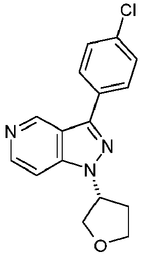 | |
| 3-(4-Chlorophenyl)-1-(tetrahydro-2H-pyran-4-ylmethyl)-1 H-pyrazolo[4,3-c]pyridine | 3-(4-Chlorophenyl)-1-[1-(2-methoxyethyl)piperidin-4-yl]-1H-pyrazolo[4,3-c]pyridine |
| 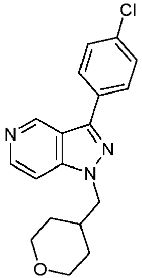 | 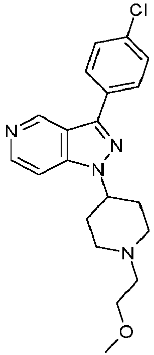 |
| 3-(4-Chlorophenyl)-1-[1-(methylsulfonyl)piperidin-4-yl]-1 H-pyrazolo[4,3-c]pyridine | 3-(4-Chlorophenyl)-1-piperidin-3-yl-1H-pyrazolo[4,3-c]pyridine |
| 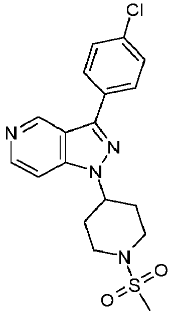 | 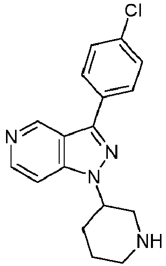 |
| 1-(1-Acetylpiperidin-4-yl)-3-(4-chlorophenyl)-1 H-pyrazolo[4,3-c]pyridine | 3-(4-Chlorophenyl)-1-[(3S)-tetrahydrofuran-3-yl]-1 H-pyrazolo[4,3-c]pyridine |
| 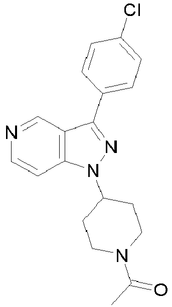 | 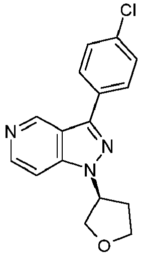 |
| 3-(4-Chlorophenyl)-1-(tetrahydrofuran-3-ylmethyl)-1 H-pyrazolo[4,3-c]pyridine | -(4-Fluorophenyl)-1-(1-methylpiperidin-4-yl)-1 H-pyrazolo[4,3-c]pyridine |
| 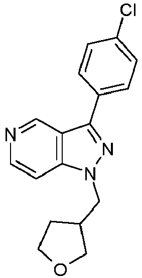 | 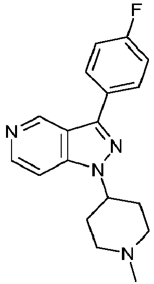 |
| 3-(4-Chlorophenyl)-1-(1-ethylpiperidin-4-yl)-1 H-pyrazolo[4,3-c]pyridine | 3-(4-Fluorophenyl)-1-piperidin-4-yl-1 H-pyrazolo[4,3-c]pyridine |
| 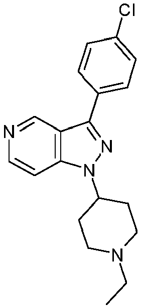 | 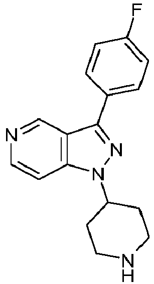 |
| 3-(4-Chlorophenyl)-1-(1-isopropylpiperidin-4-yl)-1 H-pyrazolo[4,3-c]pyridine | -[1-(Tetrahydro-2H-pyran-4-yl)-1 H-pyrazolo[4,3-c]pyridin-3-yl]benzonitrile |
| 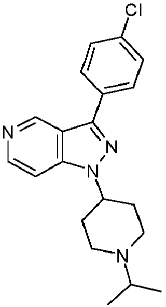 | 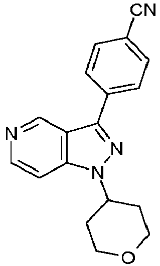 |
| -[1-(1-Methylpiperidin-4-yl)-1 H-pyrazolo[4,3-c]pyridin-3-yl]benzonitrile | |
| 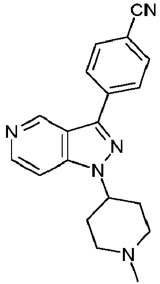 | |

Specific examples of inhibitors of VAP-1 include the compounds speficially disclosed as Examples in WO 2010/064020, namely:

| | |
|---|---|
| [2-(4-Methylphenyl)imidazo[1,2-a]pyridin-3-yl]methanol | [2-(4-Bromophenyl)-7-methylimidazo[1,2-a]pyridin-3-yl]methanol |
| 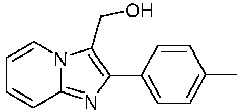 | 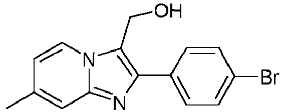 |
| [2-(2,4-Dichlorophenyl)imidazo[1,2-a]pyridin-3-yl]methanol | [2-(4-Bromophenyl)-7-ethylimidazo[1,2-a]pyridin-3-yl]methanol |
| 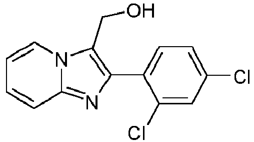 | 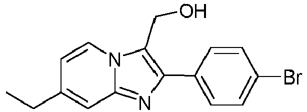 |
| [2-(4-Bromophenyl)-8-methylimidazo[1,2-a]pyridin-3-yl]methanol | [2-(2-Chlorophenyl)-7-methylimidazo[1,2-a]pyridin-3-yl]methanol |
| 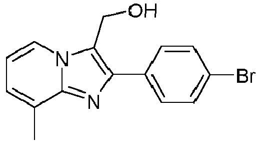 | 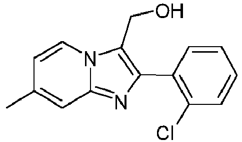 |
| [7-Methyl-2-(4-methylphenyl)imidazo[1,2-a]pyridin-3-yl]methanol | [2-(2,4-Dichlorophenyl)-7-methylimidazo[1,2-a]pyridin-3-yl]methanol |
| 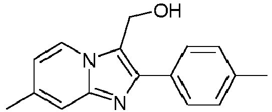 | 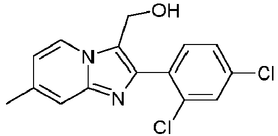 |
| [2-(3,4-Dichlorophenyl)-7-methylimidazo[1,2-a]pyridin-3-yl]methanol | [2-(4-lodophenyl)-6-methylimidazo[1,2-a]pyridin-3-yl]methanol |
| 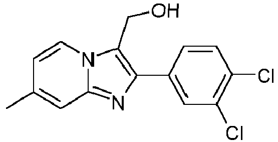 | 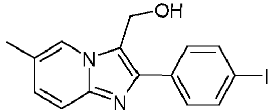 |
| [6-Methyl-2-(2-naphthyl)imidazo[1,2-a]pyridin-3-yl]methanol | [2-(2-Chlorophenyl)-6-methylimidazo[1,2-a]pyridin-3-yl]methanol |
| 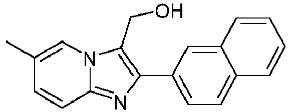 | 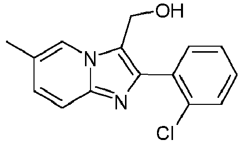 |
| [2-(3-Methoxyphenyl)-6-methylimidazo[1,2-a]pyridin-3-yl]methanol | (2-{4-[(2-Aminoethyl)amino]phenyl}-6-methylimidazo[1,2-a]pyridin-3-yl)methanol |
| 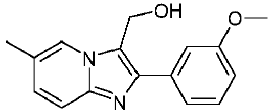 | 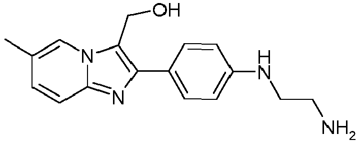 |
| 4-[3-(Hydroxymethyl)-6-methylimidazo[1,2-a]pyridin-2-yl]benzonitrile | 1-[2-(4-Chlorophenyl)-6-methylimidazo[1 ,2-a]pyridin-3-yl]ethanol |
| 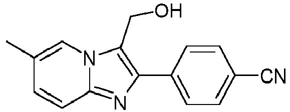 | 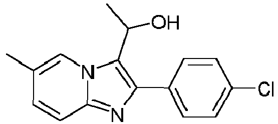 |
| [6-Methyl-2-(3-nitrophenyl)imidazo[1,2-a]pyridin-3-yl]methanol | [2-(2,4-Dichlorophenyl)-6-methylimidazo[1,2-a]pyridin-3-yl]methanol |
| 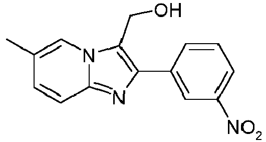 | 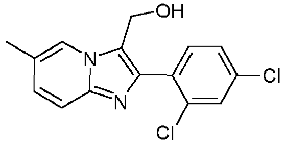 |
| [2-(4-Chlorophenyl)-6-methylimidazo[1,2-a]pyridin-3-yl]methanol | [2-(3-Methoxyphenyl)-6-(trifluoromethyl)imidazo[1,2-a]pyridin-3-yl]methanol |
| 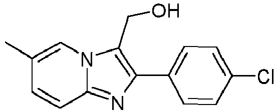 | 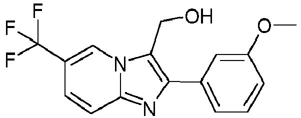 |
| 2-(4-Fluorophenyl)-6-methylimidazo[1,2-a]pyridin-3-yl]methanol | |
| 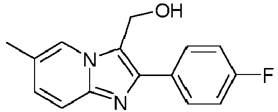 | |
| [2-(4-Chlorophenyl)-6-(trifluoromethyl)imidazo[1,2-a]pyridin-3-yl]methanol | [6-Bromo-2-(3-methoxyphenyl)imidazo[1,2-a]pyridin-3-yl]methanol |
| 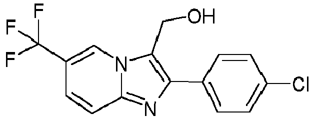 | 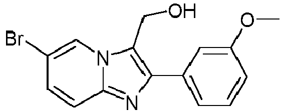 |
| [2-(4-Bromophenyl)-6-(trifluoromethyl)imidazo[1,2-a]pyridin-3-yl]methanol | 6-Chloro-2-(4-chlorophenyl)imidazo[1,2-a]pyridin-3-yl]methanol |
| 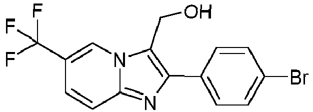 | 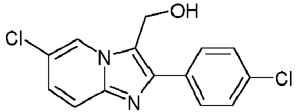 |
| | [6-Bromo-2-(4-fluorophenyl)imidazo[1,2-a]pyridin-3-yl]methanol |
| [7-Chloro-2-(4-chlorophenyl)imidazo[1,2-a]pyridin-3-yl]methanol | 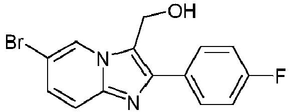 |
| 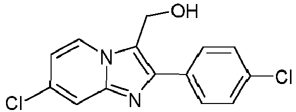 | [6-Bromo-2-(4-bromophenyl)imidazo[1,2-a]pyridin-3-yl]methanol trifluoroacetate |
| | 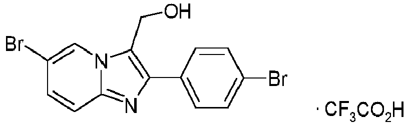 |
| [2-(4-Bromophenyl)-7-chloroimidazo[1,2-a]pyridin-3-yl]methanol trifluoroacetate | [2-(4-Bromophenyl)-6-chloroimidazo[1,2-a]pyridin-3-yl]methanol trifluoroacetate |
| 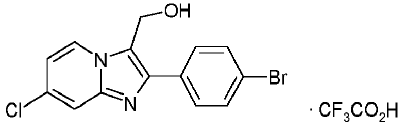 | 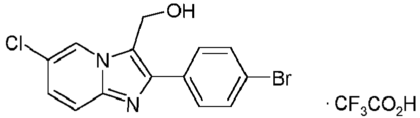 |
| [7-Chloro-2-(2,4-dichlorophenyl)imidazo[1,2-a]pyridin-3-yl]methanol | [2-(4-Chlorophenyl)-6-fluoroimidazo[1,2-a]pyridin-3-yl]methanol |
| 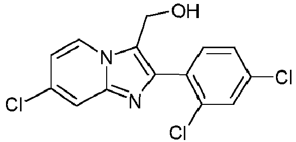 | 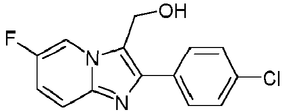 |
| [7-Chloro-2-(2,4-difluorophenyl)imidazo[1,2-a]pyridin-3-yl]methanol | [6-Bromo-2-(2,4-difluorophenyl)imidazo[1,2-a]pyridin-3-yl]methanol |
| 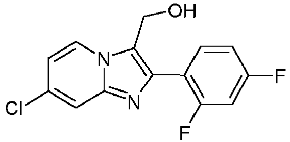 | 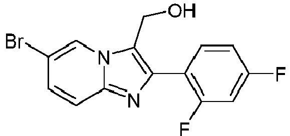 |
| [6-Chloro-2-(2,4-difluorophenyl)imidazo[1,2-a]pyridin-3-yl]methanol | 6,8-Dichloro-2-(3-methoxyphenyl)imidazo[1,2-a]pyridin-3-yl]methanol |
| 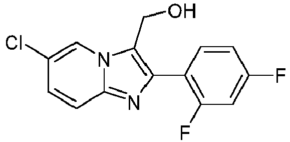 | 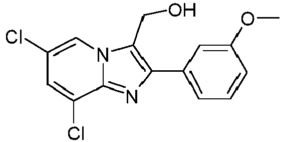 |
| [6-Bromo-2-(2,4-dichlorophenyl)imidazo[1,2-a]pyridin-3-yl]methanol | [2-(4-Bromophenyl)-6,8-dichloroimidazo[1,2-a]pyridin-3-yl]methanol |
| 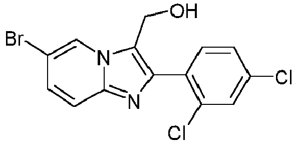 | 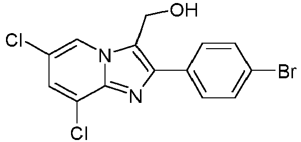 |
| [6-Chloro-2-(2,4-dichlorophenyl)imidazo[1,2-a]pyridin-3-yl]methanol | 2-(4-Bromophenyl)-3-(hydroxymethyl)imidazo[1,2-a]pyridine-6-carbonitrile |
| 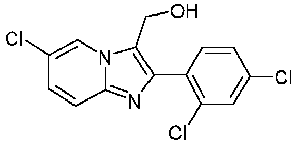 | 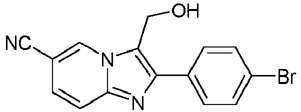 |
| [6-Bromo-2-(3,4-difluorophenyl)imidazo[1,2-a]pyridin-3-yl]methanol | |
| 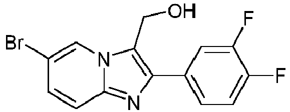 | Methyl 2-(4-bromophenyl)-3-(hydroxymethyl)imidazo[1,2-a]pyridine-6-carboxylate |
| [6-Bromo-2-(3-chloro-4-fluorophenyl)imidazo[1,2-a]pyridin-3-yl]methanol | 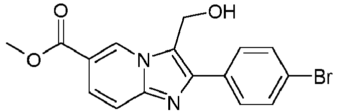 |
| 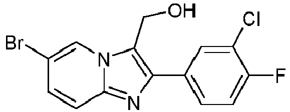 | Methyl 2-(4-chlorophenyl)-3-(hydroxymethyl)imidazo[1,2-a]pyridine-6-carboxylate hydrobromide |
| [6-Chloro-2-(3-chloro-4-fluorophenyl)imidazo[1,2-a]pyridin-3-yl]methanol | 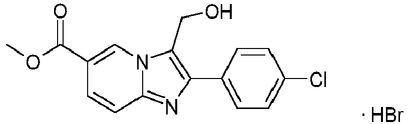 |
| 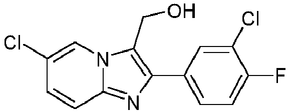 | [2-(4-Bromophenyl)imidazo[1,2-a]pyridine-3,7-diyl]dimethanol |
| | 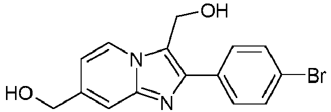 |
| [2-(4-Chlorophenyl)imidazo[1,2-a]pyridine-3,6-diyl]dimethanol | 2-(4-Chlorophenyl)-3-(hydroxymethyl)-N,N-dimethylimidazo[1,2-a]pyridine-6-carboxamide |
| 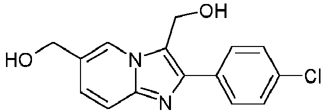 | 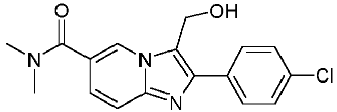 |
| [2-(4-Chlorophenyl)-6-nitroimidazo[1,2-a]pyridin-3-yl]methanol | 2-(4-Chlorophenyl)-3-(hydroxymethyl)-N-methylimidazo[1,2-a]pyridine-6-carboxamide |
| 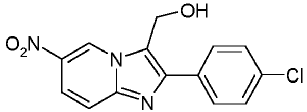 | 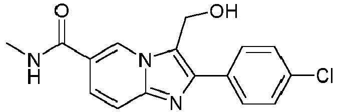 |
| [2-(4-Bromophenyl)-6-nitroimidazo[1,2-a]pyridin-3-yl]methanol hydrochloride | 2-(4-Chlorophenyl)-3-(hydroxymethyl)-N-[2-(1-methylpyrrolidin-2-yl)ethyl]imidazo[1,2-a]pyridine-6-carboxamide |
| 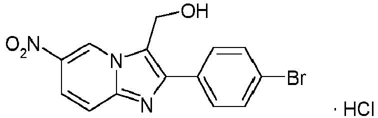 | 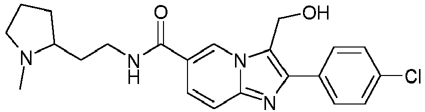 |
| {2-(4-Chlorophenyl)-6-[(4-methoxypiperidin-1-yl)carbonyl]imidazo[1,2-a]pyridin-3-yl}methanol | {2-(4-Chlorophenyl)-6-[(4-methylpiperazin-1-yl)carbonyl]imidazo[1,2-a]pyridin-3-yl}methanol |
| 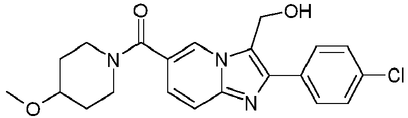 | 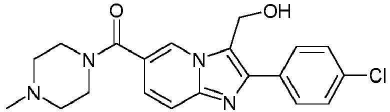 |
| 2-(4-Chlorophenyl)-3-(hydroxymethyl)-N-(3-methoxypropyl)imidazo[1,2-a]pyridine-6-carboxamide | 2-(4-Chlorophenyl)-N-(3,4-dimethoxybenzyl)-3-(hydroxymethyl)imidazo[1,2-a]pyridine-6-carboxamide |
| 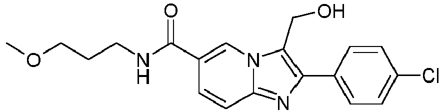 | 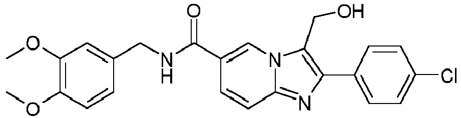 |
| 2-(4-Chlorophenyl)-3-(hydroxymethyl)-N-(2-methoxyethyl)imidazo[1,2-a]pyridine-6-carboxamide | 2-(4-Chlorophenyl)-3-(hydroxymethyl)-N-[2-(1H-imidazol-4-yl)ethyl]imidazo[1,2-a]pyridine-6-carboxamide |
| 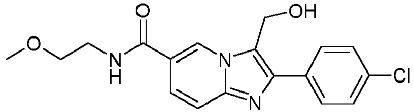 | 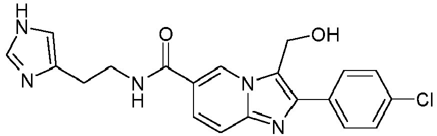 |
| [2-(4-Chlorophenyl)-6-(morpholin-4-ylcarbonyl)imidazo[1,2-a]pyridin-3-yl]methanol | |
| 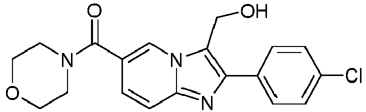 | |
| 2-(4-Chlorophenyl)-3-(hydroxymethyl)-N-(pyridin-3-ylmethyl)imidazo[1,2-a]pyridine-6-carboxamide | 1-{[2-(4-Chlorophenyl)-3-(hydroxymethyl)imidazo[1,2-a]pyridin-6-yl]carbonyl}piperidin-4-ol |
| 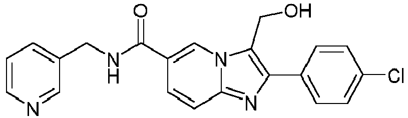 | 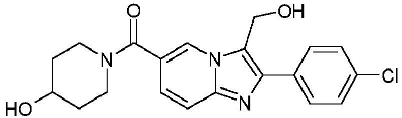 |
| -(4-Chlorophenyl)-3-(hydroxymethyl)-N-(3-hydroxypropyl)imidazo[1,2-a]pyridine-6-carboxamide | (3R)-1-{[2-(4-Chlorophenyl)-3-(hydroxymethyl)imidazo[1,2-a]pyridin-6-yl]carbonyl}pyrrolidin-3-ol |
| 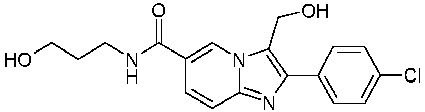 | 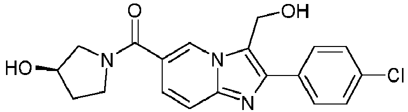 |
| (1-{[2-(4-Chlorophenyl)-3-(hydroxymethyl)imidazo[1,2-a]pyridin-6-yl]carbonyl}piperidin-4-yl)methanol | 1-{[2-(4-Chlorophenyl)-3-(hydroxymethyl)imidazo[1,2-a]pyridin-6-yl]carbonyl}pyrrolidin-3-ol |
| 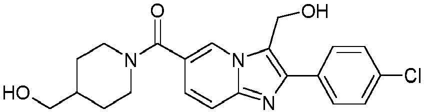 | 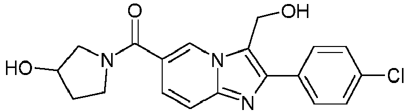 |
| 2-(4-Chlorophenyl)-3-(hydroxymethyl)-N-(2-hydroxypropyl)imidazo[1,2-a]pyridine-6-carboxamide | 1-{[2-(4-Chlorophenyl)-3-(hydroxymethyl)imidazo[1,2-a]pyridin-6-yl]carbonyl}azetidin-3-ol |
| 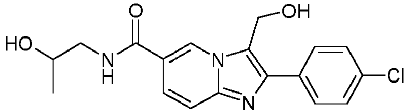 | 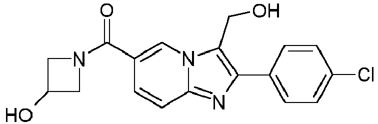 |
| 2-(4-Chlorophenyl)-N-(*trans*-4-hydroxycyclohexyl)-3-(hydroxymethyl)imidazo[1,2-a]pyridine-6-carboxamide | 2-(4-Chlorophenyl)-3-(hydroxymethyl)imidazo[1,2-a]pyridine-7-carboxamide |
| 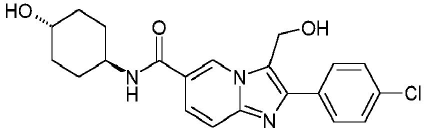 | 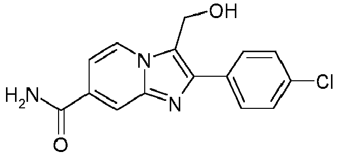 |
| 3-(Hydroxymethyl)-2-(3-methoxyphenyl)imidazo[1,2-a]pyridine-6-carboxamide | [6-amino-2-(4-chlorophenyl)imidazo[1,2-a]pyridin-3-yl]methanol |
| 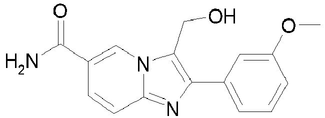 | 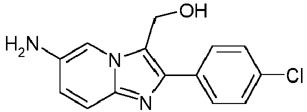 |
| 2-(4-Chlorophenyl)-3-(hydroxymethyl)imidazo[1,2-a]pyridine-6-carboxamide | N-[2-(4-chlorophenyl)-3-(hydroxymethyl)imidazo[1,2-a]pyridin-6-yl]acetamide |
| 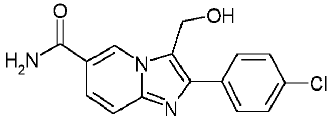 | 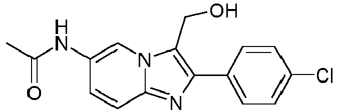 |
| -(4-Fluorophenyl)-3-(hydroxymethyl)imidazo[1,2-a]pyridine-6-carboxamide | [6-amino-2-(4-bromophenyl)imidazo[1,2-a]pyridin-3-yl]methanol |
| | 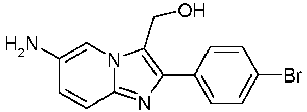 |
| 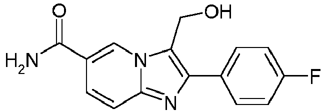 | [6-chloro-2-(4-chlorophenyl)imidazo[1,2-b]pyridazin-3-yl]methanol |
| 2-(2,4-Difluorophenyl)-3-(hydroxymethyl)imidazo[1,2-a]pyridine-6-carboxamide | 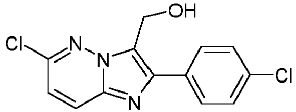 |
| 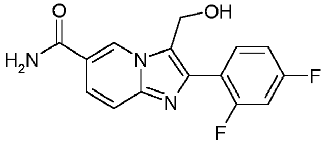 | [2-(4-Chlorophenyl)imidazo[1,2-a]pyrazin-3-yl]methanol |
| | 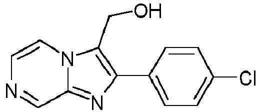 |
| 2-(2,4-Dichlorophenyl)-3-(hydroxymethyl)imidazo[1,2-a]pyridine-6-carboxamide | [6-Bromo-2-(3-methoxyphenyl)imidazo[1,2-a]pyrazin-3-yl]methanol |
| 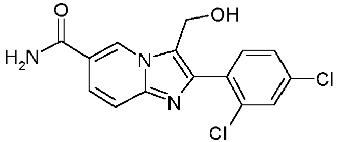 | 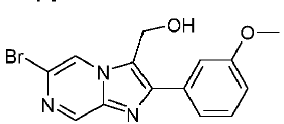 |
| 2-(3,4-Difluorophenyl)-3-(hydroxymethyl)imidazo[1,2-a]pyridine-6-carboxamide | {6-Bromo-2-[4-(trifluoromethyl)phenyl]imidazo[1,2-a]pyrazin-3-yl}methanol |
| 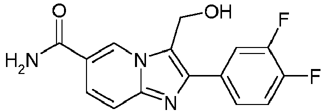 | 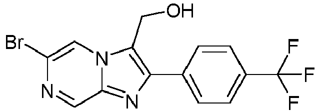 |
| [6-Bromo-2-(4-fluorophenyl)imidazo[1,2-a]pyrazin-3-yl]methanol | [2-(1-Benzofuran-5-yl)-6-bromoimidazo[1,2-a]pyrazin-3-yl]methanol |
| 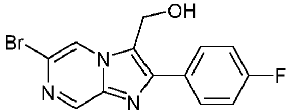 | 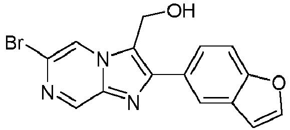 |
| [6-Bromo-2-(4-chlorophenyl)imidazo[1,2-a]pyrazin-3-yl]methanol | [6-Bromo-2-(2,3-dihydro-1,4-benzodioxin-5-yl)imidazo[1,2-a]pyrazin-3-yl]methanol |
| 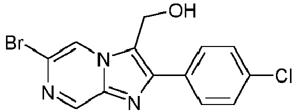 | 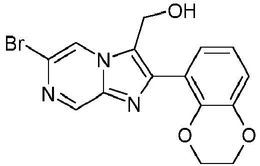 |
| [6-Bromo-2-(4-bromophenyl)imidazo[1,2-a]pyrazin-3-yl]methanol | |
| 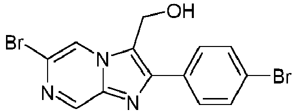 | [6-amino-2-(4-fluorophenyl)imidazo[1,2-a]pyrazin-3-yl]methanol |
| [6-Bromo-2-(2,4-dichlorophenyl)imidazo[1,2-a]pyrazin-3-yl]methanol | 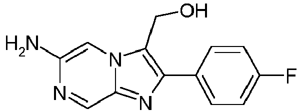 |
| 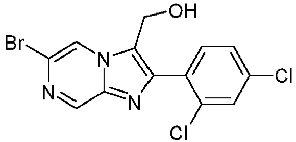 | [6-amino-2-(4-chlorophenyl)imidazo[1,2-a]pyrazin-3-yl]methanol |
| [6-Bromo-2-(2,4-difluorophenyl)imidazo[1,2-a]pyrazin-3-yl]methanol | 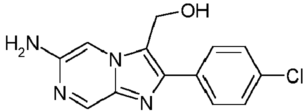 |
| 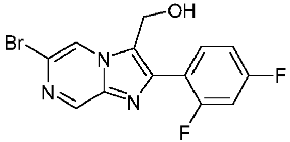 | [6-Amino-2-(4-bromophenyl)imidazo[1,2-a]pyrazin-3-yl]methanol |
| [6-Bromo-2-(4-chloro-2-fluoro-5-methylphenyl)imidazo[1,2-a]pyrazin-3-yl]methanol | 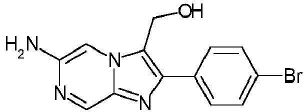 |
| 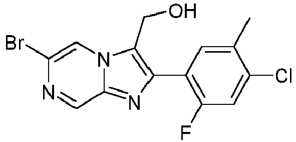 | [6-(Azetidin-1-yl)-2-(4-fluorophenyl)imidazo[1,2-a]pyrazin-3-yl]methanol |
| | 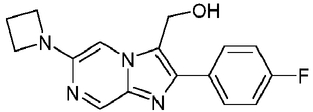 |
| [2-(4-Chlorophenyl)imidazo[1,2-a]pyrimidin-3-yl]methanol | [6-(2,4-Dichlorophenyl)-2-methylimidazo[2,1-b][1,3]thiazol-5-yl]methanol |
| 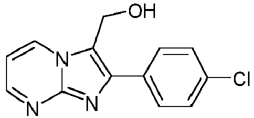 | 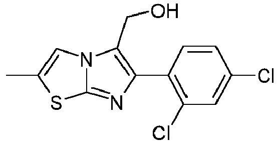 |
| [2-(2,4-Dichlorophenyl)imidazo[1,2-a]pyrimidin-3-yl]methanol | [2-Chloro-6-(4-chlorophenyl)imidazo[2,1-b][1,3]thiazol-5-yl]methanol |
| 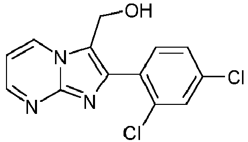 | 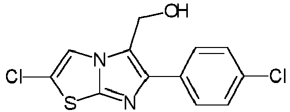 |
| [6-(4-fluorophenyl)-2-methylimidazo[2,1-b][1,3]oxazol-5-yl]methanol | Methyl 6-(4-chlorophenyl)-5-(hydroxymethyl)imidazo[2,1-b][1,3]thiazole-2-carboxylate |
| 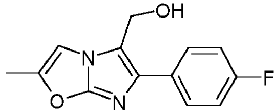 | 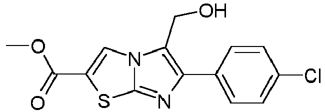 |
| [6-(4-Chlorophenyl)imidazo[2,1-b][1,3]thiazol-5-yl]methanol | [6-(4-Chlorophenyl)imidazo[2,1-b][1,3]thiazole-2,5-diyl]dimethanol |
| 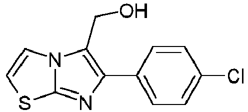 | 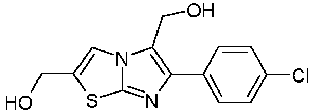 |
| [6-(4-Bromophenyl)imidazo[2,1-b][1,3]thiazol-5-yl]methanol | 1-[6-(4-Chlorophenyl)-5-(hydroxymethyl)imidazo[2,1-b][1,3]thiazol-2-yl]ethanol |
| 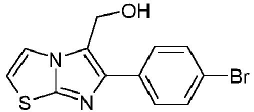 | |
| [6-(2,4-dichlorophenyl)imidazo[2,1-b][1,3]thiazol-5-yl]methanol | 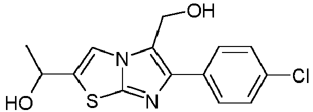 |
| 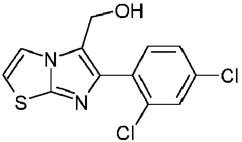 | [6-(4-Chlorophenyl)-5-(hydroxymethyl)imidazo[2,1-b][1,3]thiazol-2-yl](cyclopropyl)methanol |
| [6-(4-Bromophenyl)-2-methylimidazo[2,1-b][1,3]thiazol-5-yl]methanol | 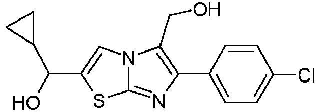 |
| 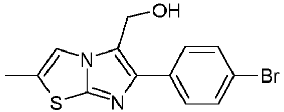 | |
| 2-[6-(4-Chlorophenyl)-5-(hydroxymethyl)imidazo[2,1-b][1,3]thiazol-2-yl]propan-2-ol | 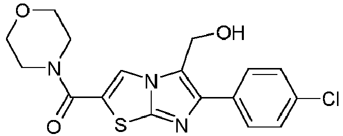 |
| 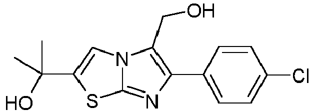 | {6-(4-Chlorophenyl)-2-[(4-methylpiperazin-1-yl)carbonyl]imidazo[2,1-b][1,3]thiazol-5-yl}methanol |
| 6-(4-Chlorophenyl)-N-ethyl-5-(hydroxymethyl)-N-methylimidazo[2,1-b][1,3]thiazole-2-carboxamide | 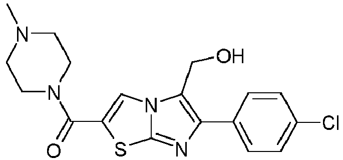 |
| 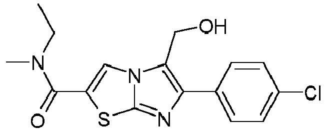 | |
| [6-(4-Chlorophenyl)-2-(morpholin-4-ylcarbonyl)imidazo[2,1-b][1,3]thiazol-5-yl]methanol | 6-(4-Chlorophenyl)-5-(hydroxymethyl)-N-propylimidazo[2,1-b][1,3]thiazole-2-carboxamide |
| | 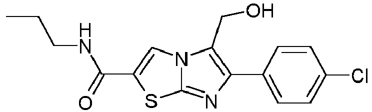 |

| Further specific Examples of VAP-1 compounds include: | |
|---|---|
| *tert*-Butyl *N*-(3-{4-[1-(4-chlorophenyl)-1*H-*pyrrolo[2,3-*c*]pyridin-3-yl]piperidin-1-yl}-3-oxopropyl)carbamate | 1-{4-[1-(4-Chlorophenyl)-1*H*-pyrrolo[2,3-c]pyridine-3-yl]piperidin-1-yl}-2-hydroxyethan-1-one |
| 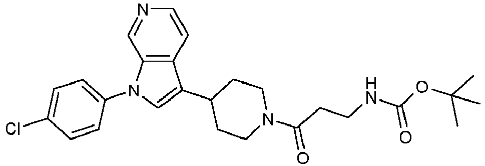 | 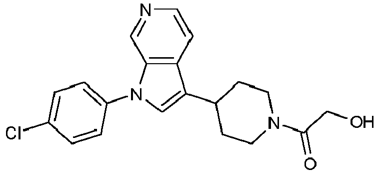 |
| 1-{4-[1-(4-Chlorophenyl)-1 *H*-pyrrolo[2,3-*c*]pyridin-3-yl]piperidin-1-yl}-2-(dimethyl-amino)ethan-1-one | 2-Amino-1-{4-[1-(4-chlorophenyl)-1 *H-*pyrrolo[2,3-c]pyridin-3-yl]piperidin-1-yl}ethan-1-one |
| 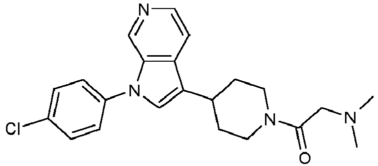 | 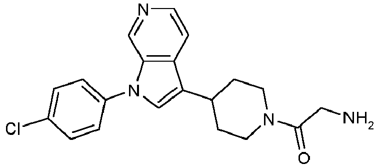 |
| 3-Amino-1-{4-[1-(4-chlorophenyl)-1 *H-*pyrrolo[2,3-c]pyridin-3-yl]piperidin-1-yl}propan-1-one | 4-{4-[1-(4-Chlorophenyl)-1 *H*-pyrrolo[2,3-*c*]pyridin-3-yl]piperidin-1-yl}butanenitrile |
| 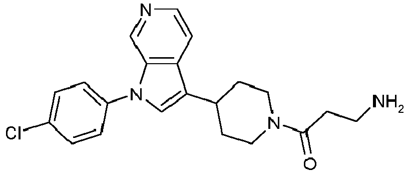 | 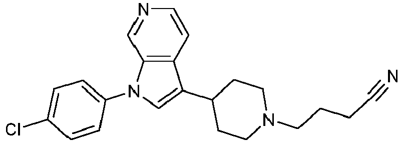 |
| 2-{4-[1-(4-Chlorophenyl)-1 *H*-pyrrolo[2,3-*c*]pyridin-3-yl]piperidin-1-yl}ethan-1-ol | [1-(4-Chlorophenyl)-1 *H*-pyrrolo[2,3-c]pyridin-3-yl]methanol |
| 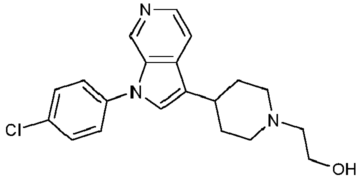 | 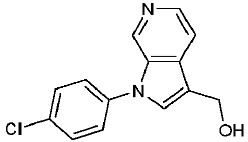 |
| 4-[1-(4-Chlorophenyl)-1*H*-pyrrolo[2,3-*c*]pyridin-3-yl]-1-(1*H*-pyrazol-3-ylmethyl)piperidine | 1-(4-Chlorophenyl)-1 *H*-pyrrolo[2,3-c]pyridine-3-carbaldehyde |
| 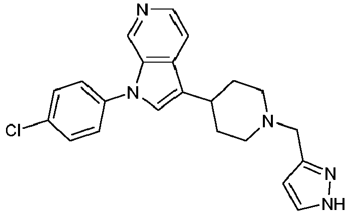 | 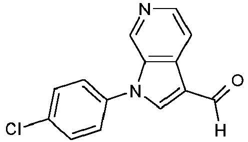 |
| | 1-{[1-(4-Chlorophenyl)-1 *H*-pyrrolo[2,3-*c*]pyridin-3-yl]methyl}-4-methylpiperazine |
| 4-[1-(4-Chlorophenyl)-1 *H*-pyrrolo[2,3-c]pyridin-3-yl]-1-[(1-methyl-1 *H*-pyrazol-4-yl)-methyl]piperidine | 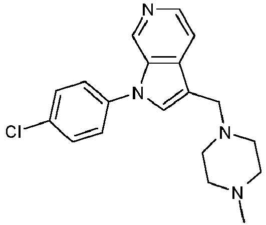 |
| 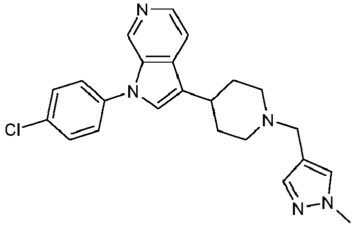 | *tert*-Butyl 4-{[1-(4-chlorophenyl)-1 *H-*pyrrolo[2,3-*c*]pyridin-3-yl] |
| 3-{4-[1-(4-Chlorophenyl)-1 *H*-pyrrolo[2,3-c]pyridine-3-yl]piperidin-1-yl}propanenitrile | 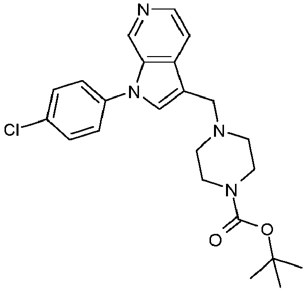 |
| 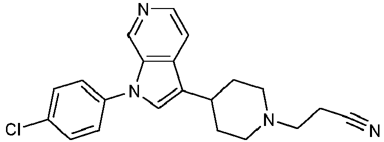 | |
| 1-{[1-(4-Chlorophenyl)-1*H*-pyrrolo[2,3-c]pyridin-3-yl]methyl}piperazine | 1-{[1-(4-Chlorophenyl)-1 *H*-pyrrolo[2,3-c]pyridin-3-yl]methyl}piperidin-4-ol |
| 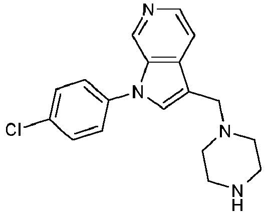 | 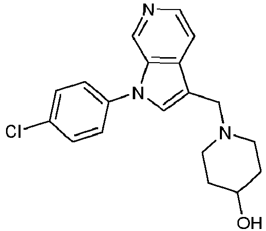 |
| 2-(1-{[1-(4-Chlorophenyl)-1 *H*-pyrrolo[2,3-*c*]pyridin-3-yl]methyl}piperidin-4-yl)ethan-1-ol | 2-({[1-(4-Chlorophenyl)-1 *H*-pyrrolo[2,3-*c*]pyridin-3-yl]methyl}amino)ethan-1-ol |
| 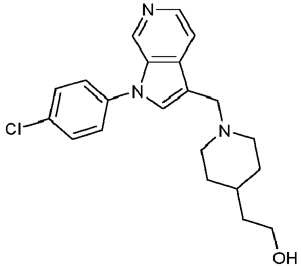 | 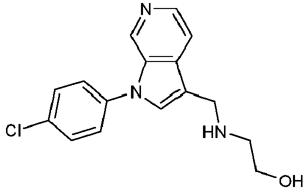 |
| (1-{[1-(4-Chlorophenyl)-1 *H*-pyrrolo[2,3-c]pyridin-3-yl]methyl}piperidin-4-yl)methanol | 4-[3-(4-Methylphenyl)imidazo[1,5-a]pyrazin-1 - yl]morpholine |
| 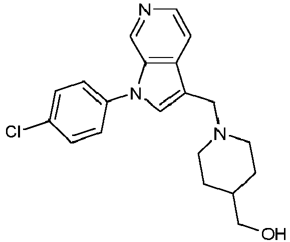 | 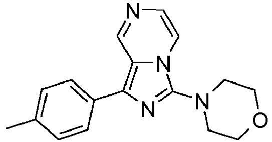 |
| | 4-[3-(4-Chlorophenyl)imidazo[1,5-a]pyrazin-1 - yl]morpholine |
| | 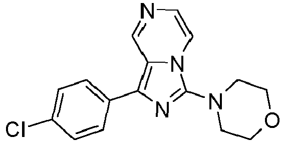 |
| 4-{[1-(4-Chlorophenyl)-1 *H*-pyrrolo[2,3-*c*]pyrid in-3-yl]methyl}morpholine | 3-(4-Chlorophenyl)-N-(2-methoxyethyl)-N-methylimidazo[1,5-a]pyrazin-1-amine |
| 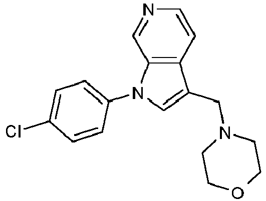 | 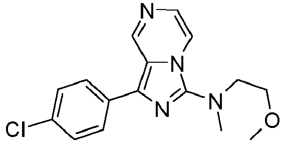 |
| 3-(4-Chlorophenyl)-N,N-dimethylimidazo[1,5-a]pyrazin-1-amine | 4-[3-(4-Methylphenyl)-4H,5H,6H,7H-imidazo[1,5-a]pyrazin-1-yl]morpholine |
| 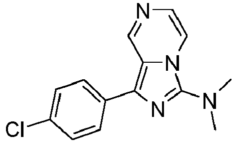 | 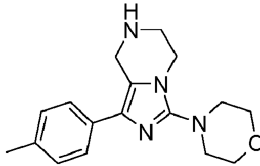 |
| 3-(4-Chlorophenyl)-1-(oxan-4-yl)imidazo[1,5-a]pyrazine | 1-[3-(4-Methylphenyl)-1-(morpholin-4-yl)-4H,5H,6H,7H-imidazo[1,5-a]pyrazin-5-yl]ethan-1-one |
| 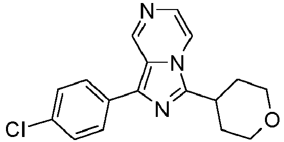 | 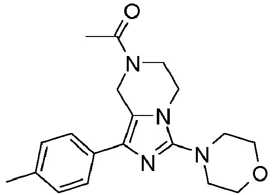 |
| 3-(4-Chlorophenyl)-1-(oxan-4-ylmethyl)imidazo[1,5-a]pyrazine | |
| 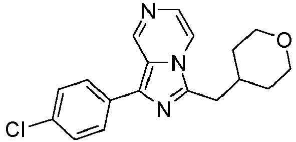 | 1-[3-(4-Methylphenyl)-1-(morpholin-4-yl)-4H,5H,6H,7H-imidazo[1,5-a]pyrazin-5-yl]propan-1-one |
| 3-(4-Chlorophenyl)-1-(oxolan-3-yl)imidazo[1,5-a]pyrazine | 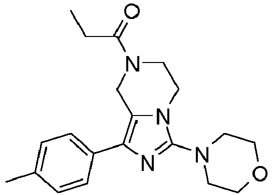 |
| 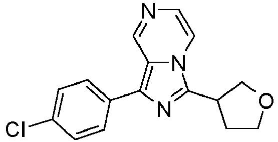 | |
| 3-(4-Chlorophenyl)-1-(4-methoxycyclohexyl)imidazo[1,5-a]pyrazine | Methyl 3-(4-methylphenyl)-1-(morpholin-4-yl)-4H,5H,6H,7H-imidazo[1,5-a]pyrazine-5-carboxylate |
| 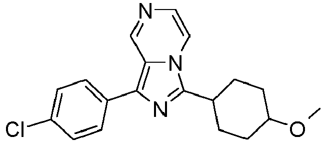 | 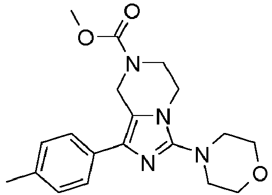 |
| 4-[3-(4-Chlorophenyl)-4H,5H,6H,7H-imidazo[1,5-a]pyrazin-1-yl]morpholine | |
| 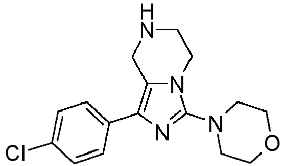 | |
| 2,2,2-Trifluoro-1-[3-(4-methylphenyl)-1-(morpholin-4-yl)-4H,5H,6H,7H-imidazo[1,5-a]pyrazin-5-yl]ethan-1-one | |
| 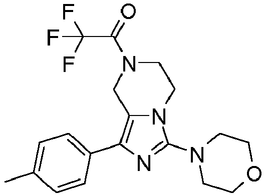 | |

Further specific Examples of VAP-1 inhibitor compounds suitable for use in the poresent invention are provided below. Any pharmaceutically acceptable salt form of the Examples is suitable for use in the present invention. Specific examples of inhibitors of VAP-1 include:
the substituted 3-haloallylamine inhibitors specifically disclosed as Examples in WO 2013/163675, in particular compounds 1-39 in Table 1 of that document;
the IMIDAZO[4,5-C]PYRIDINE AND PYRROLO[2,3-C]PYRIDINE DERIVATIVES specifically disclosed as Examples in WO2014/140592, namely:

### 3-[3-(4-Chlorophenyl)-3H-imidazo[4,5-c]pyridin-2-yl]pyridine

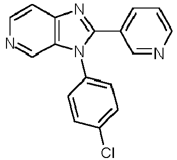

| **Structure** | **Name** |
|---|---|
| 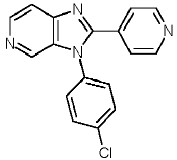 | 4-[3-(4-Chlorophenyl)-3H-imidazo[4,5-c]pyridin-2-yl]pyridine |
| 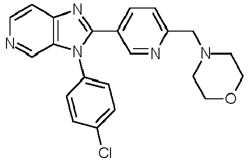 | 4-({5-[3-(4-Chlorophenyl)-3H-imidazo[4,5-c]pyridin-2-yl]pyridin-2-yl}methyl)morpholine |
| 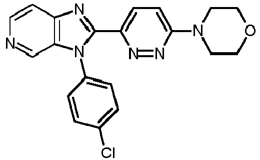 | 4-{6-[3-(4-Chlorophenyl)-3H-imidazo[4,5-c]pyridin-2-yl]pyridazin-3-yl}morpholine |
| 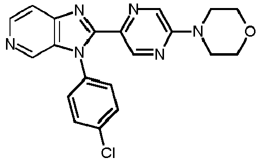 | 4-{5-[3-(4-Chlorophenyl)-3H-imidazo[4,5-c]pyridin-2-yl]pyrazin-2-yl}morpholine |
| 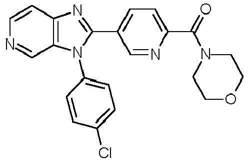 | 4-({5-[3-(4-Chlorophenyl)-3H-imidazo[4,5-c]pyridin-2-yl]pyridin-2-yl}carbonyl)morpholine |
| 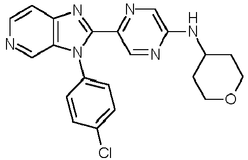 | 5-[3-(4-Chlorophenyl)-3H-imidazo[4,5-c]pyridin-2-yl]-*N-*(oxan-4-yl)pyrazin-2-amine |
| 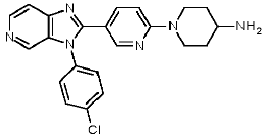 | 1-{5-[3-(4-Chlorophenyl)-3H-imidazo[4,5-c]pyridin-2-yl]pyridin-2-yl}piperidin-4-amine |
| 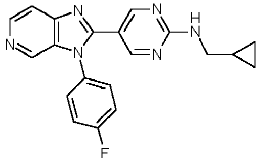 | *N*-(Cyclopropylmethyl)-5-[3-(4-fluorophenyl)-3H-imidazo[4,5-c]pyridin-2-yl]pyrimidin-2-amine |
| 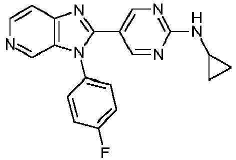 | *N*-Cyclopropyl-5-[3-(4-fluorophenyl)-3H-imidazo[4,5-c]pyridin-2-yl]pyrimidin-2-amine |
| 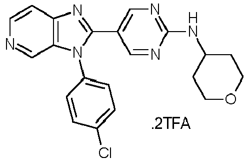 | 5-[3-(4-Chlorophenyl)-3H-imidazo[4,5-c]pyridin-2-yl]-*N-*(oxan-4-yl)pyrimid in-2-amine; bis(trifluoroacetic acid) |
| 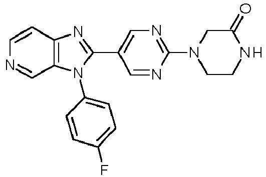 | 4-{5-[3-(4-Fluorophenyl)-3H-imidazo[4,5-c]pyridin-2-yl]pyrimidin-2-yl}piperazin-2-one |
| 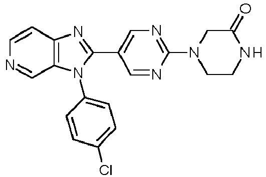 | 4-{5-[3-(4-Chlorophenyl)-3H-imidazo[4,5-c]pyridin-2-yl]pyrimidin-2-yl}piperazin-2-one |
| 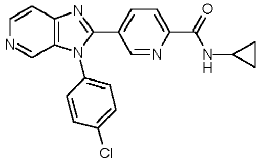 | 5-[3-(4-Chlorophenyl)-3H-imidazo[4,5-c]pyridin-2-yl]-*N-*cyclopropylpyridine-2-carboxamide |
| 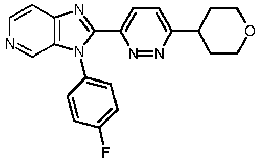 | 3-[3-(4-Fluorophenyl)-3H-imidazo[4,5-c]pyridin-2-yl]-6-(oxan-4-yl)pyridazine |
| 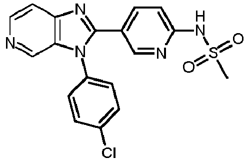 | *N*-{5-[3-(4-Chlorophenyl)-3H-imidazo[4,5-c]pyridin-2-yl]pyridin-2-yl}methanesulfonamide |
| 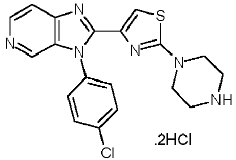 | 1-{4-[3-(4-Chlorophenyl)-3H-imidazo[4,5-c]pyridin-2-yl]-1,3-thiazol-2-yl}piperazine dihydrochloride |
| 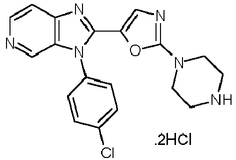 | 1-{5-[3-(4-Chlorophenyl)-3H-imidazo[4,5-c]pyridin-2-yl]-1,3-oxazol-2-yl}piperazine dihydrochloride |
| 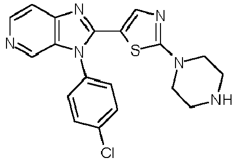 | 1-{5-[3-(4-Chlorophenyl)-3H-imidazo[4,5-c]pyridin-2-yl]-1,3-thiazol-2-yl}piperazine |
| 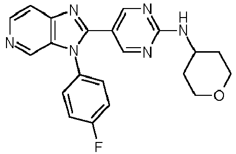 | 5-[3-(4-Fluorophenyl)-3H-imidazo[4,5-c]pyridin-2-yl]-*N-*(oxan-4-yl)pyrimid in-2-amine |
| 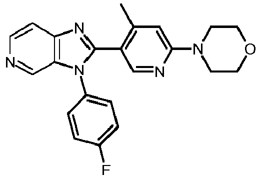 | 4-{5-[3-(4-Fluorophenyl)-3H-imidazo[4,5-c]pyridin-2-yl]-4-methylpyridin-2-yl}morpholine |
| 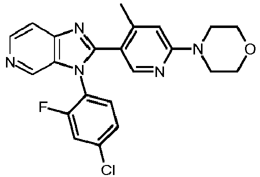 | 4-{5-[3-(4-Chloro-2-fluorophenyl)-3H-imidazo[4,5-c]pyridin-2-yl]-4-methylpyridin-2-yl}morpholine |
| 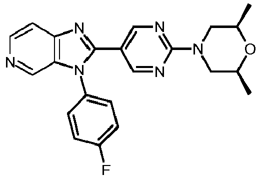 | (2R,6S)-4-{5-[3-(4-Fluorophenyl)-3H-imidazo[4,5-c]pyridin-2-yl]pyrimidin-2-yl}-2,6-dimethylmorpholine |
| 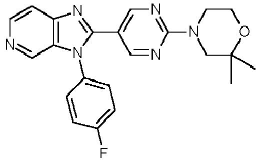 | 4-{5-[3-(4-Fluorophenyl)-3H-imidazo[4,5-c]pyridin-2-yl]pyrimidin-2-yl}-2,2-dimethyl morpho line |
| 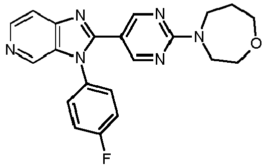 | 4-{5-[3-(4-Fluorophenyl)-3H-imidazo[4,5-c]pyridin-2-yl]pyrimidin-2-yl}-1,4-oxazepane |
| 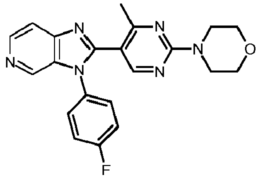 | 4-{5-[3-(4-Fluorophenyl)-3H-imidazo[4,5-c]pyridin-2-yl]-4-methylpyrimidin-2-yl}morpholine |
| 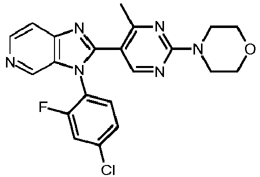 | 4-{5-[3-(4-Chloro-2-fluorophenyl)-3H-imidazo[4,5-c]pyridin-2-yl]-4-methylpyrimidin-2-yl}morpholine |
| 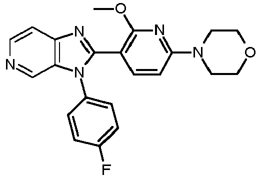 | 4-{5-[3-(4-Fluorophenyl)-3H-imidazo[4,5-c]pyridin-2-yl]-6-methoxypyridin-2-yl}morpholine |
| 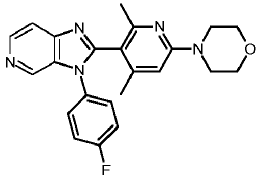 | 4-{5-[3-(4-Fluorophenyl)-3H-imidazo[4,5-c]pyridin-2-yl]-4,6-dimethylpyridin-2-yl}morpholine |
| 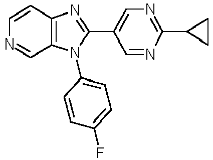 | 2-Cyclopropyl-5-[3-(4-fluorophenyl)-3H-imidazo[4,5-c]pyridin-2-yl]pyrimidine |
| 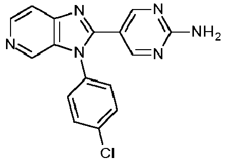 | 5-[3-(4-Chlorophenyl)-3H-imidazo[4,5-c]pyridin-2-yl]pyrimidin-2-amine |
| 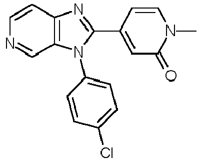 | 4-[3-(4-Chlorophenyl)-3H-imidazo[4,5-c]pyridin-2-yl]-1-methyl-1,2-dihydropyridin-2-one |
| 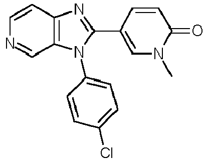 | 5-[3-(4-Chlorophenyl)-3H-imidazo[4,5-c]pyridin-2-yl]-1-methyl-1,2-dihydropyridin-2-one |
| 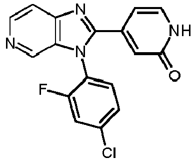 | 4-[3-(4-Chloro-2-fluorophenyl)-3H-imidazo[4,5-c]pyridin-2-yl]-1,2-dihydropyridin-2-one |
| 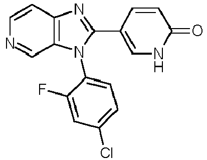 | 5-[3-(4-Chloro-2-fluorophenyl)-3H-imidazo[4,5-c]pyridin-2-yl]-1,2-dihydropyridin-2-one |
| 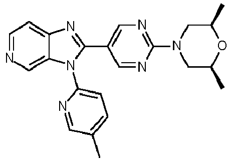 | (2R,6S)-2,6-Dimethyl-4-{5-[3-(5-methylpyridin-2-yl)-3H-imidazo[4,5-c]pyridin-2-yl]pyrimidin-2-yl}morpholine |
| 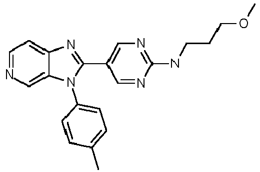 | *N*-(3-Methoxypropyl)-5-[3-(4-methylphenyl)-3H-imidazo[4,5-c]pyrid in-2-yl]pyri mid in-2-amine |
| 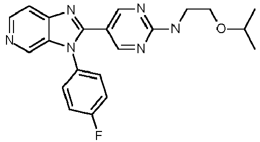 | 5-[3-(4-Fluorophenyl)-3H-imidazo[4,5-c]pyridin-2-yl]-*N*-[2-(propan-2-yloxy)ethyl]pyrimidin-2-amine |
| 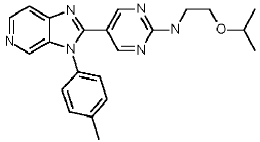 | 5-[3-(4-Methylphenyl)-3H-imidazo[4,5-c]pyridin-2-yl]-*N*-[2-(propan-2-yloxy)ethyl]pyrimidin-2-amine |
| 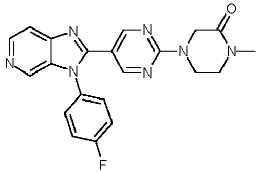 | 4-{5-[3-(4-Fluorophenyl)-3H-imidazo[4,5-c]pyridin-2-yl]pyrimidin-2-yl}-1-methylpiperazin-2-one |
| 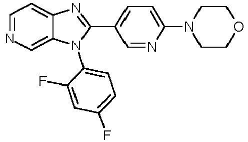 | 4-{5-[3-(2,4-Difluorophenyl)-3H-imidazo[4,5-c]pyridin-2-yl]pyridin-2-yl}morpholine |
| 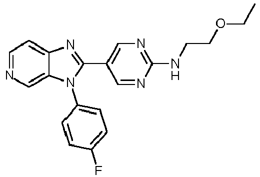 | *N*-(2-Ethoxyethyl)-5-[3-(4-fluorophenyl)-3H-imidazo[4,5-c]pyridin-2-yl]pyrimidin-2-amine |
| 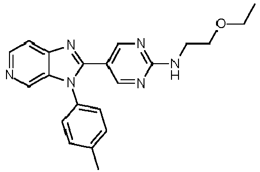 | *N*-(2-Ethoxyethyl)-5-[3-(4-methylphenyl)-3H-imidazo[4,5-c]pyridin-2-yl]pyrimidin-2-amine |

### 5-[3-(4-Chlorophenyl)-3H-imidazo[4,5-c]pyridin-2-yl]-1H-imidazole

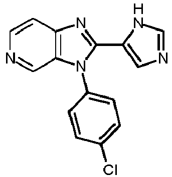

| **Structure** | **Name** |
|---|---|
| 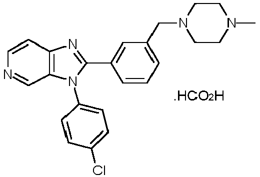 | 1-({3-[3-(4-Chlorophenyl)-3H-imidazo[4,5-c]pyridin-2-yl]phenyl}methyl)-4-methylpiperazine; formic acid |
| 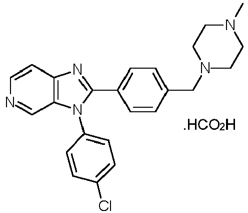 | 1-({4-[3-(4-Chlorophenyl)-3H-imidazo[4,5-c]pyridin-2-yl]phenyl}methyl)-4-methylpiperazine; formic acid |
| 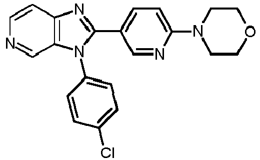 | 4-{5-[3-(4-Chlorophenyl)-3H-imidazo[4,5-c]pyridin-2-yl]pyridin-2-yl}morpholine |
| 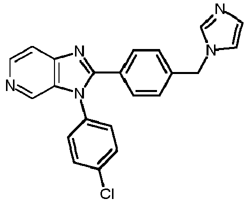 | 1-({4-[3-(4-Chlorophenyl)-3H-imidazo[4,5-c]pyridin-2-yl]phenyl}methyl)-1 H-imidazole |
| 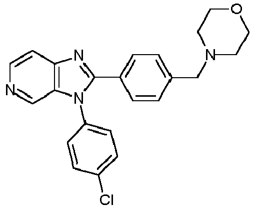 | 4-({4-[3-(4-Chlorophenyl)-3H-imidazo[4,5-c]pyridin-2-yl]phenyl}methyl)morpholine |
| 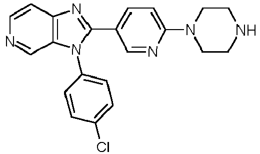 | 1-{5-[3-(4-Chlorophenyl)-3H-imidazo[4,5-c]pyridin-2-yl]pyridin-2-yl}piperazine |
| 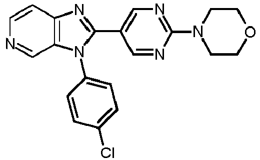 | 4-{5-[3-(4-Chlorophenyl)-3H-imidazo[4,5-c]pyridin-2-yl]pyrimidin-2-yl}morpholine |
| 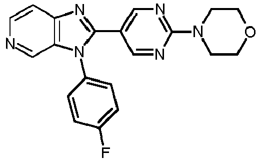 | 4-{5-[3-(4-Fluorophenyl)-3H-imidazo[4,5-c]pyridin-2-yl]pyrimidin-2-yl}morpholine |
| 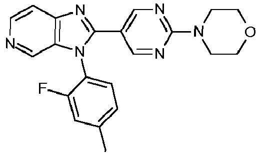 | 4-{5-[3-(2-Fluoro-4-methylphenyl)-3H-imidazo[4,5-c]pyridin-2-yl]pyrimidin-2-yl}morpholine |
| 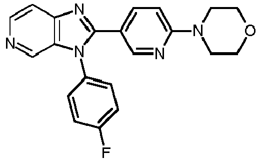 | 4-{5-[3-(4-Fluorophenyl)-3H-imidazo[4,5-c]pyridin-2-yl]pyridin-2-yl}morpholine |
| 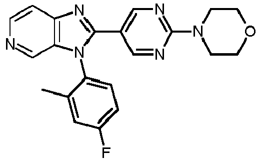 | 4-{5-[3-(4-Fluoro-2-methylphenyl)-3H-imidazo[4,5-c]pyridin-2-yl]pyrimidin-2-yl}morpholine |
| 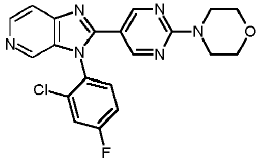 | 4-{5-[3-(2-Chloro-4-fluorophenyl)-3H-imidazo[4,5-c]pyridin-2-yl]pyrimidin-2-yl}morpholine |
| 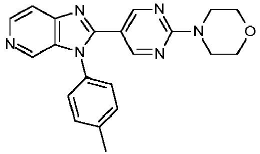 | 4-{5-[3-(4-Methylphenyl)-3H-imidazo[4,5-c]pyridin-2-yl]pyrimidin-2-yl}morpholine |
| 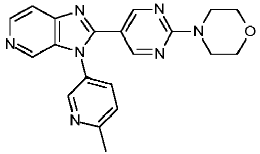 | 4-{5-[3-(6-Methylpyridin-3-yl)-3H-imidazo[4,5-c]pyridin-2-yl]pyrimidin-2-yl}morpholine |
| 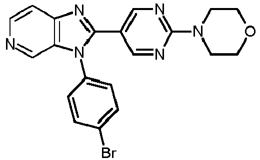 | 4-{5-[3-(4-Bromophenyl)-3H-imidazo[4,5-c]pyridin-2-yl]pyrimidin-2-yl}morpholine |
| 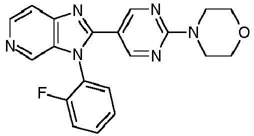 | 4-{5-[3-(2-Fluorophenyl)-3H-imidazo[4,5-c]pyridin-2-yl]pyrimidin-2-yl}morpholine |
| 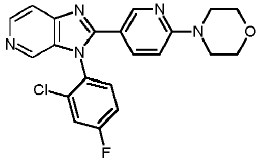 | 4-{5-[3-(2-Chloro-4-fluorophenyl)-3H-imidazo[4,5-c]pyridin-2-yl]pyridin-2-yl}morpholine |
| 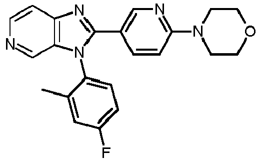 | 4-{5-[3-(4-Fluoro-2-methylphenyl)-3H-imidazo[4,5-c]pyridin-2-yl]pyrid in-2-yl}morpholine |
| 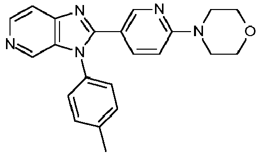 | 4-{5-[3-(4-Methylphenyl)-3H-imidazo[4,5-c]pyridin-2-yl]pyridin-2-yl}morpholine |
| 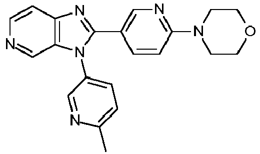 | 4-{5-[3-(6-Methylpyridin-3-yl)-3H-imidazo[4,5-c]pyridin-2-yl]pyridin-2-yl}morpholine |
| 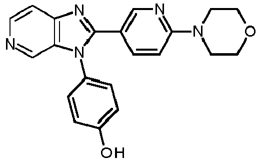 | 4-{2-[6-(Morpholin-4-yl)pyrid in-3-yl]-3H-imidazo[4,5-c]pyridin-3-yl}phenol |
| 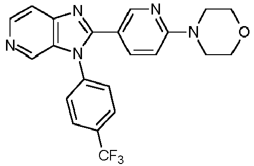 | 4-(5-{3-[4-(Trifluoromethyl)phenyl]-3H-imidazo[4,5-c]pyridin-2-yl}pyridin-2-yl)morpholine |
| 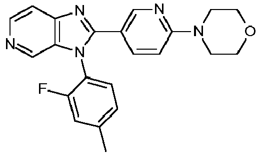 | 4-{5-[3-(2-Fluoro-4-methylphenyl)-3H-imidazo[4,5-c]pyridin-2-yl]pyridin-2-yl}morpholine |
| 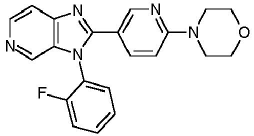 | 4-{5-[3-(2-Fluorophenyl)-3H-imidazo[4,5-c]pyridin-2-yl]pyridin-2-yl}morpholine |
| 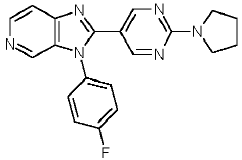 | 5-[3-(4-Fluorophenyl)-3H-imidazo[4,5-c]pyridin-2-yl]-2-(pyrrolidin-1-yl)pyrimidine |
| 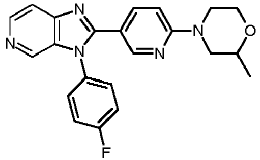 | 4-{5-[3-(4-Fluorophenyl)-3H-imidazo[4,5-c]pyridin-2-yl]pyridin-2-yl}-2-methylmorpholine |
| 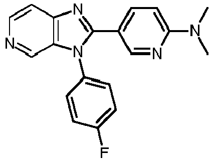 | 5-[3-(4-Fluorophenyl)-3H-imidazo[4,5-c]pyridin-2-yl]-*N,N-*dimethylpyridin-2-amine |
| 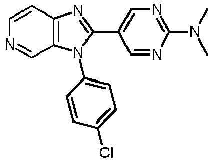 | 5-[3-(4-Chlorophenyl)-3H-imidazo[4,5-c]pyridin-2-yl]-*N,N-*dimethylpyrimidin-2-amine |

| | |
|---|---|
| **4-{4-[3-(4-Fluorophenyl)-3H-imidazo[4,5-c]pyridin-2-yl]pyridin-2-yl}morpholine** | **4-{5-[3-(4-Ch loro-3-fluorophenyl)-3H-imidazo[4,5-c]pyridin-2-yl]pyrimidin-2-yl}morpholine** |
| 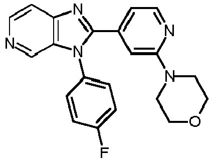 | 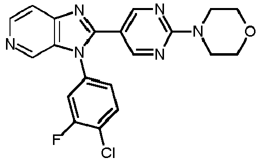 |

| **Structure** | **Name** |
|---|---|
| 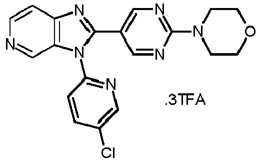 | 4-{5-[3-(5-Chloropyridin-2-yl)-3H-imidazo[4,5-c]pyridin-2-yl]pyrimidin-2-yl}morpholine; tris(trifluoroacetic acid) |
| 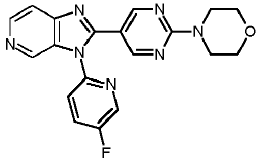 | 4-{5-[3-(5-Fluoropyridin-2-yl)-3H-imidazo[4,5-c]pyridin-2-yl]pyrimid in-2-yl}morpholine |
| 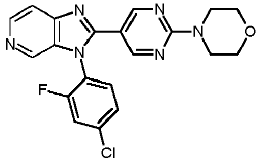 | 4-{5-[3-(4-Chloro-2-fluorophenyl)-3H-imidazo[4,5-c]pyridin-2-yl]pyrimidin-2-yl}morpholine |
| 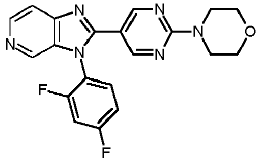 | 4-{5-[3-(2,4-Difluorophenyl)-3H-imidazo[4,5-c]pyridin-2-yl]pyrimidin-2-yl}morpholine |
| 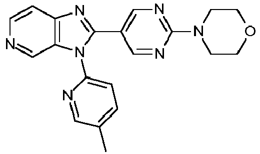 | 4-{5-[3-(5-Methylpyridin-2-yl)-3H-imidazo[4,5-c]pyridin-2-yl]pyrimidin-2-yl}morpholine |
| 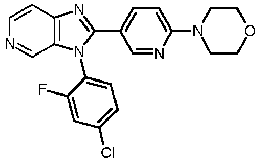 | 4-{5-[3-(4-Chloro-2-fluorophenyl)-3H-imidazo[4,5-c]pyridin-2-yl]pyridin-2-yl}morpholine |
| 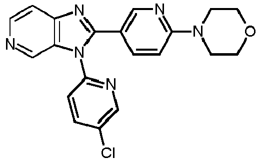 | 4-{5-[3-(5-Chloropyridin-2-yl)-3H-imidazo[4,5-c]pyridin-2-yl]pyridin-2-yl}morpholine |
| 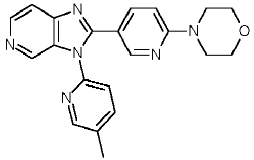 | 4-{5-[3-(5-Methylpyridin-2-yl)-3H-imidazo[4,5-c]pyridin-2-yl]pyridin-2-yl}morpholine |
| 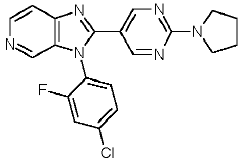 | 5-[3-(4-Chloro-2-fluorophenyl)-3H-imidazo[4,5-c]pyridin-2-yl]-2-(pyrrolidin-1-yl)pyrimidine |
| 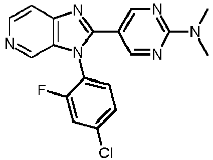 | 5-[3-(4-Chloro-2-fluorophenyl)-3H-imidazo[4,5-c]pyridin-2-yl]-*N,N*-dimethylpyrimidin-2-amine |

| | |
|---|---|
| *N*-(1-{5-[3-(4-Chlorophenyl)-3H-imidazo[4,5-c]pyridin-2-yl]pyridin-2-yl}piperidin-4-yl)acetamide | *N*-(1-{5-[3-(4-Chlorophenyl)-3H-imidazo[4,5-c]pyridin-2-yl]pyridin-2-yl}piperidin-4-yl)methanesulfonamide |
| 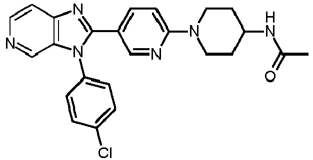 | 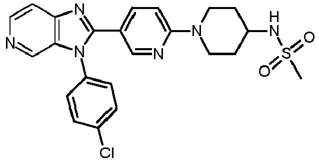 |
| 1-(4-{5-[3-(4-Fluorophenyl)-3H-imidazo[4,5-c]pyridin-2-yl]pyridin-2-yl}piperazin-1-yl)ethan-1-one | 1-{5-[3-(4-Fluorophenyl)-3H-imidazo[4,5-c]pyridin-2-yl]pyridin-2-yl}-4-methanesulfonylpiperazine |
| 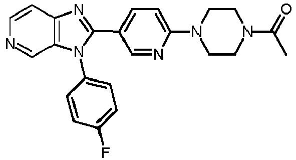 | 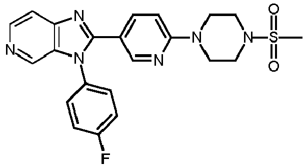 |
| 1-(4-{5-[3-(4-Fluorophenyl)-3H-imidazo[4,5-c]pyridin-2-yl]pyridin-2-yl}-1,4-diazepan-1-yl)ethan-1-one; bis(trifluoroacetic acid) | 4-{5-[3-(4-Chlorophenyl)-3H-imidazo[4,5-c]pyridin-2-yl]pyridin-2-yl}piperazine-1-carboxamide dihydrochloride |
| 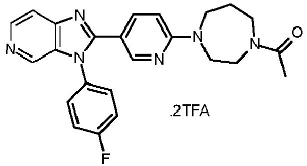 | 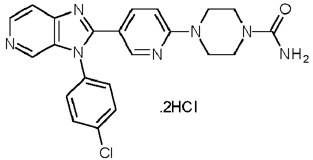 |

| Structure | Name |
|---|---|
| 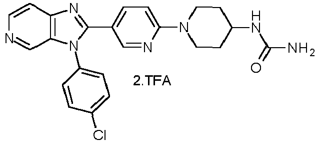 | (1-{5-[3-(4-Chlorophenyl)-3H-imidazo[4,5-c]pyridin-2-yl]pyridin-2-yl}piperidin-4-yl)urea; bis(trifluoroacetic acid) |
| 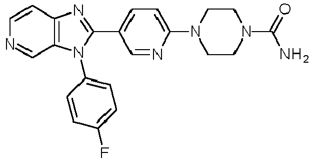 | 4-{5-[3-(4-Fluorophenyl)-3H-imidazo[4,5-c]pyridin-2-yl]pyridin-2-yl}piperazine-1-carboxamide |
| 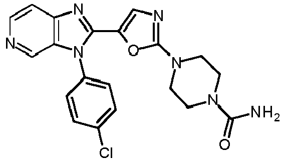 | 4-{5-[3-(4-Chlorophenyl)-3H-imidazo[4,5-c]pyridin-2-yl]-1,3-oxazol-2-yl}piperazine-1-carboxamide |
| 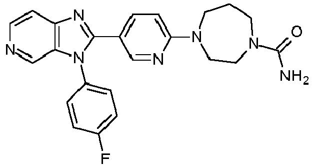 | 4-{5-[3-(4-Fluorophenyl)-3H-imidazo[4,5-c]pyridin-2-yl]pyridin-2-yl}-1,4-diazepane-1-carboxamide |

| | |
|---|---|
| 4-(5-{3-Phenyl-3H-imidazo[4,5-c]pyridin-2-yl}pyrimidin-2-yl)morpholine | 4-{5-[3-(4-Cyclopropylphenyl)-3H-imidazo[4,5-c]pyridin-2-yl]pyrimidin-2-yl}morpholine |
| 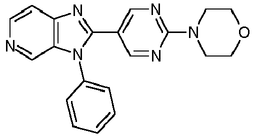 | 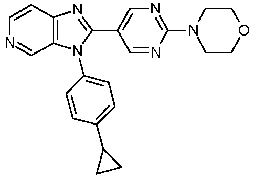 |
| | 4-{4-Methyl-5-[3-(4-methylphenyl)-3H-imidazo[4,5-c]pyridin-2-yl]pyridin-2-yl}morpholine |
| | 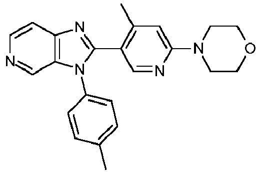 |

| Structure | Name |
|---|---|
| 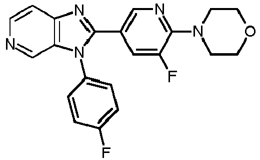 | 4-{3-Fluoro-5-[3-(4-fluorophenyl)-3H-imidazo[4,5-c]pyridin-2-yl]pyridin-2-yl}morpholine |
| 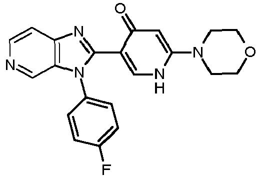 | 5-[3-(4-Fluorophenyl)-3H-imidazo[4,5-c]pyridin-2-yl]-2-(morpholin-4-yl)-1,4-dihydropyridin-4-one |
| 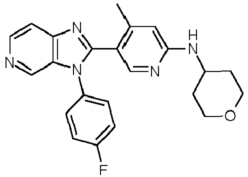 | 5-[3-(4-Fluorophenyl)-3H-imidazo[4,5-c]pyridin-2-yl]-4-methyl-*N*-(oxan-4-yl)pyridin-2-amine |
| 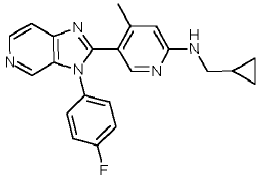 | *N*-(Cyclopropylmethyl)-5-[3-(4-fluorophenyl)-3H-imidazo[4,5-c]pyridin-2-yl]-4-methylpyridin-2-amine |
| 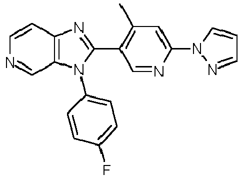 | 5-[3-(4-Fluorophenyl)-3H-imidazo[4,5-c]pyridin-2-yl]-4-methyl-2-(1 H-pyrazol-1-yl)pyridine |
| 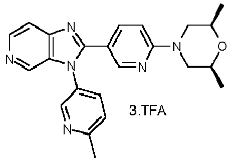 | (2R,6S)-2,6-Dimethyl-4-{5-[3-(6-methylpyridin-3-yl)-3H-imidazo[4,5-c]pyridin-2-yl]pyridin-2-yl}morpholine; tris(trifluoroacetic acid) |
| 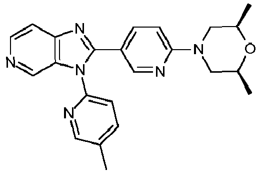 | (2R,6S)-2,6-Dimethyl-4-{5-[3-(5-methylpyridin-2-yl)-3H-imidazo[4,5-c]pyridin-2-yl]pyridin-2-yl}morpholine |
| 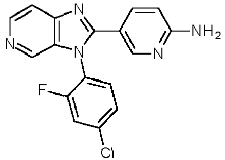 | 5-[3-(4-Chloro-2-fluorophenyl)-3H-imidazo[4,5-c]pyridin-2-yl]pyridin-2-amine |
| 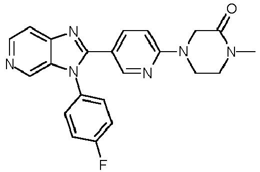 | 4-{5-[3-(4-Fluorophenyl)-3H-imidazo[4,5-c]pyridin-2-yl]pyridin-2-yl}-1-methylpiperazin-2-one |
| 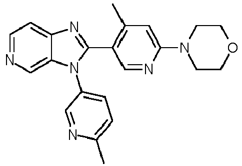 | 4-{4-Methyl-5-[3-(6-methylpyridin-3-yl)-3H-imidazo[4,5-c]pyridin-2-yl]pyrid in-2-yl}morpholine |

### 4-{5-[3-(4-Fluorophenyl)-3H-imidazo[4,5-c]pyridin-2-yl]-6-methylpyridin-2-yl}morpholine

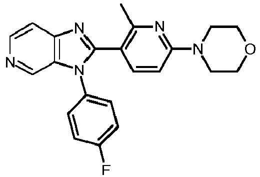

| Structure | Name |
|---|---|
| 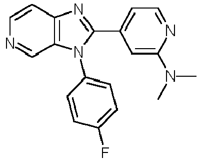 | 4-[3-(4-Fluorophenyl)-3H-imidazo[4,5-c]pyridin-2-yl]-*N,N-*dimethylpyridin-2-amine |
| 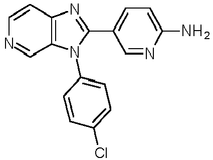 | 5-[3-(4-Chlorophenyl)-3H-imidazo[4,5-c]pyridin-2-yl]pyridin-2-amine |
| 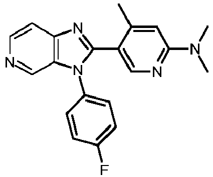 | 5-[3-(4-Fluorophenyl)-3H-imidazo[4,5-c]pyridin-2-yl]-*N,N*,4-trimethylpyridin-2-amine |

| | |
|---|---|
| 5-[3-(4-Fluorophenyl)-3H-imidazo[4,5-c]pyridin-2-yl]-2-(oxolan-3-yloxy)pyridine | 1-Cyclopropyl-4-[3-(4-fluorophenyl)-3H-imidazo[4,5-c]pyridin-2-yl]-1,2-dihydropyridin-2-one |
| 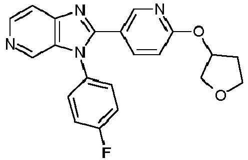 | 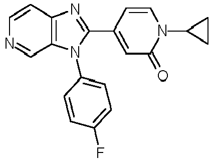 |
| 5-[3-(4-Fluorophenyl)-3H-imidazo[4,5-c]pyridin-2-yl]-2-(oxan-4-yloxy)pyridine | |
| 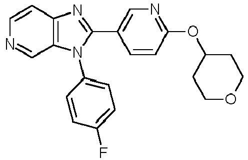 | 4-[3-(4-Chloro-2-fluorophenyl)-3H-imidazo[4,5-c]pyridin-2-yl]-1-cyclopropyl-1,2-dihydropyridin-2-one |
| 4-[3-(4-Fluorophenyl)-3H-imidazo[4,5-c]pyridin-2-yl]-1-methyl-1,2-dihydropyridin-2-one | 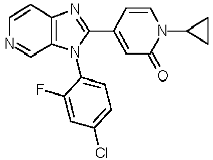 |
| 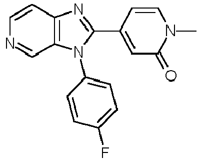 | *N*-(2-Methoxyethyl)-*N*-methyl-5-[3-(4-methylphenyl)-3H-imidazo[4,5-c]pyridin-2-yl]pyrimidin-2-amine |
| | 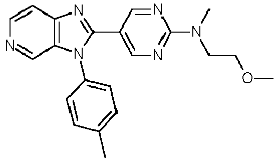 |

| Structure | Name |
|---|---|
| 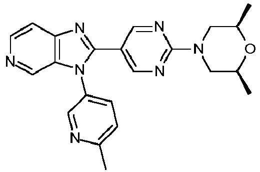 | (2R,6S)-2,6-Dimethyl-4-{5-[3-(6-methylpyridin-3-yl)-3H-imidazo[4,5-c]pyridin-2-yl]pyrimidin-2-yl}morpholine |
| 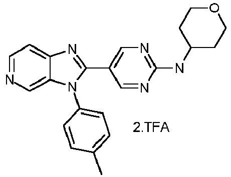 | 5-[3-(4-Methylphenyl)-3H-imidazo[4,5-c]pyridin-2-yl]-*N-*(oxan-4-yl)pyrimidin-2-amine; bis(trifluoroacetic acid) |

### 4-[1-(4-Chlorophenyl)-1H-pyrrolo[2,3-c]pyridin-2-yl]pyridine

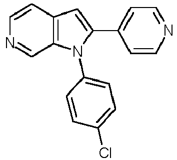

| Structure | Name |
|---|---|
| 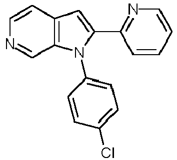 | 2-[1-(4-Chlorophenyl)-1 H-pyrrolo[2,3-c]pyridin-2-yl]pyridine |
| 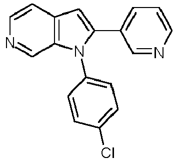 | 3-[1-(4-Chlorophenyl)-1 H-pyrrolo[2,3-c]pyridin-2-yl]pyridine |
| 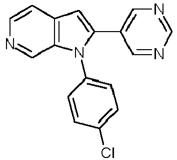 | 5-[1-(4-Chlorophenyl)-1 H-pyrrolo[2,3-c]pyridin-2-yl]pyrimidine |
| 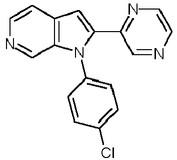 | 2-[1-(4-Chlorophenyl)-1 H-pyrrolo[2,3-c]pyridin-2-yl]pyrazine |
| 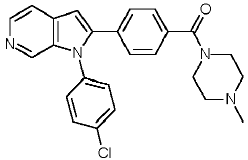 | 1-({4-[1-(4-Chlorophenyl)-1H-pyrrolo[2,3-c]pyridin-2-yl]phenyl}carbonyl)-4-methylpiperazine |
| 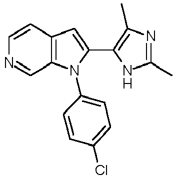 | 5-[1-(4-Chlorophenyl)-1 H-pyrrolo[2,3-c]pyridin-2-yl]-2,4-dimethyl-1H-imidazole |
| 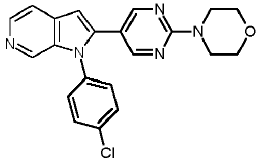 | 4-{5-[1-(4-Chlorophenyl)-1 H-pyrrolo[2,3-c]pyridin-2-yl]pyrimidin-2-yl}morpholine |
| 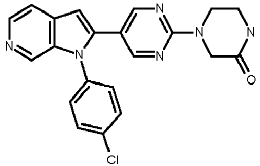 | 4-{5-[1-(4-Chlorophenyl)-1 H-pyrrolo[2,3-c]pyridin-2-yl]pyrimidin-2-yl}piperazin-2-one |
| 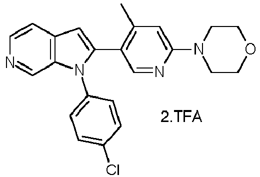 | 4-{5-[1-(4-Chlorophenyl)-1 H-pyrrolo[2,3-c]pyridin-2-yl]-4-methylpyridin-2-yl}morpholine; bis(trifluoroacetic acid) |
| 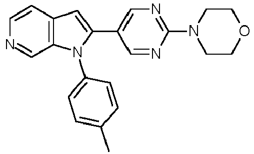 | 4-{5-[1-(4-Methylphenyl)-1 H-pyrrolo[2,3-c]pyridin-2-yl]pyrimidin-2-yl}morpholine |
| 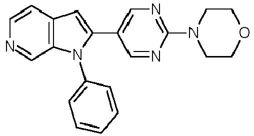 | 4-(5-{1-Phenyl-1 H-pyrrolo[2,3-c]pyridin-2-yl}pyrimidin-2-yl)morpholine |
| 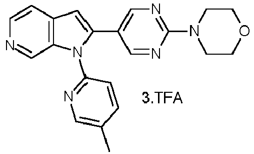 | 4-{5-[1-(5-Methylpyridin-2-yl)-1 H-pyrrolo[2,3-c]pyridin-2-yl]pyrimidin-2-yl}morpholine; tris(trifluoroacetic acid) |
| 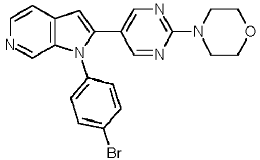 | 4-{5-[1-(4-Bromophenyl)-1 H-pyrrolo[2,3-c]pyridin-2-yl]pyrimidin-2-yl}morpholine |
| 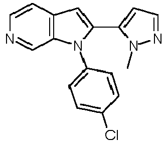 | 5-[1-(4-Chlorophenyl)-1 H-pyrrolo[2,3-c]pyridin-2-yl]-1-methyl-1 H-pyrazole |
| 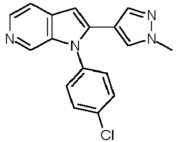 | 4-[1-(4-Chlorophenyl)-1 H-pyrrolo[2,3-c]pyridin-2-yl]-1-methyl-1 H-pyrazole |
| 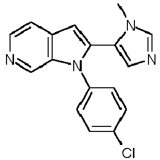 | 5-[1-(4-Chlorophenyl)-1 H-pyrrolo[2,3-c]pyridin-2-yl]-1-methyl-1 H-imidazole |
| 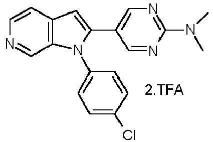 | 5-[1-(4-Chlorophenyl)-1 H-pyrrolo[2,3-c]pyridin-2-yl]-*N,N-*dimethylpyrimidin-2-amine; bis(trifluoroacetic acid) |
| 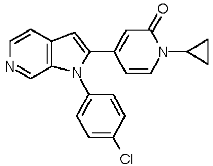 | 4-[1-(4-Chlorophenyl)-1 H-pyrrolo[2,3-c]pyridin-2-yl]-1-cyclopropyl-1,2-dihydropyridin-2-one |
| 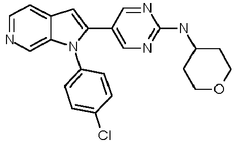 | 5-[1-(4-Chlorophenyl)-1 H-pyrrolo[2,3-c]pyridin-2-yl]-*N-*(oxan-4-yl)pyrimidin-2-amine |
| 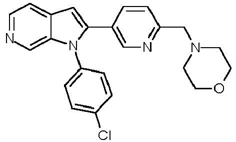 | 4-({5-[1-(4-Chlorophenyl)-1 H-pyrrolo[2,3-c]pyridin-2-yl]pyridin-2-yl}methyl)morpholine |
| 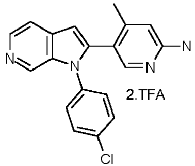 | 5-[1-(4-Chlorophenyl)-1 H-pyrrolo[2,3-c]pyridin-2-yl]-4-methylpyridin-2-amine; bis(trifluoroacetic acid) |
| 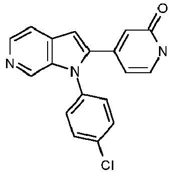 | 4-[1-(4-Chlorophenyl)-1 H-pyrrolo[2,3-c]pyridin-2-yl]-1,2-dihydropyridin-2-one |
| 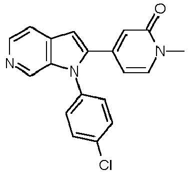 | 4-[1-(4-Chlorophenyl)-1 H-pyrrolo[2,3-c]pyridin-2-yl]-1-methyl-1,2-dihydropyridin-2-one |
| 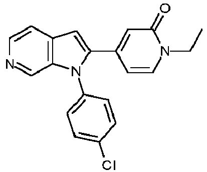 | 4-[1-(4-Chlorophenyl)-1 H-pyrrolo[2,3-c]pyridin-2-yl]-1-ethyl-1,2-dihydropyridin-2-one |
| 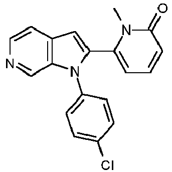 | 6-[1-(4-Chlorophenyl)-1 H-pyrrolo[2,3-c]pyridin-2-yl]-1-methyl-1,2-dihydropyridin-2-one |
| 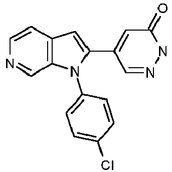 | 5-[1-(4-Chlorophenyl)-1 H-pyrrolo[2,3-c]pyridin-2-yl]-2,3-dihydropyridazin-3-one |
| 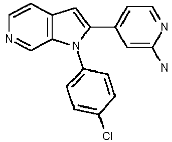 | 4-[1-(4-Chlorophenyl)-1 H-pyrrolo[2,3-c]pyridin-2-yl]pyridin-2-amine |
| 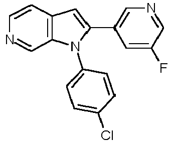 | 3-[1-(4-Chlorophenyl)-1 H-pyrrolo[2,3-c]pyridin-2-yl]-5-fluoropyridine |
| 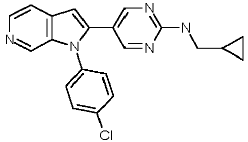 | 5-[1-(4-Chlorophenyl)-1 H-pyrrolo[2,3-c]pyridin-2-yl]-*N-*(cyclopropylmethyl)pyrimidin-2-amine |
| 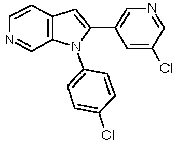 | 3-Chloro-5-[1-(4-chlorophenyl)-1 H-pyrrolo[2,3-c]pyridin-2-yl]pyridine |
| 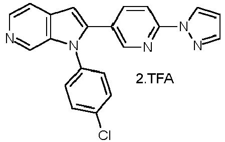 | 5-[1-(4-Chlorophenyl)-1 H-pyrrolo[2,3-c]pyridin-2-yl]-2-(1 H-pyrazol-1-yl)pyridine; bis(trifluoroacetic acid) |
| 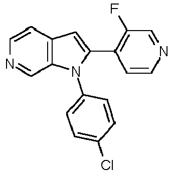 | 4-[1-(4-Chlorophenyl)-1 H-pyrrolo[2,3-c]pyridin-2-yl]-3-fluoropyridine |
| 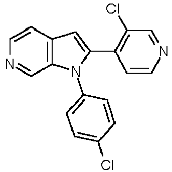 | 3-Chloro-4-[1-(4-chlorophenyl)-1 H-pyrrolo[2,3-c]pyridin-2-yl]pyridine |
| 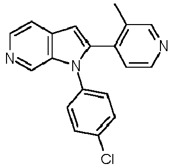 | 4-[1-(4-Chlorophenyl)-1 H-pyrrolo[2,3-c]pyridin-2-yl]-3-methyl pyridine |

### 1-Cyclopropyl-4-{1-phenyl-1H-pyrrolo[2,3-c]pyridin-2-yl}-1,2-dihydropyridin-2-one

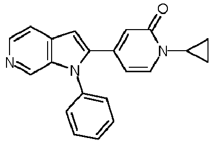

the inhibitors of SSAO activity specifically disclosed as Examples in WO2014/140591, namely:

### 4-[2-(6-Aminopyridin-3-yl)-1-(4-chlorophenyl)-1H-pyrrolo[2,3-c]pyridin-3-yl]piperidine-1-carboxamide

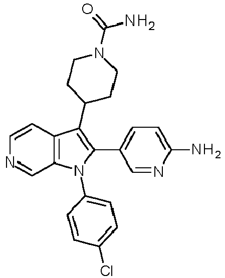

| Structure | Name |
|---|---|
| 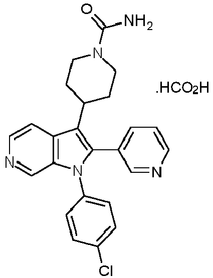 | 4-[1-(4-Chlorophenyl)-2-(pyridin-3-yl)-1 H-pyrrolo[2,3-c]pyridin-3-yl]piperidine-1-carboxamide; formic acid |
| 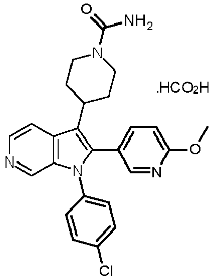 | 4-[1-(4-Chlorophenyl)-2-(6-methoxypyridin-3-yl)-1 H-pyrrolo[2,3-c]pyridin-3-yl]piperidine-1-carboxamide; formic acid |
| 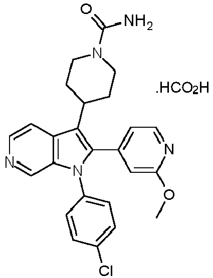 | 4-[1-(4-Chlorophenyl)-2-(2-methoxypyridin-4-yl)-1 H-pyrrolo[2,3-c]pyridin-3-yl]piperidine-1-carboxamide; formic acid |
| 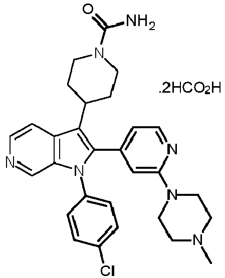 | 4-[1-(4-Chlorophenyl)-2-[2-(4-methylpiperazin-1-yl)pyridin-4-yl]-1 H-pyrrolo[2,3-c]pyridin-3-yl]piperidine-1-carboxamide; bis(formic acid) |
| 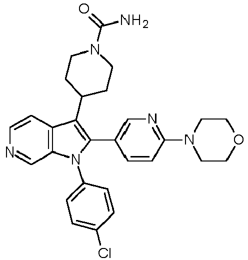 | 4-[1-(4-Chlorophenyl)-2-[6-(morpholin-4-yl)pyridin-3-yl]-1 H-pyrrolo[2,3-c]pyridin-3-yl]piperidine-1-carboxamide |
| 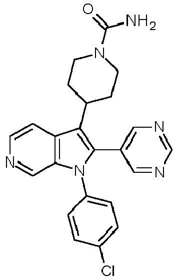 | 4-[1-(4-Chlorophenyl)-2-(pyrimidin-5-yl)-1 H-pyrrolo[2,3-*c*]pyridin-3-yl]piperidine-1-carboxamide |
| 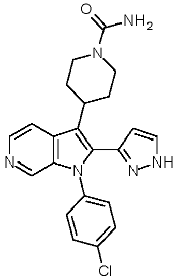 | 4-[1-(4-Chlorophenyl)-2-(1 H-pyrazol-3-yl)-1 H-pyrrolo[2,3-*c*]pyridin-3-yl]piperidine-1-carboxamide |
| 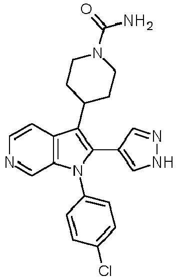 | 4-[1-(4-Chlorophenyl)-2-(1 H-pyrazol-4-yl)-1 H-pyrrolo[2,3-*c*]pyridin-3-yl]piperidine-1-carboxamide |
| 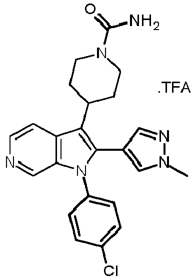 | 4-[1-(4-Chlorophenyl)-2-(1-methyl-1 H-pyrazol-4-yl)-1 H-pyrrolo[2,3-c]pyridin-3-yl]piperidine-1-carboxamide; trifluoroacetic acid |

### 4-[1-(4-Chlorophenyl)-2-(1-methyl-1H-pyrazol-5-yl)-1 H-pyrrolo[2,3-c]pyridin-3-yl]piperidine-1-carboxamide

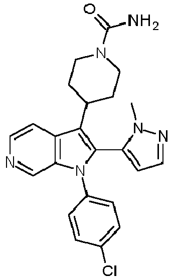

| Structure | Name |
|---|---|
| 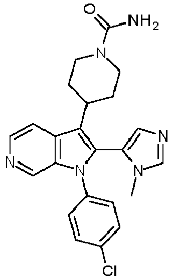 | 4-[1-(4-Chlorophenyl)-2-(1-methyl-1 H-imidazol-5-yl)-1 H-pyrrolo[2,3-c]pyridin-3-yl]piperidine-1-carboxamide |
| 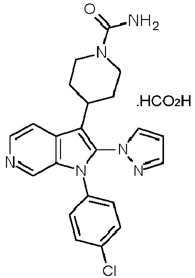 | 4-[1-(4-Chlorophenyl)-2-(1 H-pyrazol-1-yl)-1 H-pyrrolo[2,3-*c*]pyridin-3-yl]piperidine-1-carboxamide; formic acid |
| 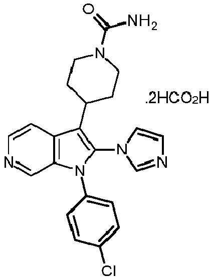 | 4-[1-(4-Chlorophenyl)-2-(1 H-imidazol-1-yl)-1 H-pyrrolo[2,3-*c*]pyridin-3-yl]piperidine-1-carboxamide; bis(formic acid) |
| 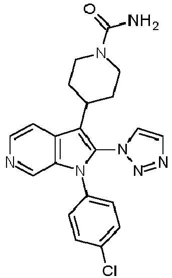 | 4-[1-(4-Chlorophenyl)-2-(1 H-1 ,2,3-triazol-1-yl)-1 H-pyrrolo[2,3-*c*]pyridin-3-yl]piperidine-1-carboxamide |
| 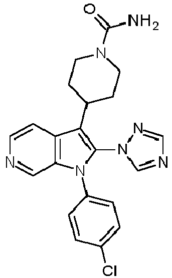 | 4-[1-(4-Chlorophenyl)-2-(1 H-1 ,2,4-triazol-1-yl)-1 H-pyrrolo[2,3-*c*]pyridin-3-yl]piperidine-1-carboxamide |

| | |
|---|---|
| 4-[1 -(4-Chlorophenyl)-3-(piperidin-4-yl)-1 H-pyrrolo[2,3-c]pyridin-2-yl]pyridine | |
| 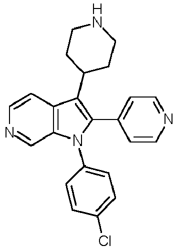 | 4-[1-(4-Chlorophenyl)-2-(2H-1,2,3,4-tetrazol-5-yl)-1H-pyrrolo[2,3-c]pyridin-3-yl]piperidine; bis(trifluoroacetic acid) |
| 5-[1-(4-Chlorophenyl)-3-(piperidin-4-yl)-1 H-pyrrolo[2,3-c]pyridin-2-yl]pyridin-2-amine | 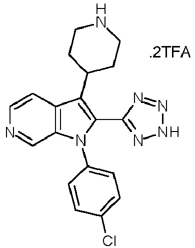 |
| 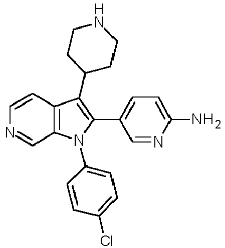 | |
| {[1 -(4-Chlorophenyl)-2-(pyridin-4-yl)-1 H-pyrrolo[2,3-c]pyridin-3-yl]methyl}dimethyl amine | |
| 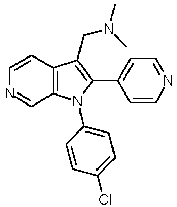 | |
| {[1-(4-Chlorophenyl)-2-(pyridin-3-yl)-1 H-pyrrolo[2,3-c]pyridin-3-yl]methyl}dimethyl amine | |
| 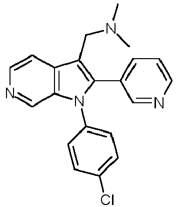 | |

Further specific Examples of VAP-1 inhibitor compounds suitable for use in the present invention are the Examples taught in co-pending application PCT/GB2015/052691, the content of which is hereby incorporated by reference in its entirety. Any pharmaceutically acceptable salt form of the Examples is suitable for use in the present invention. The Examples are:
5-[3-(4-Fluorophenyl)-3H-imidazo[4,5-c]pyridin-2-yl]-2,3-dihydro-1H-indol-2-one 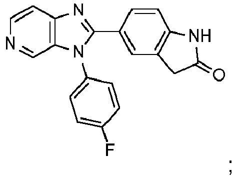
5-[3-(4-Fluorophenyl)-3H-imidazo[4,5-c]pyridin-2-yl]-1H-1,3-benzodiazole 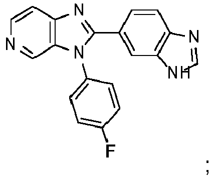
1-({5-[3-(4-Fluorophenyl)-3H-imidazo[4,5-c]pyridin-2-yl]pyrimidin-2-yl}amino)-2-methylpropan-2-ol 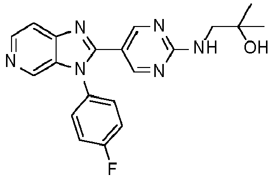
2-Methyl-1-({5-[3-(4-methylphenyl)-3H-imidazo[4,5-c]pyridin-2-yl]pyrimidin-2-yl}amino)propan-2-ol 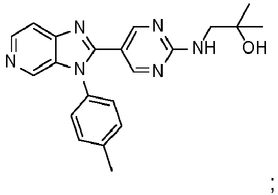
4-{4-[3-(4-Chlorophenyl)-3H-imidazo[4,5-c]pyridin-2-yl]piperidin-1-yl}pyridine; bis(formic acid) 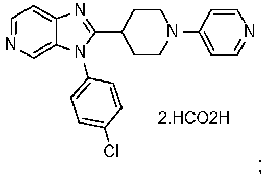
6-{4-[3-(4-Chlorophenyl)-3H-imidazo[4,5-c]pyridin-2-yl]piperidin-1-yl}-3,4-dihydropyrimidin-4-one 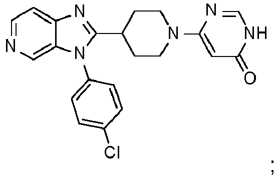
3-{[3-(4-Chlorophenyl)-3H-imidazo[4,5-c]pyridin-2-yl]methyl}pyridine; bis(formic acid) 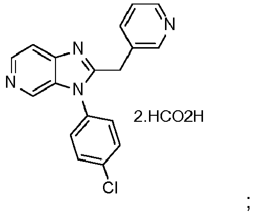
1-{3-[3-(4-chlorophenyl)-3H-imidazo[4,5-c]pyridin-2-yl]propyl}-1H-imidazole 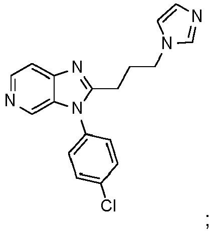
3-(4-Fluorophenyl)-N-(oxan-4-ylmethyl)-3H-imidazo[4,5-c]pyridine-2-carboxamide

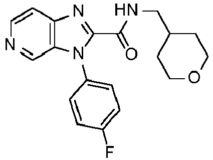

Further specific Examples of VAP-1 inhibitor compounds suitable for use in the present invention are the Examples taught in co-pending application PCT/GB2015/052690, the content of which is hereby incorporated by reference in its entirety. Any pharmaceutically acceptable salt form of the Examples is suitable for use in the present invention. The Examples are:
4-{5-[3-(5-Fluoropyridin-2-yl)-3H-imidazo[4,5-c]pyridin-2-yl]pyridin-2-yl}morpholine 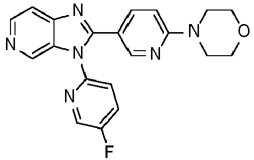
4-{5-[3-(2,4-Difluorophenyl)-3H-imidazo[4,5-c]pyridin-2-yl]-4-methylpyridin-2-yl}morpholine 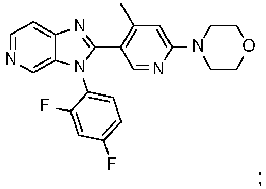
5-[3-(2,4-Difluorophenyl)-3H-imidazo[4,5-c]pyridin-2-yl]-N-(oxan-4-yl)pyrimidin-2-amine 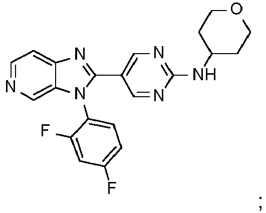
*N,N*-Diethyl-5-[3-(6-methylpyridin-3-yl)-3H-imidazo[4,5-c]pyridin-2-yl]pyrimidin-2-amine 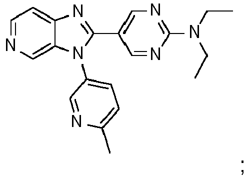
*N,N*-Diethyl-5-[3-(4-fluorophenyl)-3H-imidazo[4,5-c]pyridin-2-yl]pyrimidin-2-amine 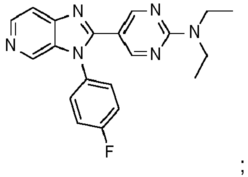
*N,N*-Diethyl-5-[3-(4-methylphenyl)-3H-imidazo[4,5-c]pyridin-2-yl]pyrimidin-2-amine 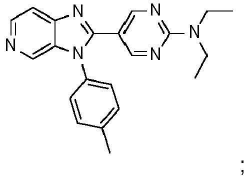
*N,N*-Diethyl-5-[3-(5-methylpyridin-2-yl)-3H-imidazo[4,5-c]pyridin-2-yl]pyrimidin-2-amine 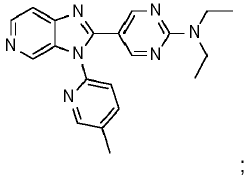
4-{5-[3-(2-Fluoro-4-methylphenyl)-3H-imidazo[4,5-c]pyridin-2-yl]-4-methylpyridin-2-yl}morpholine 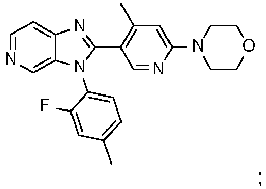
4-{5-[3-(4-Chlorophenyl)-3H-imidazo[4,5-c]pyridin-2-yl]-4-methylpyridin-2-yl}morpholine 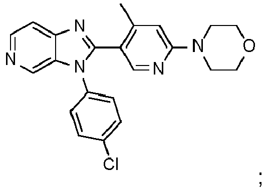
5-[3-(4-Methylphenyl)-3H-imidazo[4,5-c]pyridin-2-yl]-N-(oxan-4-yl)pyridin-2-amine 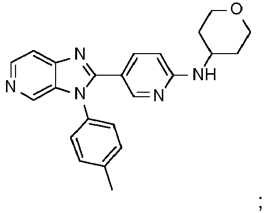
2-(4,4-Difluoropiperidin-1-yl)-5-[3-(4-methylphenyl)-3H-imidazo[4,5-c]pyridin-2-yl]pyridine 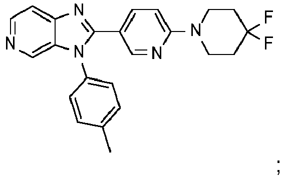
4-{5-[3-(5-Chloropyridin-2-yl)-3H-imidazo[4,5-c]pyridin-2-yl]-4-methylpyridin-2-yl}morpholine 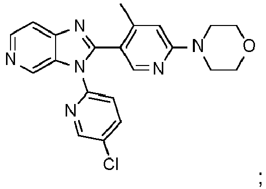
4-{4-Methyl-5-[3-(5-methylpyridin-2-yl)-3H-imidazo[4,5-c]pyridin-2-yl]pyridin-2-yl}morpholine 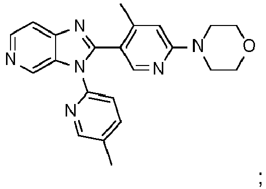
4-{5-[3-(5-Fluoropyridin-2-yl)-3H-imidazo[4,5-c]pyridin-2-yl]-4-methylpyridin-2-yl}morpholine; tris(trifluoroacetic acid) 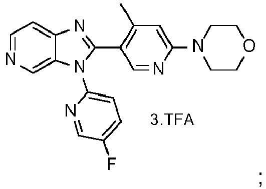
5-[3-(4-Fluorophenyl)-3H-imidazo[4,5-c]pyridin-2-yl]-N-(oxan-4-yl)pyridin-2-amine; 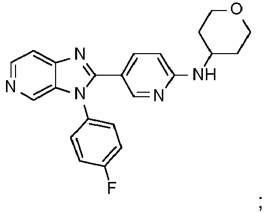
4-{5-[3-(4-Fluorophenyl)-3H-imidazo[4,5-c]pyridin-2-yl]pyridin-2-yl}thiomorpholine 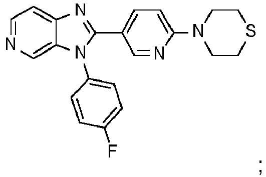
*N*-Cyclopropyl-5-[3-(4-methylphenyl)-3H-imidazo[4,5-c]pyridin-2-yl]pyridin-2-amine 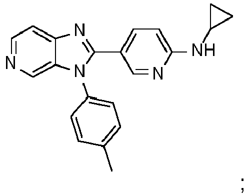
5-[3-(6-Methylpyridin-3-yl)-3H-imidazo[4,5-c]pyridin-2-yl]-2-(pyrrolidin-1-yl)pyridine 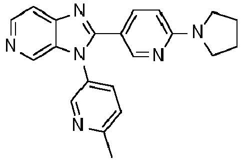
2-(4-Fluoropiperidin-1-yl)-5-[3-(6-methylpyridin-3-yl)-3H-imidazo[4,5-c]pyridin-2-yl]pyridine 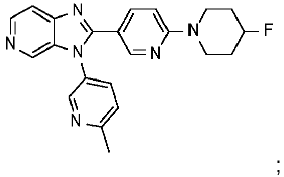
5-[3-(4-Fluorophenyl)-3H-imidazo[4,5-c]pyridin-2-yl]-*N*-[2-(morpholin-4-yl)ethyl]pyridin-2-amine 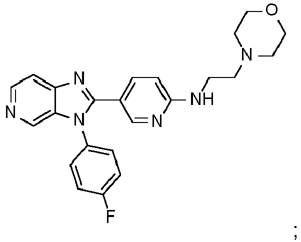
5-[3-(4-Methylphenyl)-3H-imidazo[4,5-c]pyridin-2-yl]-*N*-[2-(morpholin-4-yl)ethyl]pyridin-2-amine 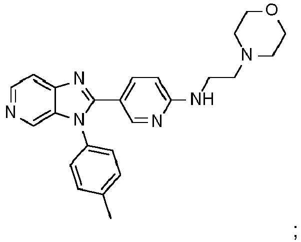
*N*-Cyclopropyl-5-[3-(4-fluorophenyl)-3H-imidazo[4,5-c]pyridin-2-yl]pyridin-2-amine 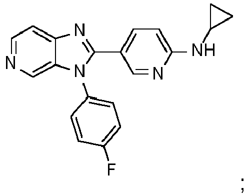
*N*-Cyclopropyl-5-[3-(6-methylpyridin-3-yl)-3H-imidazo[4,5-c]pyridin-2-yl]pyridin-2-amine 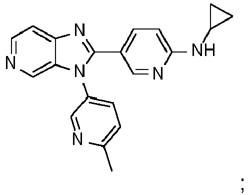
5-[3-(4-Methylphenyl)-3H-imidazo[4,5-c]pyridin-2-yl]-*N*-(propan-2-yl)pyridin-2-amine 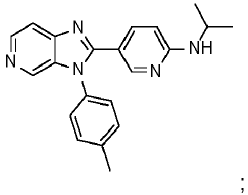
5-[3-(6-Methylpyridin-3-yl)-3H-imidazo[4,5-c]pyridin-2-yl]-2-(pyrrolidin-1-yl)pyrimidine 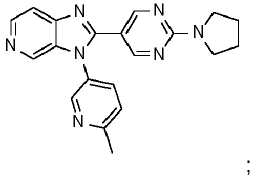
5-[3-(5-Methylpyridin-2-yl)-3H-imidazo[4,5-c]pyridin-2-yl]-2-(pyrrolidin-1-yl)pyrimidine 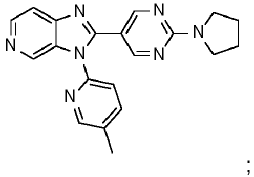
5-[3-(5-Fluoropyridin-2-yl)-3H-imidazo[4,5-c]pyridin-2-yl]-2-(pyrrolidin-1-yl)pyrimidine 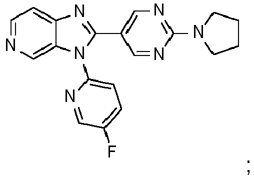
4-{4-[3-(6-Methylpyridin-3-yl)-3H-imidazo[4,5-c]pyridin-2-yl]phenyl}morpholine 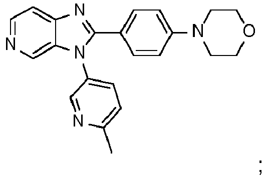
5-[3-(4-Methylphenyl)-3H-imidazo[4,5-c]pyridin-2-yl]-2-(pyrrolidin-1-yl)pyrimidine 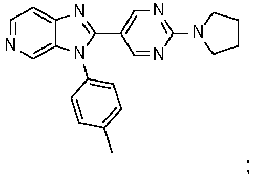
4-{4-[3-(5-Methylpyridin-2-yl)-3H-imidazo[4,5-c]pyridin-2-yl]phenyl}morpholine 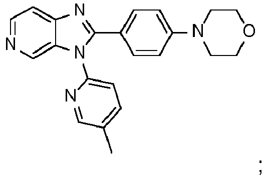
2-Methyl-5-{2-[4-(pyrrolidin-1-yl)phenyl]-3H-imidazo[4,5-c]pyridin-3-yl}pyridine 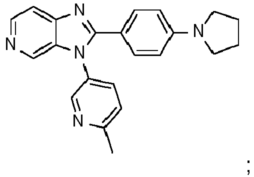
5-{2-[2-Fluoro-4-(pyrrolidin-1-yl)phenyl]-3H-imidazo[4,5-c]pyridin-3-yl}-2-methylpyridine 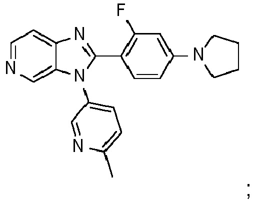
4-{3-Fluoro-4-[3-(6-methylpyridin-3-yl)-3H-imidazo[4,5-c]pyridin-2-yl]phenyl}morpholine 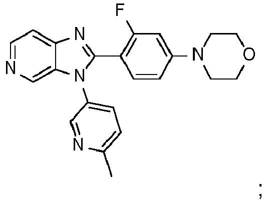
5-{2-[3-Fluoro-4-(pyrrolidin-1-yl)phenyl]-3H-imidazo[4,5-c]pyridin-3-yl}-2-methylpyridine 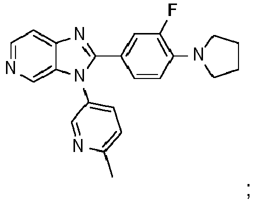
*N*-{4-[3-(6-Methylpyridin-3-yl)-3H-imidazo[4,5-c]pyridin-2-yl]phenyl)oxan-4-amine 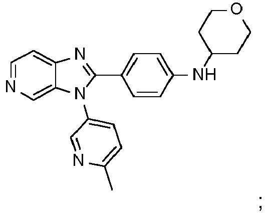
5-Methyl-2-{2-[4-(pyrrolidin-1-yl)phenyl]-3H-imidazo[4,5-c]pyridin-3-yl}pyridine 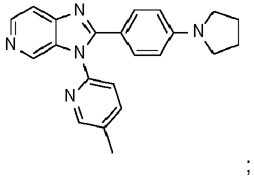
5-{2-[4-(4-Fluoropiperidin-1-yl)phenyl]-3H-imidazo[4,5-c]pyridin-3-yl}-2-methylpyridine 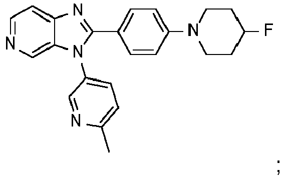
2-Chloro-5-[3-(4-chlorophenyl)-3H-imidazo[4,5-c]pyridin-2-yl]pyridine 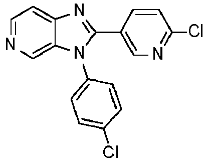
2-Chloro-5-[3-(4-fluorophenyl)-3H-imidazo[4,5-c]pyridin-2-yl]pyridine

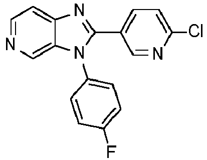

In an embodiment, the VAP-1 inhibitor suitable for use in the present invention is selected from the group consisiting of:
Procarbazine 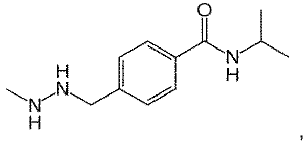
Isocarboxazid 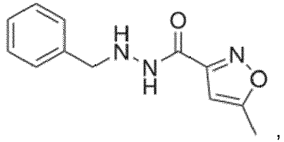 Guanabenz 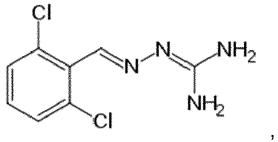
(S)-Carbidopa 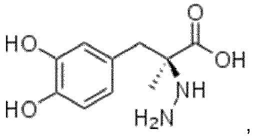 and
Benserazide 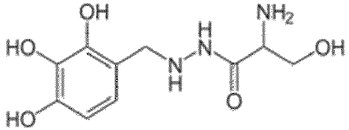
and pharmaceutically acceptable salts thereof.

The peripheral decarboxylase inhibitors benserazide and (S) carbidopa, often administered in combination with L-dopa in the treatment of Parkinson's disease, are also known to be very good inhibitors of VAP-1. Racemic Benserazide is preferred for use in the present invention. In an embodiment the Benserazide for use in the present invention is the (R) enantiomer or the (S) enantiomer.

Carbidopa exists as (R) and (S) enantiomers. Carbidopa is typically available as a mixture of the (R) and (S) enantiomers. Reference herein to "(S) carbidopa" includes any composition or mixture comprising (S) carbidopa, including for example substantially pure (S) carbidopa, or mixtures of (S) and (R) carbidopa, such as racemic mixtures. In an embodiment, the term "(S) carbidopa" as used herein means substantially pure (S) carbidopa.

### Steroids

The term "steroid" as used herein means any steroid suitable for use in the combined preparations according to the second and third aspects of the invention. The term "steroid" is also intended to encompass a combination of two or more steroids employed in the compositions and in the practice of the methodsof the present invention.

Suitable steroids include glucocorticoids. Examples of glucocorticoid steroids include prednisolone, prednisone, methyl prednisolone, triamcinolone, dexamethasone, hydrocortisone, deflazacourt, betamethasone and budenoside or pharmaceutically acceptable salts thereof. Particularly preferred steroids include prednisolone, or a pharmaceutically acceptable salt thereof; and prednisone, or a pharmaceutically acceptable salt thereof.

### VAP-1 Inhibitors for the Treatment of Pain

Pain is an unpleasant condition which may interfere with a person's quality of life. An unmet medical need exists for new or improved treatments for pain. Improved treatments may provide any or all of the following: superior pain reduction; faster pain relief; increased compliance; decreased likelihood of addiction; reduced treatment-related side effects; the ability to reduce exposure to other therapeutic agents that exhibit dose-dependent treatment-related side effects; or any other perceptible therapeutic benefit.

The applicants have discovered that compounds having VAP-1 inhibitory activity are surprisingly effective in the treatment of pain, including inflammatory and neuropathic pain. *In vivo* data in well-established models of pain is provided herein. This data demonstrates the efficacy of a broad range of VAP-1 inhibitors in the treatment of pain. Thus, the applicant demonstrates a credible link between the inhibition of VAP-1 activity and utility in the treatment pain. It is therefore expected that substantially all VAP-1 inhibitors will be effective in the treatment of pain. The following Examples of VAP-1 inhibitors having utility for the treatment of pain are non-limiting, and should be considered as merely illustrative of the broad scope of the invention. Furthermore, it has been surprisingly found that the effect of a VAP-1 inhibitor, such as (S)-carbidopa, on pain is independent of an effect (if any) on inflammation.

WO 2010/031789 (the content of which is herein incorporated by reference) discloses a promising class of SSAO inhibitor compounds, especially promising is Example 16, which is the free base of (3*S*)-Tetrahydrofuran-3-yl (4*S*)-4-isopropyl-1,4,6,7-tetrahydro-5H-imidazo[4,5-c]pyridine-5-carboxylate, and has the following structure:

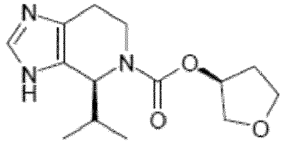

Following extensive investigations, it has been found that (3*S*)-Tetrahydrofuran-3-yl(4*S*)-4-isopropyl-1,4,6,7-tetrahydro-5H-imidazo[4,5-c]pyridine-5-carboxylate is surprisingly effective in the treatment of pain. This discovery is already the subject of co-pending UK patent application number application GB1507048.5, therefore the use of (3*S*)-Tetrahydrofuran-3-yl (4*S*)-4-isopropyl-1,4,6,7-tetrahydro-5H-imidazo[4,5-c]pyridine-5-carboxylate per se for the treatment of pain may be excluded from the scope of claims directed to the first aspect of the present invention. Nonetheless, the efficacy of (3*S*)-Tetrahydrofuran-3-yl(4*S*)-4-isopropyl-1,4,6,7-tetrahydro-5H-imidazo[4,5-c]pyridine-5-carboxylate in the treatment of pain (see Figure 1) supports the broadest sense of the first aspect of the invention, namely that VAP-1 inhibitors are useful for the treatment of pain, including inflammatory and neuropathic pain.

It has also been found that the VAP-1 inhibitor LJP1207 is surprisingly effective in the treatment of pain (see Figure 2).

It has also been found that the VAP-1 inhibitor (*S*)-carbidopa is surprisingly effective in the treatment of pain (see Figure 4).

It has also been found that the VAP-1 inhibitor 1-(4-{5-[3-(4-Fluorophenyl)-3H-imidazo[4,5-c]pyridin-2-yl]pyridin-2-yl}piperazin-1-yl)ethan-1-one (referred to as Compound 2) is surprisingly effective in the treatment of pain (see Figure 7).

It has also been found that the VAP-1 inhibitor 1-{5-[3-(4-Fluorophenyl)-3H-imidazo[4,5-c]pyridin-2-yl]pyridin-2-yl}-4-methanesulfonylpiperazine (referred to as Compound 3) is surprisingly effective in the treatment of pain (see Figure 8).

It has also been found that the VAP-1 inhibitor 4-{5-[3-(4-Fluorophenyl)-3H-imidazo[4,5-c]pyridin-2-yl]pyridin-2-yl}morpholine (referred to as Compound 4) is surprisingly effective in the treatment of pain (see Figure 9).

In an embodiment the present invention makes available a VAP-1 inhibitor for, or for use in the manufacture of a medicament for, the treatment of pain. In another embodiment, the present invention makes available a VAP-1 inhibitor for, or for use in the manufacture of a medicament for, the treatment of pain, provided that the VAP-1 inhibitor is other than (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof. In an embodiment the pain is inflammatory pain. In an embodiment, the pain is neuropathic pain.

In an embodiment the present invention makes available a method for the treatment of pain, which comprises administering to a subject suffering from pain an effective amount of a VAP-1 inhibitor. In another embodiment, the present invention makes available a method for the treatment of pain, which comprises administering to a subject suffering from pain an effective amount of a VAP-1 inhibitor, provided that the VAP-1 inhibitor is other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof. In an embodiment the pain is inflammatory pain. In an embodiment, the pain is neuropathic pain.

In an embodiment the present invention makes available a pharmaceutical composition for the treatment of pain, which comprises: a VAP-1 inhibitor and a pharmaceutically acceptable carrier, excipient, or diluent. In another embodiment the present invention makes available a pharmaceutical composition for the treatment of pain, which comprises: a VAP-1 inhibitor other than (3S)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof; and a pharmaceutically acceptable carrier, excipient, or diluent. In an embodiment the pain is inflammatory pain. In an embodiment, the pain is neuropathic pain.

In an embodiment, the VAP-1 inhibitor has the structure of any one of the specific Examples of VAP-1 inhibitor compounds, polypeptides or proteins disclosed herein. In a particular embodiment the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (S)-carbidopa, LJP1207, BTT1023, RTU-1096, PXS4728, ASP8232 and benserazide or a hydrate or pharmaceutically acceptable salt thereof. In a particular embodiment, the VAP-1 inhibitor is (S)-carbidopa, or a hydrate or a pharmaceutically acceptable salt thereof.

### VAP-1 Inhibitor and Steroid Combination for Treatment of Pain

In a second aspect of the invention, it has been found that a VAP-1 inhibitor in combination with a steroid is surprisingly effective in the treatment of pain. By surprisingly effective it is meant that the VAP-1 inhibitor and the steroid together provide a therapeutic effect which is greater than the therapeutic effect of the VAP-1 inhibitor and the steroid when dosed individually. In an embodiment, a VAP-1 inhibitor in combination with a steroid provides synergistic beneficial effects in the treatment of pain. In another embodiment, administration of a VAP-1 inhibitor in combination with a steroid allows the ability to reduce exposure to the steroid in order to reduce, minimise or eliminate dose-dependent treatment-related side effects that would otherwise be observed for monotherapy using steroid alone.

In an embodiment, the present invention makes available a combined preparation for, or for use in the manufacture of a medicament for, the treatment of pain, which comprises: a VAP-1 inhibitor and a steroid. In an embodiment the pain is inflammatory pain. In an embodiment, the pain is neuropathic pain.

In an embodiment, the present invention makes available a method for the treatment of pain, which comprises administering to a subject suffering from pain an effective amount of a VAP-1 inhibitor and a steroid. In an embodiment the pain is inflammatory pain. In an embodiment, the pain is neuropathic pain.

In an embodiment, the present invention makes available a pharmaceutical composition for the treatment of pain, which comprises: a VAP-1 inhibitor; a steroid; and a pharmaceutically acceptable carrier, excipient, or diluent. In an embodiment the pain is inflammatory pain. In an embodiment, the pain is neuropathic pain.

In an embodiment, the VAP-1 inhibitor has the structure of any one of the specific Examples of VAP-1 inhibitor compounds, polypeptides or proteins disclosed herein. In a particular embodiment the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (S)-carbidopa, LJP1207, BTT1023, RTU-1096, PXS4728, ASP8232 and benserazide or a hydrate or pharmaceutically acceptable salt thereof. In a particular embodiment, the VAP-1 inhibitor is (S)-carbidopa, or a hydrate or a pharmaceutically acceptable salt thereof.

In an embodiment the steroid is a glucocorticoid. In an embodiment the steroid is a glucocorticoid selected from prednisolone, prednisone, methyl prednisolone, triamcinolone, dexamethasone, hydrocortisone, deflazacourt, betamethasone and budenoside or pharmaceutically acceptable salts thereof. In another embodiment, the steroid is a combination of two or more of any of the aforementioned steroids or salts thereof. In particular embodiments, the steroid is prednisolone, or a pharmaceutically acceptable salt thereof. In particular embodiments, the steroid is prednisone, or a pharmaceutically acceptable salt thereof. Any combination of any VAP-1 inhibitor and any steroid is considered suitable for use in the claimed invention, and is therefore disclosed herein.

In an embodiment, the VAP-1 inhibitor compound is (S)-carbidopa, or a pharmaceutically acceptable salt thereof, and the steroid is prednisolone, or a pharmaceutically acceptable salt thereof.

In an embodiment, the VAP-1 inhibitor compound is (S)-carbidopa, or a pharmaceutically acceptable salt thereof, and the steroid is prednisone, or a pharmaceutically acceptable salt thereof.

In an embodiment, the VAP-1 inhibitor compound is (S)-carbidopa, or a pharmaceutically acceptable salt thereof, and the steroid is methyl prednisolone, or a pharmaceutically acceptable salt thereof.

In an embodiment, the VAP-1 inhibitor compound is (*S*)-carbidopa, or a pharmaceutically acceptable salt thereof, and the steroid is triamcinolone, or a pharmaceutically acceptable salt thereof.

In an embodiment, the VAP-1 inhibitor compound is (*S*)-carbidopa, or a pharmaceutically acceptable salt thereof, and the steroid is dexamethasone, or a pharmaceutically acceptable salt thereof.

In an embodiment, the VAP-1 inhibitor compound is (*S*)-carbidopa, or a pharmaceutically acceptable salt thereof, and the steroid is hydrocortisone, or a pharmaceutically acceptable salt thereof.

In an embodiment, the VAP-1 inhibitor compound is (*S*)-carbidopa, or a pharmaceutically acceptable salt thereof, and the steroid is deflazacourt, or a pharmaceutically acceptable salt thereof.

In an embodiment, the VAP-1 inhibitor compound is (*S*)-carbidopa, or a pharmaceutically acceptable salt thereof, and the steroid is betamethasone, or a pharmaceutically acceptable salt thereof.

In an embodiment, the VAP-1 inhibitor compound is (*S*)-carbidopa, or a pharmaceutically acceptable salt thereof, and the steroid is budenoside, or a pharmaceutically acceptable salt thereof.

In an embodiment, the VAP-1 inhibitor compound is LJP1207, or a pharmaceutically acceptable salt thereof, and the steroid is prednisolone or a pharmaceutically acceptable salt thereof.

In an embodiment, the VAP-1 inhibitor compound is LJP1207, or a pharmaceutically acceptable salt thereof, and the steroid is prednisone or a pharmaceutically acceptable salt thereof.

In an embodiment, the VAP-1 inhibitor compound is LJP1207, or a pharmaceutically acceptable salt thereof, and the steroid is methyl prednisolone or a pharmaceutically acceptable salt thereof.

In an embodiment, the VAP-1 inhibitor compound is LJP1207, or a pharmaceutically acceptable salt thereof, and the steroid is triamcinolone or a pharmaceutically acceptable salt thereof.

In an embodiment, the VAP-1 inhibitor compound is LJP1207, or a pharmaceutically acceptable salt thereof, and the steroid is dexamethasone or a pharmaceutically acceptable salt thereof.

In an embodiment, the VAP-1 inhibitor compound is LJP1207, or a pharmaceutically acceptable salt thereof, and the steroid is hydrocortisone or a pharmaceutically acceptable salt thereof.

In an embodiment, the VAP-1 inhibitor compound is LJP1207, or a pharmaceutically acceptable salt thereof, and the steroid is deflazacourt or a pharmaceutically acceptable salt thereof.

In an embodiment, the VAP-1 inhibitor compound is LJP1207, or a pharmaceutically acceptable salt thereof, and the steroid is betamethasone or a pharmaceutically acceptable salt thereof.

In an embodiment, the VAP-1 inhibitor compound is LJP1207, or a pharmaceutically acceptable salt thereof, and the steroid is budenoside or a pharmaceutically acceptable salt thereof.

In an embodiment, the VAP-1 inhibitor compound is BTT1023, or a pharmaceutically acceptable salt thereof, and the steroid is prednisolone or a pharmaceutically acceptable salt thereof.

In an embodiment, the VAP-1 inhibitor compound is BTT1023, or a pharmaceutically acceptable salt thereof, and the steroid is prednisone or a pharmaceutically acceptable salt thereof.

In an embodiment, the VAP-1 inhibitor compound is BTT1023, or a pharmaceutically acceptable salt thereof, and the steroid is methyl prednisolone or a pharmaceutically acceptable salt thereof.

In an embodiment, the VAP-1 inhibitor compound is BTT1023, or a pharmaceutically acceptable salt thereof, and the steroid is triamcinolone or a pharmaceutically acceptable salt thereof.

In an embodiment, the VAP-1 inhibitor compound is BTT1023, or a pharmaceutically acceptable salt thereof, and the steroid is dexamethasone or a pharmaceutically acceptable salt thereof.

In an embodiment, the VAP-1 inhibitor compound is BTT1023, or a pharmaceutically acceptable salt thereof, and the steroid is hydrocortisone or a pharmaceutically acceptable salt thereof.

In an embodiment, the VAP-1 inhibitor compound is BTT1023, or a pharmaceutically acceptable salt thereof, and the steroid is deflazacourt or a pharmaceutically acceptable salt thereof.

In an embodiment, the VAP-1 inhibitor compound is BTT1023, or a pharmaceutically acceptable salt thereof, and the steroid is betamethasone or a pharmaceutically acceptable salt thereof.

In an embodiment, the VAP-1 inhibitor compound is BTT1023, or a pharmaceutically acceptable salt thereof, and the steroid is budenoside or a pharmaceutically acceptable salt thereof.

In an embodiment, the VAP-1 inhibitor compound is RTU-1096, or a pharmaceutically acceptable salt thereof, and the steroid is prednisolone or a pharmaceutically acceptable salt thereof.

In an embodiment, the VAP-1 inhibitor compound is RTU-1096, or a pharmaceutically acceptable salt thereof, and the steroid is prednisone or a pharmaceutically acceptable salt thereof.

In an embodiment, the VAP-1 inhibitor compound is RTU-1096, or a pharmaceutically acceptable salt thereof, and the steroid is methyl prednisolone or a pharmaceutically acceptable salt thereof.

In an embodiment, the VAP-1 inhibitor compound is RTU-1096, or a pharmaceutically acceptable salt thereof, and the steroid is triamcinolone or a pharmaceutically acceptable salt thereof.

In an embodiment, the VAP-1 inhibitor compound is RTU-1096, or a pharmaceutically acceptable salt thereof, and the steroid is dexamethasone or a pharmaceutically acceptable salt thereof.

In an embodiment, the VAP-1 inhibitor compound is RTU-1096, or a pharmaceutically acceptable salt thereof, and the steroid is hydrocortisone or a pharmaceutically acceptable salt thereof.

In an embodiment, the VAP-1 inhibitor compound is RTU-1096, or a pharmaceutically acceptable salt thereof, and the steroid is deflazacourt or a pharmaceutically acceptable salt thereof.

In an embodiment, the VAP-1 inhibitor compound is RTU-1096, or a pharmaceutically acceptable salt thereof, and the steroid is betamethasone or a pharmaceutically acceptable salt thereof.

In an embodiment, the VAP-1 inhibitor compound is RTU-1096, or a pharmaceutically acceptable salt thereof, and the steroid is budenoside or a pharmaceutically acceptable salt thereof.

In an embodiment, the VAP-1 inhibitor compound is PXS4728, or a pharmaceutically acceptable salt thereof, and the steroid is prednisolone or a pharmaceutically acceptable salt thereof.

In an embodiment, the VAP-1 inhibitor compound is PXS4728, or a pharmaceutically acceptable salt thereof, and the steroid is prednisone or a pharmaceutically acceptable salt thereof.

In an embodiment, the VAP-1 inhibitor compound is PXS4728, or a pharmaceutically acceptable salt thereof, and the steroid is methyl prednisolone or a pharmaceutically acceptable salt thereof.

In an embodiment, the VAP-1 inhibitor compound is PXS4728, or a pharmaceutically acceptable salt thereof, and the steroid is triamcinolone or a pharmaceutically acceptable salt thereof.

In an embodiment, the VAP-1 inhibitor compound is PXS4728, or a pharmaceutically acceptable salt thereof, and the steroid is dexamethasone or a pharmaceutically acceptable salt thereof.

In an embodiment, the VAP-1 inhibitor compound is PXS4728, or a pharmaceutically acceptable salt thereof, and the steroid is hydrocortisone or a pharmaceutically acceptable salt thereof.

In an embodiment, the VAP-1 inhibitor compound is PXS4728, or a pharmaceutically acceptable salt thereof, and the steroid is deflazacourt or a pharmaceutically acceptable salt thereof.

In an embodiment, the VAP-1 inhibitor compound is PXS4728, or a pharmaceutically acceptable salt thereof, and the steroid is betamethasone or a pharmaceutically acceptable salt thereof.

In an embodiment, the VAP-1 inhibitor compound is PXS4728, or a pharmaceutically acceptable salt thereof, and the steroid is budenoside or a pharmaceutically acceptable salt thereof.

In an embodiment, the VAP-1 inhibitor compound is ASP8232, or a pharmaceutically acceptable salt thereof, and the steroid is prednisolone or a pharmaceutically acceptable salt thereof.

In an embodiment, the VAP-1 inhibitor compound is ASP8232, or a pharmaceutically acceptable salt thereof, and the steroid is prednisone or a pharmaceutically acceptable salt thereof.

In an embodiment, the VAP-1 inhibitor compound is ASP8232, or a pharmaceutically acceptable salt thereof, and the steroid is methyl prednisolone or a pharmaceutically acceptable salt thereof.

In an embodiment, the VAP-1 inhibitor compound is ASP8232, or a pharmaceutically acceptable salt thereof, and the steroid is triamcinolone or a pharmaceutically acceptable salt thereof.

In an embodiment, the VAP-1 inhibitor compound is ASP8232, or a pharmaceutically acceptable salt thereof, and the steroid is dexamethasone or a pharmaceutically acceptable salt thereof.

In an embodiment, the VAP-1 inhibitor compound is ASP8232, or a pharmaceutically acceptable salt thereof, and the steroid is hydrocortisone or a pharmaceutically acceptable salt thereof.

In an embodiment, the VAP-1 inhibitor compound is ASP8232, or a pharmaceutically acceptable salt thereof, and the steroid is deflazacourt or a pharmaceutically acceptable salt thereof.

In an embodiment, the VAP-1 inhibitor compound is ASP8232, or a pharmaceutically acceptable salt thereof, and the steroid is betamethasone or a pharmaceutically acceptable salt thereof.

In an embodiment, the VAP-1 inhibitor compound is ASP8232, or a pharmaceutically acceptable salt thereof, and the steroid is budenoside or a pharmaceutically acceptable salt thereof.

In an embodiment, the VAP-1 inhibitor compound is benserazide, or a pharmaceutically acceptable salt thereof, and the steroid is prednisolone or a pharmaceutically acceptable salt thereof.

In an embodiment, the VAP-1 inhibitor compound is benserazide, or a pharmaceutically acceptable salt thereof, and the steroid is prednisone or a pharmaceutically acceptable salt thereof.

In an embodiment, the VAP-1 inhibitor compound is benserazide, or a pharmaceutically acceptable salt thereof, and the steroid is methyl prednisolone or a pharmaceutically acceptable salt thereof.

In an embodiment, the VAP-1 inhibitor compound is benserazide, or a pharmaceutically acceptable salt thereof, and the steroid is triamcinolone or a pharmaceutically acceptable salt thereof.

In an embodiment, the VAP-1 inhibitor compound is benserazide, or a pharmaceutically acceptable salt thereof, and the steroid is dexamethasone or a pharmaceutically acceptable salt thereof.

In an embodiment, the VAP-1 inhibitor compound is benserazide, or a pharmaceutically acceptable salt thereof, and the steroid is hydrocortisone or a pharmaceutically acceptable salt thereof.

In an embodiment, the VAP-1 inhibitor compound is benserazide, or a pharmaceutically acceptable salt thereof, and the steroid is deflazacourt or a pharmaceutically acceptable salt thereof.

In an embodiment, the VAP-1 inhibitor compound is benserazide, or a pharmaceutically acceptable salt thereof, and the steroid is betamethasone or a pharmaceutically acceptable salt thereof.

In an embodiment, the VAP-1 inhibitor compound is benserazide, or a pharmaceutically acceptable salt thereof, and the steroid is budenoside or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is prednisolone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is prednisone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is methyl prednisolone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is triamcinolone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is dexamethasone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is hydrocortisone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is deflazacourt or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is betamethasone or a pharmaceutically acceptable salt thereof.

In an embodiment, the VAP-1 inhibitor compound is 1-(4-{5-[3-(4-Fluorophenyl)-3H-imidazo[4,5-c]pyridin-2-yl]pyridin-2-yl}piperazin-1-yl)ethan-1-one, or a pharmaceutically acceptable salt thereof, and the steroid is budenoside or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is prednisolone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is prednisone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is methyl prednisolone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is triamcinolone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is dexamethasone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is hydrocortisone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is deflazacourt or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is betamethasone or a pharmaceutically acceptable salt thereof.

In an embodiment, the VAP-1 inhibitor compound is 1-{5-[3-(4-Fluorophenyl)-3H-imidazo[4,5-c]pyridin-2-yl]pyridin-2-yl}-4-methanesulfonylpiperazine, or a pharmaceutically acceptable salt thereof, and the steroid is budenoside or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is prednisolone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is prednisone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is methyl prednisolone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is triamcinolone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is dexamethasone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is hydrocortisone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is deflazacourt or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is betamethasone or a pharmaceutically acceptable salt thereof.

In an embodiment, the VAP-1 inhibitor compound is 4-{5-[3-(4-Fluorophenyl)-3H-imidazo[4,5-c]pyridin-2-yl]pyridin-2-yl}morpholine, or a pharmaceutically acceptable salt thereof, and the steroid is budenoside or a pharmaceutically acceptable salt thereof.

### (3S)-Tetrahydrofuran-3-yl(4S)-4-isopropyl-1,4,6,7-tetrahydro-5H-imidazo[4,5-c]pyridine-5-carboxylate and Steroid Combination.

As set out above, the applicant has found that a VAP-1 inhibitor in combination with a steroid is surprisingly effective in the treatment of pain.

It is therefore expected that the VAP-1 inhibitor (3*S*)-Tetrahydrofuran-3-yl(4*S*)-4-isopropyl-1,4,6,7-tetrahydro-5H-imidazo[4,5-c]pyridine-5-carboxylate in combination with a steroid will be surprisingly effective as a medicament, particularly in the treatment of pain. Therefore, in an embodiment, the applicant makes available a combined preparation, which comprises: (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof; and a steroid. In an embodiment, the combined preparation is useful for the treatment of pain. In an embodiment the pain is inflammatory pain. In an embodiment, the pain is neuropathic pain. In an embodiment the (3*S*)-Tetrahydrofuran-3-yl(4*S*)-4-isopropyl-1,4,6,7-tetrahydro-5H-imidazo[4,5-c]pyridine-5-carboxylate in combination with a steroid are synergistic when used for the treatment of pain, including inflammatory and neuropathic pain.
In an embodiment, the applicant makes available a method of treating pain comprising administering to a subject suffering from pain an effective amount of (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof and a steroid. In an embodiment the pain is inflammatory pain. In an embodiment, the pain is neuropathic pain.

In an embodiment, the applicant makes available a pharmaceutical composition, which comprises: (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof; a steroid; and a pharmaceutically acceptable carrier, excipient, or diluent. In an embodiment, the pharmaceutical composition is useful for the treatment of pain. In an embodiment the pain is inflammatory pain. In an embodiment, the pain is neuropathic pain.

In an embodiment, the pharmaceutically acceptable salt of (3*S*)-Tetrahydrofuran-3-yl(4*S*)-4-isopropyl-1,4,6,7-tetrahydro-5H-imidazo[4,5-c]pyridine-5-carboxylate may be a mesylate or a sulfate, or a hydrate thereof. A particular salt is the mesylate salt. An alternative salt is the sulphate salt, which typically exists as a hydrate; in an embodiment the hydrate is (3*S*)-Tetrahydrofuran-3-yl (4*S*)-4-isopropyl-1,4,6,7-tetrahydro-5H-imidazo[4,5-c]pyridine-5-carboxylate sulphate 1.5 H₂O.

In an embodiment the steroid is a glucocorticoid. In an embodiment the steroid is a glucocorticoid selected from prednisolone, prednisone, methyl prednisolone, triamcinolone, dexamethasone, hydrocortisone, deflazacourt, betamethasone and budenoside or pharmaceutically acceptable salts thereof. In particular embodiments, the steroid is prednisolone, or a pharmaceutically acceptable salt thereof. In particular embodiments, the steroid is prednisone, or a pharmaceutically acceptable salt thereof.

In an embodiment, (3*S*)-Tetrahydrofuran-3-yl (4*S*)-4-isopropyl-1,4,6,7-tetrahydro-5H-imidazo[4,5-c]-pyridine-5-carboxylate or a hydrate or a pharmaceutically acceptable salt thereof is combined with a glucocorticoid steroid selected from any one of prednisolone, prednisone, methyl prednisolone, triamcinolone, dexamethasone, hydrocortisone, deflazacourt, betamethasone and budenoside or pharmaceutically acceptable salts thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with prednisolone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with prednisone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with methyl prednisolone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with triamcinolone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with dexamethasone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with hydrocortisone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with deflazacourt, or a pharmaceutically acceptable salt thereof.In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with betamethasone, or a pharmaceutically acceptable salt thereof.

In a particular embodiment, (3*S*)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof is combined with budenoside, or a pharmaceutically acceptable salt thereof.

Unless stated to the contrary, the term "(3*S*)-Tetrahydrofuran-3-yl (4*S*)-4-isopropyl-1,4,6,7 tetrahydro-5H-imidazo[4,5-c]pyridine-5-carboxylate" as used herein includes a mixture of the (3S,4S) and (3R,4R) enantiomers. In an embodiment (3*S*)-Tetrahydrofuran-3-yl (4*S*)-4-isopropyl-1,4,6,7-tetrahydro-5H-imidazo[4,5-c]pyridine-5-carboxylate, and salts thereof, has an absolute purity of >95%, preferably >99%, more preferably >99.5%. In an embodiment (3*S*)- Tetrahydrofuran-3-yl (4*S*)-4-isopropyl-1,4,6,7-tetrahydro-5H-imidazo[4,5-c]pyridine-5- carboxylate means the (3*S*,4*S*) enantiomer having an enantiomeric purity of >95%, preferably >99%, more preferably >99.5%. In an embodiment (3*S*)-Tetrahydrofuran-3-yl (4*S*)-4-isopropyl-1,4,6,7-tetrahydro-5H-imidazo[4,5-c]pyridine-5-carboxylate has a diastereoisomeric purity of >95%, preferably >99%, more preferably >99.5%.

A typical dosage of (3*S*)-Tetrahydrofuran-3-yl (4*S*)-4-isopropyl-1,4,6,7-tetrahydro-5H-imidazo[4,5-c]pyridine-5-carboxylate is 2 to 20 mg/kg, administered one or more times per day or by continuous infusion. A typical total daily dosage for a human is 1 to 2000mg/day, preferably from 200 to 2000mg/day, more preferably from 500 to 2000mg/day. In an embodiment, (3*S*)-Tetrahydrofuran-3-yl (4*S*)-4-isopropyl-1,4,6,7-tetrahydro-5H-imidazo[4,5-c]pyridine-5-carboxylate is dosed three times per day. In an embodiment, (3*S*)-Tetrahydrofuran-3-yl (4*S*)-4-isopropyl-1,4,6,7-tetrahydro-5H-imidazo[4,5-c]pyridine-5-carboxylate is dosed three times per day in doses of from 200 to 600mg. In an embodiment, (3*S*)-Tetrahydrofuran-3-yl (4*S*)-4-isopropyl-1,4,6,7-tetrahydro-5H-imidazo[4,5-c]pyridine-5-carboxylate is dosed three times per day in doses of 400mg.

(3S)-Tetrahydrofuran-3-yl (4*S*)-4-isopropyl-1,4,6,7-tetrahydro-5H-imidazo[4,5-c]- pyridine-5-carboxylate may be administered in a variety of dosage forms. Thus, it can be administered orally, for example as a tablet, a capsule, a troche, a lozenge, an aqueous or oily suspension, a dispersible powder or granule. The drug is preferably administered via the oral route. It will be understood, however, that the specific dose level for any particular patient will depend upon a variety of factors including the age, body weight, general health, sex, diet, time of administration, drug combination and the severity of the particular condition undergoing therapy.

### COMPOSITIONS

A pharmaceutical composition containing the active ingredient, or active ingredients in the case of a combined preparation, may be in any suitable form, for example aqueous or non-aqueous solutions or suspensions, dispersible powders or granules, transdermal or transmucosal patches, creams, ointments or emulsions.

The pharmaceutical composition may be in the form of a sterile injectable aqueous or non-aqueous (e.g. oleaginous) solution or suspension. The sterile injectable preparation may also be in a sterile injectable solution or suspension in a non-toxic parenterally-acceptable diluent or solvent, for example as a solution in 1,3-butanediol. Among the acceptable vehicles and solvents that may be employed are water, phosphate buffer solution, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables. Suspensions may be formulated according to the known art using those suitable dispersing or wetting agents and suspending agents.

Aqueous suspensions contain the active ingredient, or active ingredients in the case of a combined preparation, in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents such as a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Non-aqueous (i.e. oily) suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are known.

The active agent may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical delivery, transdermal and transmucosal patches, creams, ointments, jellies, solutions or suspensions may be employed. For sub-lingual delivery, fast dissolving tablet formulations may be used, as well as a number of the presentations described above. For oral administration, the drug may be administered as tablets, capsules or liquids.

Formulations may conveniently be presented in unit dosage form, e.g., tablets and sustained release capsules, and in liposomes, and may be prepared by any method known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds of the invention may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Such a dosage may be given orally or parenterally. Multiple doses may be administered over a period of time, such as at least a week, a month, several months, a year, or several years, or throughout the course of the condition. The frequency of dosage may be at least once per month, once per week, or once per day.

### COMBINED PREPARATIONS

The components of a combined preparation according to the second and third aspects of the invention may be for simultaneous, separate, or sequential use.

The term "combined preparation" as used herein refers to a "kit of parts" in the sense that the combination components of (a) a VAP-1 inhibitor and (b) a steroid can be dosed independently or by use of different fixed combinations with distinguished amounts of the combination components (a) and (b). The components can be administered simultaneously or one after the other. If the components are administered one after the other, preferably the time interval between administrations is chosen such that the effect on the treated disorder or disease in the combined use of the components is greater than the effect which would be obtained by use of only any one of the combination components (a) and (b).

The components of the combined preparation may be present in one combined unit dosage form, or as a first unit dosage form of component (a) and a separate, second unit dosage form of component (b). The ratio of the total amounts of the combination component (a) to the combination component (b) to be administered in the combined preparation can be varied, for example in order to cope with the needs of a patient sub-population to be treated, or the needs of the single patient, which can be due, for example, to the particular disease, age, sex, or body weight of the patients.

Preferably, there is at least one beneficial effect, for example an enhancing of the effect of the VAP-1 inhibitor, or a mutual enhancing of the effect of the combination components (a) and (b), for example a more than additive effect, additional advantageous effects, fewer side effects, less toxicity, or a combined therapeutic effect compared with a non-effective dosage of one or both of the combination components (a) and (b), and very preferably a synergism of the combination components (a) and (b).

The VAP-1 inhibitor and the steroid may be administered sequentially to the subject, i.e. the VAP-1 inhibitor may be administered before, with, or after the steroid.

The VAP-1 inhibitor and the steroid may be administered to the subject within 96 hours, 72 hours, 48 hours, 24 hours, or 12 hours, of each other.

Alternatively, the VAP-1 inhibitor and the steroid may be co-administered to the subject, for example as a composition comprising the VAP-1 inhibitor and the steroid, or by simultaneous administration of separate doses of the VAP-1 inhibitor and the steroid.

According to some embodiments, a plurality of doses of the VAP-1 inhibitor, and/or a plurality of doses of the steroid, is administered to the subject.

According to some embodiments, a dose of the VAP-1 inhibitor is administered before, with, or after each administration of two or more doses of the steroid.

For example, a dose of VAP-1 inhibitor may be administered within 96 hours, 72 hours, 48 hours, 24 hours, or 12 hours, of each administration of two or more doses of the steroid.

The choice of appropriate dosages of the components used in combination therapy according to the present invention can be determined and optimized by the skilled person, for example, by observation of the patient, including the patient's overall health, and the response to the combination therapy. Optimization, for example, may be necessary if it is determined that a patient is not exhibiting the desired therapeutic effect or conversely, if the patient is experiencing undesirable or adverse side effects that are too many in number or are of a troublesome severity.

The doses of the components used in combination therapy according to the invention should be chosen to provide a therapeutically effective amount of the components in combination. An "effective amount" of the combination therapy is an amount that results in a reduction of at least one pathological parameter associated with pain. For example, in some embodiments, an effective amount of the combination therapy is an amount that is effective to achieve a reduction of at least about 10%, 20%, 30%, 40%, 50%, 60%, 70%, 80%, or 90%, in the parameter, compared to the expected reduction in the parameter associated with the pain without the combination therapy.
For example, the parameter may be a score resulting from an assessment under the Western Ontario and McMaster Universities Arthritis Index (WOMAC), such as the WOMAC® 3.1 Index, for example for pain during walking, using stairs, in bed, sitting or lying, and standing, or daily activity, physical function or stiffness scores. Alternatively the parameter may be a score from an assessment on the Visual Analogue Scale (VAS), Pain Intensity (PI) Scale, Wong-Baker FACES Pain Rating Scale, 0-10 Numeric Pain Rating Scale, Verbal Pain Intensity Scale or Descriptor Differential Scale.

According to the invention, combination treatment may be employed to increase the therapeutic effect of the VAP-1 inhibitor or steroid, compared with the effect of the VAP-1 inhibitor or steroid as a monotherapy, or to decrease the doses of the individual components in the resulting combinations while preventing or further reducing the risk of unwanted or harmful side effects of the individual components.

A typically prescribed dose range for a steroid as a monotherapy, in particular a glucocorticoid such as prednisone or prednisolone, is 0.3-1 mg/kg/day (suitably 0.7 or 0.75mg/kg/day), or 0.3mg/kg/day to 10mg/kg/week, in humans.

A typically prescribed dose range for a VAP-1 inhibitor as a monotherapy in humans is 20-200mg/day for (S) carbidopa (suitably 30mg/day or 75 mg/day), and 25-300mg/day (suitably 25mg/day or 50mg/day) for benserazide.

In one embodiment, the VAP-1 inhibitor and the steroid are each prescribed at a dose that is within a typically prescribed dose range for each compound as a monotherapy. The compounds may be prescribed as separate dosages or as a combination dosage. Such combinations provide increased efficacy compared with the effect of either compound as a monotherapy.

In another embodiment, the VAP-1 inhibitor and the steroid are each prescribed at a dose that is below a typically prescribed dose for each component as a monotherapy, but at doses that have therapeutic efficacy in combination. The components may be prescribed as separate dosages or as a combination dosage. The dosages of the components in combination may be selected to provide a similar level of therapeutic efficacy as the VAP-1 inhibitor or the steroid as a monotherapy, but with the advantage that the lower doses of the VAP-1 inhibitor and/or the steroid reduce the risk of adverse side effects compared to the prescribed dosages of each compound as a monotherapy.

In another embodiment, the prescribed dosage of the VAP-1 inhibitor is within a typically prescribed dose range for monotherapy, and the steroid is prescribed at a dosage that is below a typically prescribed dose for monotherapy.

In a further embodiment, the prescribed dosage of the VAP-1 inhibitor is below a typically prescribed dose for monotherapy, and the steroid is prescribed at a dosage that is within a typically prescribed dose range for monotherapy.

Preferred dosages below the typically prescribed dose for monotherapy are doses that are up to 50%, or up to 25%, of the typically prescribed dose.

When administered in separate dosages, the VAP-1 inhibitor and the steroid may be administered substantially simultaneously (for example, within about 60 minutes, about 50 minutes, about 40 minutes, about 30 minutes, about 20 minutes, about 10 minutes, about 5 minutes, or about 1 minute of each other) or separated in time by about 1 hour, about 2 hours, about 4 hours, about 6 hours, about 10 hours, about 12 hours, about 24 hours, about 36 hours, about 72 hours, or about 96 hours, or more.

The skilled person will be able to determine, and optimise, a suitable time course for sequential administration, depending on the particular combination of the VAP-1 inhibitor and the steroid. The time course is preferably selected such that there is at least one beneficial effect, for example an enhancing of the effect of the VAP-1 inhibitor or the steroid, or a mutual enhancing of the effect of the combination components, for example a more than additive effect, additional advantageous effects, fewer side effects, less toxicity, or a combined therapeutic effect compared with a non-effective dosage of one or both of the combination components, and very preferably a synergism of the combination components.

It will be appreciated that the optimum time course will depend on factors such as the time taken for the peak plasma concentration of the compound to be reached after administration, and the elimination half-life of each compound. Preferably the time difference is less than the half-life of the first component to be administered.

The skilled person will also be able to determine appropriate timing for administration. In certain embodiments, the VAP-1 inhibitor may be administered in the morning, and the steroid administered at least once later in the day. In other embodiments, the VAP-1 inhibitor and the steroid may be administered at substantially the same time.

The subject may receive doses of the VAP-1 inhibitor and the steroid over a period of weeks, months, or years. For example, 1 week, 2 weeks, 3 weeks, 1 month, 2 months, 3 months, 4 months, 5 months, 6 months, 7 months, 8 months, 9 months, 10 months, 11 months, 1 year, 2 years, 3 years, 4 years, 5 years, or more.

In general, the components of a combination of the invention may be administered by known means, in any suitable formulation, by any suitable route. Suitable routes of administration may include by oral, rectal, nasal, topical (including buccal and sublingual), sublingual, transdermal, intrathecal, transmucosal or parenteral (including subcutaneous, intramuscular, intravenous and intradermal) administration. In some embodiments, the VAP-1 inhibitor and the steroid are administered orally.

Suitable pharmaceutical compositions and dosage forms may be prepared using conventional methods known to those in the field of pharmaceutical formulation and described in the relevant texts and literature, for example, in Remington: The Science and Practice of Pharmacy (Easton, Pa.: Mack Publishing Co., 1995).

It is especially advantageous to formulate combined preparations of the invention in unit dosage form for ease of administration and uniformity of dosage. The term "unit dosage forms" as used herein refers to physically discrete units suited as unitary dosages for the individuals to be treated. That is, the compositions are formulated into discrete dosage units each containing a predetermined, "unit dosage" quantity of an active agent calculated to produce the desired therapeutic effect in association with the required pharmaceutical carrier. The specifications of unit dosage forms of the invention are dependent on the unique characteristics of the active agent to be delivered. Dosages can further be determined by reference to the usual dose and manner of administration of the ingredients. It should be noted that, in some cases, two or more individual dosage units in combination provide a therapeutically effective amount of the active agent, for example, two tablets or capsules taken together may provide a therapeutically effective dosage, such that the unit dosage in each tablet or capsule is approximately 50% of the therapeutically effective amount.

Preparations according to the invention for parenteral administration include sterile aqueous and non-aqueous solutions, suspensions, and emulsions. Injectable aqueous solutions contain the active agent in water-soluble form. Examples of non-aqueous solvents or vehicles include fatty oils, such as olive oil and corn oil, synthetic fatty acid esters, such as ethyl oleate or triglycerides, low molecular weight alcohols such as propylene glycol, synthetic hydrophilic polymers such as polyethylene glycol, liposomes, and the like. Parenteral formulations may also contain adjuvants such as solubilizers, preservatives, wetting agents, emulsifiers, dispersants, and stabilizers, and aqueous suspensions may contain substances that increase the viscosity of the suspension, such as sodium carboxymethyl cellulose, sorbitol, and dextran. Injectable formulations may be rendered sterile by incorporation of a sterilizing agent, filtration through a bacteria-retaining filter, irradiation, or heat. They can also be manufactured using a sterile injectable medium. The active agent may also be in dried, e.g., lyophilized, form that may be rehydrated with a suitable vehicle immediately prior to administration via injection.

In addition to the formulations described previously, the active agent may be formulated as a depot preparation for controlled release of the active agent, preferably sustained release over an extended time period. These sustained release dosage forms are generally administered by implantation (for example, subcutaneously or intramuscularly or by intramuscular injection).

Combined preparations of the invention may be packaged with instructions for administration of the components on the combination. The instructions may be recorded on a suitable recording medium or substrate. For example, the instructions may be printed on a substrate, such as paper or plastic. The instructions may be present as a package insert, in the labeling of the container or components thereof (i.e., associated with the packaging or sub-packaging). In other embodiments, the instructions are present as an electronic storage data file present on a suitable computer readable storage medium, for example, CD-ROM, diskette. Some or all components of the combined preparation may be packaged in suitable packaging to maintain sterility.

### Preparation of (3S)-Tetrahydrofuran-3-yl (4S)-4-isopropyl-1,4,6,7-tetrahydro-5H-imidazo[4,5-c]pyridine-5-carboxylate (Referred to as Compound 1).

The following abbreviations have been used:
- Aq: Aqueous
- DCM: Dichloromethane
- DIPEA: Diisopropylethylamine
- Ee: Enantiomeric excess
- ES+: Electrospray
- EtOAc: Ethyl acetate
- H: Hour(s)
- HPLC: High performance liquid chromatography
- HRMS: High resolution mass spectrometry
- LCMS: Liquid chromatography mass spectrometry
- M: Molar
- MeOH: Methanol
- [MH+]: Protonated molecular ion
- min: Minutes
- RP: Reverse phase
- MS: Mass spectrometry
- RT: Retention time
- sat: Saturated
- THF: Tetrahydrofuran
- TFA: Trifluoroacetic acid

### Experimental Methods

All reagents were commercial grade and were used as received without further purification, unless otherwise specified. Reagent grade solvents were used in all cases. Analytical LCMS was performed on a Waters ZQ mass spectrometer connected to an Agilent 1100 HPLC system. Analytical HPLC was performed on an Agilent 1100 system. High-resolution mass spectra (HRMS) were obtained on an Agilent MSD-TOF connected to an Agilent 1100 HPLC system. During the analyses the calibration was checked by two masses and automatically corrected when needed. Spectra are acquired in positive electrospray mode. The acquired mass range was m/z 100-1100. Profile detection of the mass peaks was used. Flash chromatography was performed on either a CombiFlash Companion system equipped with RediSep silica columns or a Flash Master Personal system equipped with Strata SI-1 silica gigatubes. Reverse Phase HPLC was performed on a Gilson system (Gilson 322 pump with Gilson 321 equilibration pump and Gilson 215 autosampler) equipped with Phenomenex Synergi Hydro RP 150 x 10 mm, YMC ODS-A 100/150 x 20 mm or Chirobiotic T 250 x 10 mm columns. Reverse phase column chromatography was performed on a Gilson system (Gilson 321 pump and Gilson FC204 fraction collector) equipped with Merck LiChroprep® RP-18 (40-63 µm) silica columns. The compounds were automatically named using ACD 6.0. All compounds were dried in a vacuum oven overnight.

Analytical HPLC and LCMS data were obtained with:
System A: Phenomenex Synergi Hydro RP (C18, 30 x 4.6 mm, 4 µm), gradient 5-100% CH₃CN (+0.085% TFA) in water (+0.1% TFA), 1.5 mL/min, with a gradient time of 1.75 min, 200 nm, 30 °C; or System B: Phenomenex Synergi Hydro RP (C18, 150 x 4.6 mm, 4 µm), gradient 5-100% CH₃CN (+0.085% TFA) in water (+0.1 % TFA), 1.5 mL/min with a gradient time of 7 min, 200 nm, 30 °C.

Chiral HPLC data were obtained with:
System C: Chirobiotic V polar ionic mode (150 x 4.6 mm), 70% MeOH in 10 mM aq ammonium formate buffer, 1.0 mL/min, over 10 min, 200 nm, 30 °C.

### INTERMEDIATE 1

### 4-Isopropyl-4,5,6,7-tetrahydro-1H-imidazo[4,5-c]pyridine hydrochloride

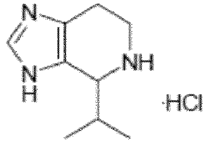

Histamine dihydrochloride (61.9 g, 336 mmol) was dissolved in a solution of NaOH (33.6 g, 841 mmol) in water (125 mL) and MeOH (500 mL), and isobutyraldehyde (61.4 mL, 672 mmol) was added. The reaction mixture was heated under reflux at 80 °C for 24 h, cooled to room temperature, the pH was adjusted to 7 with 1 M aq HCI solution (250 mL) and the solvents were removed *in vacuo.* The residue was dissolved in warm MeOH (300 mL), allowed to stand for 1h, filtered and the solvents were removed *in vacuo.* The residue was stirred in MeOH (50 mL) and acetone (400 mL) for 2 h and was cooled to 4°C for 2 h. The resulting precipitate was filtered and washed with acetone (100 mL) to give 4-isopropyl-4,5,6,7-tetrahydro-1H-imidazo[4,5-c]pyridine hydrochloride (33.0 g, 48.7%) as a white solid. Analytical LCMS: purity >90% (System A, RT = 0.51 min), ES+: 166.4 [MH]+.

### INTERMEDIATE 2

### 4-Nitrophenyl 4-isopropyl-1,4,6,7-tetrahydro-5H-imidazo[4,5-c]pyridine-5-Carboxylate

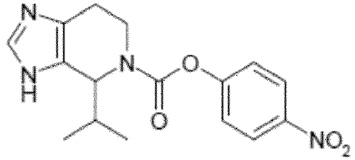

Intermediate 1 (2.78 g, 8.28 mmol, 60% pure) and DIPEA (5.27 mL, 30.3 mmol) were dissolved in DCM (100 mL). The reaction mixture was cooled to 0 °C and 4-nitrophenyl chloroformate (4.07 g, 20.2 mmol) was added. The reaction mixture was stirred at room temperature for 18 h. The reaction mixture was washed with sat aq NaHCO₃ solution (5 x 100 mL), dried (MgSO4) and the solvents were removed *in vacuo* to give 4-nitrophenyl 4-isopropyl-1,4,6,7-tetrahydro-5H-imidazo[4,5-c]pyridine-5-carboxylate (5.28 g, crude) as a yellow gum. Analytical HPLC: purity 41% (System B, RT = 4.70 min); Analytical LCMS: purity 86% (System A, RT = 1.70 min), ES+: 331.0 [MH]+.

### (3S)-Tetrahydrofuran-3-yl (4S)-4-isopropyl-1,4,6,7-tetrahydro-5H-imidazo[4,5-c]-pyridine-5-carboxylate

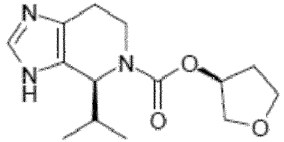

NaH (0.40 g, 10.0 mmol, 60% dispersion in mineral oil) was suspended in anhydrous THF (20 mL), cooled to 0 °C and (S)-3-hydroxytetrahydrofuran (0.88 g, 0.68 mL, 10.0 mmol) was added. The suspension was stirred at 0 °C for 30 min then added to a solution of Intermediate 2 (3.30 g, 10.0 mmol, 70% pure) in THF (60 mL) and the reaction mixture was stirred at room temperature. Two additional such portions of NaH and (S)-3-hydroxytetrahydrofuran in THF were added after 5 and 29 h, respectively. After 2 d the reaction mixture was quenched with water (10 mL) and the solvents were removed *in vacuo.* The residue was dissolved in EtOAc (100 mL), washed with 1 M aq Na₂CO₃ solution (4 x 100 mL), dried (MgSO₄) and the solvents were removed *in vacuo.* The residue was purified by column chromatography (normal phase, 20 g, Strata SI-1, silica gigatube, DCM (200 mL) followed by 2%, 4% and 5% MeOH in DCM (200 mL each)) and reverse phase HPLC (YMC ODS-A 100 x 20 mm, 5 µm, 25 mL/min, gradient 30% to 60% (over 7 min) then 100% (3 min) MeOH in 10% MeOH/water) to give (3S)- tetrahydrofuran-3-yl 4-isopropyl-1,4,6,7-tetrahydro-5H-imidazo[4,5 c]pyridine-5- carboxylate (34.8 mg, 1.1%) as a white solid. Analytical HPLC: purity 100% (System B, RT = 3.63 min); Analytical LCMS: purity 100% (System B, RT = 4.01 min), ES+: 280.1 [MH]+. (3S)-Tetrahydrofuran-3-yl-4-isopropyl-1,4,6,7-tetrahydro-5H-imidazo[4,5-c]pyridine-5- carboxylate (39.91 mg) was dissolved in 10 mM ammonium formate buffer and MeOH (2 mL, 1:1) and purified twice by reverse phase chiral HPLC (Chirobiotic T 250 x 10 mm, 3 mL/min, isocratic run 70% MeOH in 10 mM ammonium formate buffer (40 min), pH 7.4) to give a single diastereoisomer, (3S)-tetrahydrofuran-3-yl (4S)-4-isopropyl-1,4,6,7- tetrahydro-5H-imidazo[4,5-c]pyridine-5-carboxylate (6.90 mg, 99% ee). Analytical HPLC: purity 100% (System B, RT = 3.63 min); Chiral HPLC: purity 99.5% (System C, RT = 2.22 min); Analytical LCMS: purity 100% (System B, RT = 3.90 min), ES+: 280.1 [MH]+ ; HRMS calculated for C₁₄H₂₁N₃O₃: 279.1583, found 279.1571.

### (3S)-Tetrahydrofuran-3-yl (4S)-4-isopropyl-1,4,6,7-tetrahydro-5H-imidazo[4,5-c]-pyridine-5-carboxylate, Methananesulfonic acid salt

(3*S*)-Tetrahydrofuran-3-yl (4*S*)-4-isopropyl-1,4,6,7-tetrahydro-5H-imidazo[4,5-c]pyridine-5-carboxylate free base (460mg, 1.65mmol) was dissolved in EtOAc (10mL) at room temperature to give a clear colourless solution. Methanesulphonic acid (107µL) was added portion-wise with gentle heating. The solution was allowed to cool to room temperature overnight. The resulting crystals were collected by filtration, washed with EtOAc (2 x 10mL) and dried overnight at 40°C *in vacuo.* (3S) Tetrahydrofuran-3-yl (4S)-4- isopropyl-1,4,6,7-tetrahydro-5H-imidazo[4,5-c]pyridine-5-carboxylate mesylate salt was obtained with a 99% yield (615mg) as a white crystalline solid. HPLC: Retention time 2.27min, purity 99.5%. Melting point: 189°C. LCMS: Retention time 4.19min, ES⁺ 280.0 [MH]⁺, 100% purity. Chiral HPLC: Retention time 3.70min, >99.5% de. ¹H NMR (400MHz, CDCl₃): ^{δ}_{H} 8.72 (1H, m, NHCHNH+), 5.29 (1H, m, OCH), 5.05 (0.5H, d, *J* 8.4Hz, CCHN), 4.89 (0.5H, d, *J* 7.6Hz, CCHN), 4.59 (0.5H, m, NCH_{A}CH_{B}), 4.39 (0.5H, m, NCH_{A}CH_{B}), 3.97-3.85 (4H, m, CH₂OCH₂), 3.20 (1H, m, NCH_{A}CH_{B}), 2.89 (3H, s, CH₃SO₃⁻), 2.89-2.72 (2H, m, CCH₂CH₂N), 2.23-2.07 (3H, m, CH(CH₃)₂, OCH₂CH₂), 1.16 (3H, d, *J* 6.4Hz, CH3) and 1.06-0.96 (3H, m, CH₃).

### Preparation of 1-(4-{5-[3-(4-Fluorophenyl)-3H-imidazo[4,5-c]pyridin-2-yl]pyridin-2-yl}piperazin-1-yl)ethan-1-one (Compound 2).

### 1-(4-{5-[3-(4-Fluorophenyl)-3H-imidazo[4,5-c]pyridin-2-yl]pyridin-2-yl}piperazin-1-yl)ethan-1-one has the following structure:

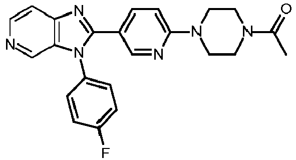

This compound is Example 86 of published patent application WO 2014/140592, the synthesis of which compound is described in detail therein.

### Preparation of 1-{5-[3-(4-Fluorophenyl)-3H-imidazo[4,5-c]pyridin-2-yl]pyridin-2-yl}-4-methanesulfonylpiperazine (Compound 3).

### 1-{5-[3-(4-Fluorophenyl)-3H-imidazo[4,5-c]pyridin-2-yl]pyridin-2-yl}-4-methanesulfonylpiperazine has the following structure:

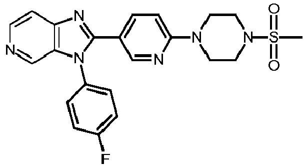

This compound is Example 89 of published patent application WO 2014/140592, the synthesis of which compound is described in detail therein.

### Preparation of 4-{5-[3-(4-Fluorophenyl)-3H-imidazo[4,5-c]pyridin-2-yl]pyridin-2-yl}morpholine (Compound 4).

### 4-{5-[3-(4-Fluorophenyl)-3H-imidazo[4,5-c]pyridin-2-yl]pyridin-2-yl}morpholine has the following structure:

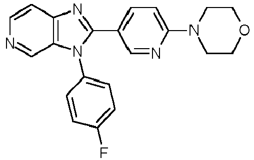

This compound is Example 54 of published patent application WO 2014/140592, the synthesis of which compound is described in detail therein.

### BIOLOGICAL DATA

### EXAMPLE 1

### Evaluation of (3S)-Tetrahydrofuran-3-yl(4S)-4-isopropyl-1,4,6,7-tetrahydro-5H-imidazo[4,5-c]pyridine-5-carboxylate mesylate salt (Compound 1) on CFA (Complete Freunds Adjuvant) induced hypersensitivity in rat

(3S)-Tetrahydrofuran-3-yl (4*S*)-4-isopropyl-1,4,6,7-tetrahydro-5H-imidazo[4,5-c]pyridine-5-carboxylate was investigated (Figure 1) in the CFA thermal hyperalgesia model, which is an established model for inflammatory pain. (4*S*)-4-isopropyl-1,4,6,7-tetrahydro-5H-imidazo[4,5-c]pyridine-5-carboxylate was found to be effective in a dose-dependent manner, and comparably in efficacy to the gold standard benchmark indomethacin. In more detail:

Assessment of the anti-hyperalgesic properties of *(3S)*-tetrahydrofuran-3-yl (4S)-4-isopropyl-1,4,6,7-tetrahydro-5H-imidazo[4,5-c]pyridine-5-carboxylate mesylate salt was determined through measurement of weight bearing following CFA induced hypersensitivity. Naive rats distribute their body weight equally between the two hind paws. However, when the injected (left) hind paw is painful, the weight is re-distributed so that less weight is put on the affected paw (decrease in weight bearing on injured paw). Weight bearing through each hind limb was measured using a rat incapacitance tester (Linton Instruments, UK). Rats were placed in the incapacitance tester with the hind paws on separate sensors and the average force exerted by both hind limbs was recorded over 4 seconds. The injection of CFA also induces an oedema that can be assessed by paw volume; this is measured using a plethysmometer. The rat's hind paw is placed into the cylinder containing a solution and the volume of displaced liquid determines the paw volume.

Naive male, Sprague Dawley rats were acclimatised with food and water available ad libitum. Habituation to the incapacitance tester was performed. Baseline weight bearing and paw volume recordings were taken prior to induction of insult. Inflammatory hypersensitivity was induced by intraplantar injection of CFA (100µl of 1mg/ml solution) into the left hind paw. A pre-treatment weight bearing and paw volume measurement was taken to assess hypersensitivity 23 hours post-CFA. Animals were then ranked and randomised according to CFA window in a Latin square design. *(3S)-*Tetrahydrofuran-3-yl (4S)-4-isopropyl-1,4,6,7- tetrahydro-5H-imidazo[4,5-c]pyridine-5-carboxylate mesylate salt was then given at 150, 250 and 500mg/kg p.o. (dosed in water at 2mL/Kg, pH ∼5-6), alongside vehicle and reference group (indomethacin), n=9-10 per group. Weight bearing was assessed in all groups 1, 2 and 4 hours post compound administration. Paw volume was assessed 4 hours post compound administration. Data was analysed by comparing treatment groups to control group at each time point. Weight bearing (g) readings were taken for both right and left hind paws and the difference calculated. Data is expressed as % reversal of the hypersensitivity to pain, (post dose reading - pre dose reading)/ (naive reading - pre dose reading) x 100, where naive weight bearing difference - pre dose weight bearing difference is defined as the CFA window to be reversed. Statistical analysis was conducted by means of repeated measures ANOVA followed by Planned comparison test using InVivoStat (invivostat.co.uk), (p<0.05 considered significant).
(3S) Tetrahydrofuran-3-yl (4S)-4-isopropyl-1,4,6,7-tetrahydro-5H-imidazo[4,5-c]pyridine-5- carboxylate mesylate salt at 150mg/kg showed no significant reversal of hypersensitivity at any time point, 250mg/kg showed a significant reversal at 4 hours, however 500mg/kg was effective at all time points with maximum effect seen at 4 hours post dose. No effect was seen on paw volume with *(3S)-*tetrahydrofuran-3-yl (4S)-4-isopropyl-1,4,6,7-tetrahydro-5Himidazo[4,5-c]pyridine-5-carboxylate mesylate salt.

% Reversal of established CFA-induced hypersensitivity is shown in Figure 1.

### EXAMPLE 2

### Effect of LJP1207 on CFA (Complete Freunds Adjuvant) model

LJP1207 [N'-(2-phenyl-allyl)-hydrazine hydrochloride] is a potent (human SSAO, IC(50) = 17 nM), selective, and orally available SSAO inhibitor that blocks both the enzymatic and adhesion functions of SSAO/VAP-1 (Salter-Cid et al., J Pharmacol Exp Ther. 2005 Nov;315(2):553-62).

### Adjuvant-induced arthritis model

Rats injected into the hindpaw with a mixture of *Mycobacterium butirricum* emulsified in light mineral oil develop a severe polyarthritis which shares some features in common with human rheumatoid arthritis (RA), such as swelling of the extremities, cartilage degradation, loss of joint function and lymphocyte infiltration into diseased joints. This model was originally described more than 60 years ago (Pearson et al., Arthritis and Rheumatology, 1959, 2(5), 440-459) and is still the most widely used assay to identify chemical agents having a potential therapeutic efficacy in RA. Furthermore, this model allows evaluation of analgesic activity of anti-rheumatic drugs in pathologically induced pain. Furthermore, this is a well-established model for inflammatory pain, especially longer-term (i.e. chronic) inflammatory pain.

### Experimental details

Male Lewis rats (220-230g) were housed in propylene cages with food and water *ad libitum.* The light cycle was automatically controlled and the room temperature thermostatically regulated to 21 ± 1°C. Prior to the start of the experiment animals were housed in these conditions for 6-8 days.

Rats were anaesthetized before induction of CFA and then were treated orally starting from day 0 to day 28 with LJP1207 (30mg/kg po qd) or methotrexate (MTX, 0.25mg/kg ip 3 times per week). Control rats received the vehicle e.g. PBS starting from day 0. Arthritis was induced by injecting in the tail base 100µl of 6mg/ml of *Mycobacterium butirricum* suspended in complete adjuvant. The development of arthritis was assessed at day 7, 14, 18, 21, 25 and 28.

Randall Selitto assay was performed on both hindpaws at day 7, 14, 18, 21, 25 and 28. Briefly, pressure was applied through a tip to the plantar surface of the hindpaw at a constant rate using an analgesiometer (Ugo Basile, Comerio, Italy) to the point at which the animal struggled, squealed or attempted to bite. The test was run by an observer unaware of the treatment. The force (expressed in grams) at which the animal began to struggle was assumed to represent the nociceptive threshold and served as the end point.

All data are presented as the mean ± SEM. Statistical analysis was performed by two-way ANOVA test for multiple comparisons followed by Bonferroni's test. Statistical significance was set at p<0.05 compared to vehicle control (*P<0.05, **P<0.01, ***P<0.001).

### Results

The Randal Selitto assay is designed to measure pain perception given by gradual increase of weight on the paw. It is a measure of mechanical hyperalgesia. The results in Figure 2 show that treatment with LJP1207 caused a significant inhibition of paw withdrawal. In particular, LJP1207 gave a significant inhibition at day 21, 25 and 28. MTX gave a significant inhibition starting at day 18 up to day 28.

### EXAMPLE 3

### Effect of (S)-carbidopa, both alone and in combination with prednisolone on CFA (Complete Freunds Adjuvant) induced hypersensitivity in rat

Assessment of the anti-hyperalgesic properties of (S)-Carbidopa was determined through measurement of weight bearing following CFA induced hypersensitivity. Naive rats distribute their body weight equally between the two hind paws. However, when the injected (left) hind paw is painful, the weight is re-distributed so that less weight is put on the affected paw (decrease in weight bearing on injured paw). Weight bearing through each hind limb was measured using a rat incapacitance tester (Linton Instruments, UK). Rats were placed in the incapacitance tester with the hind paws on separate sensors and the average force exerted by both hind limbs was recorded over 4 seconds. The injection of CFA also induces an oedema that can be assessed by paw volume; this is measured using a plethysmometer. The rat's hind paw is placed into the cylinder containing a solution and the volume of displaced liquid determines the paw volume.

Naive male, Sprague Dawley rats were acclimatised with food and water available ad libitum. Habituation to the incapacitance tester was performed. Baseline weight bearing and paw volume recordings were taken prior to induction of insult. Inflammatory hypersensitivity was induced by intraplantar injection of CFA (100µl of 1mg/ml solution) into the left hind paw. A pre-treatment weight bearing and paw volume measurement was taken to assess hypersensitivity 23 hours post-CFA. Animals were then ranked and randomised according to CFA window in a Latin square design.

In Part A, animals were treated with either Vehicle (1% Methylcellulose (MC) in water), Prednisolone 0.3, 1, 3 & 10mg/kg, or Indomethacin 10mg/kg (5mL/kg dose volume) 24 hours post CFA. Weight bearing was measured at 1 and 3 hours post treatment.

In Part B, animals were treated with either Vehicle (5% DMSO, 0.5% Hydroxypropyl methylcellulose (HPMC) in water), (S)-carbidopa 3, 10, 30 & 100mg/kg, or Indomethacin 10mg/kg (10mL/kg dose volume) 24 hours post CFA. Weight bearing was measured at 1 and 3 hours post treatment and oedema was measured 3 hours post treatment.

In Part C, animals were treated with either Vehicle (5% DMSO 0.5% HPMC) or (S)-carbidopa, 3, 10mg/kg and then with Vehicle (1%MC) or Prednisolone 0.3mg/kg (5mL/kg dose volume for each treatment) 24 hours post CFA. Weight bearing was measured at 1 and 3 hours post treatment.

Data were analysed by comparing treatment groups to the vehicle control group at each time point.

Weight bearing (g) readings were taken for both right and left hind paws and the difference calculated. Data is expressed as % reversal of the hypersensitivity to pain. Paw Volume (mL) readings were taken for the left hind paws. Data are expressed as % reversal of the oedema. Calculation: (post dose reading - pre dose reading)/ (naive reading - pre dose reading) x 100, where naive weight bearing difference - pre dose weight bearing difference is defined as the CFA window to be reversed. Statistical analysis was conducted by means of repeated measures ANOVA followed by Planned comparison test using InVivoStat (invivostat.co.uk), (p<0.05 considered significant).

### RESULTS

Intraplantar injection of CFA induced hypersensitivity as detected by a shift in weight bearing between injured and non-injured hind paws 24 hours post dose. CFA also induced a marked oedema in the injected paw in both studies. In line with previous studies, indomethacin (10mg/kg) produced a marked reversal of the hypersensitivity measured using weight bearing.
Part A: Prednisolone (0.3-10mg/kg) alone dose-dependently inhibited the hypersensitivity response (see Figure 3).
Part B: (S)-Carbidopa (3-100mg/kg) alone dose-dependently inhibited the hypersensitivity response (see Figure 4) but had no effect on oedema (See Figure 5)
Part C: Minimally/moderately effective doses of (S)-Carbidopa (3 & 10mg/kg) and prednisolone (0.3mg/kg) were selected to be administered in combination in order to evaluate potential synergistic effects.

Co-dosing prednisolone (0.3mg/kg) with (S)-carbidopa had the same analgesic effect as 10mg/kg prednisolone alone, suggesting that steroid dosing can be reduced by more than 10-fold when co-dosed with (S)-carbidopa (see Figure 6).

The results also show evidence of synergy between prednisolone and (S)-carbidopa (see Figure 6). Synergy can be calculated according to the methods taught in references [1] and [2]:
[1] Webb JL, Effect of more than one inhibitor. Enzyme and metabolic inhibitors. 1. New York: Academic Press; 1963, p. 66-79 (488-512)
[2] Greco WR, Bravo G, and Parsons JC (1995) The search for synergy: a critical review from a response surface perspective. Pharmacol Rev 47: 331-385.

### EXAMPLE 4

### Effect of 1-(4-{5-[3-(4-Fluorophenyl)-3H-imidazo[4,5-c]pyridin-2-yl]pyridin-2-yl}piperazin-1-yl)ethan-1-one (Compound 2) on CFA (Complete Freunds Adjuvant) induced hyper-sensitivity in rat

Assessment of the anti-hyperalgesic properties of 1-(4-{5-[3-(4-Fluorophenyl)-3H-imidazo[4,5-c]pyridin-2-yl]pyridin-2-yl}piperazin-1-yl)ethan-1-one was determined by the method described in Example 3. Animals were treated orally with either Vehicle (30% aqueous hydroxypropyl-beta-cyclodextrin), 1-(4-{5-[3-(4-Fluorophenyl)-3H-imidazo[4,5-c]pyridin-2-yl]pyridin-2-yl}piperazin-1-yl)ethan-1-one 1, 3 and 10mg/kg, or Indomethacin 10mg/kg (3mL/kg dose volume) 24 hours post CFA. Weight bearing was measured at 1 and 4 hours post treatment.

### RESULTS

1-(4-{5-[3-(4-Fluorophenyl)-3H-imidazo[4,5-c]pyridin-2-yl]pyridin-2-yl}piperazin-1yl)ethan-1-one dose-dependently inhibited the hypersensitivity response with the 10mg/kg dose significantly inhibiting the hyperactivity response at both 1 and 4 hours post administration (see Figure 7).

### EXAMPLE 5

### Effect of 1-{5-[3-(4-Fluorophenyl)-3H-imidazo[4,5-c]pyridin-2-yl]pyridin-2-yl}-4-methanesulfonylpiperazine (Compound 3) on CFA (Complete Freunds Adjuvant) induced hyper-sensitivity in rat

Assessment of the anti-hyperalgesic properties of 1-{5-[3-(4-Fluorophenyl)-3H-imidazo[4,5-c]pyridin-2-yl]pyridin-2-yl}-4-methanesulfonylpiperazine was determined by the method described in Example 3. Animals were treated orally with either Vehicle (30% aqueous hydroxypropyl-beta-cyclodextrin), 1-{5-[3-(4-Fluorophenyl)-3H-imidazo[4,5-c]pyridin-2-yl]pyridin-2-yl}-4-methanesulfonylpiperazine 1, 3 and 10mg/kg, or Indomethacin 10mg/kg (3mL/kg dose volume) 24 hours post CFA. Weight bearing was measured at 1 and 4 hours post treatment.

### RESULTS

1-{5-[3-(4-Fluorophenyl)-3H-imidazo[4,5-c]pyridin-2-yl]pyridin-2-yl}-4 methanesulfonylpiperazine dose-dependently inhibited the hypersensitivity response with the 10mg/kg dose significantly inhibiting the hyperactivity response at both 1 and 4 hours post administration, and the lower dose of 3mg/kg showing a significant reduction at 4 hours post administration (see Figure 8).

### EXAMPLE 6

### Effect of 4-{5-[3-(4-Fluorophenyl)-3H-imidazo[4,5-c]pyridin-2-yl]pyridin-2-yl]morpholine (Compound 4) on CCI (chronic constriction injury) induced neuropathic pain in rat

The Chronic Constriction Injury (CCI) model of neuropathic pain involves unilateral loose ligation of four ligatures around the left sciatic nerve at mid-thigh level spaced 1mm apart. This procedure results in the development of hyperalgesia, allodynia and spontaneous pain (ectopic discharges) which can be measured using mechanical and thermal behavioural assessments. As such, this model is believed to mimic some of the symptoms and aetiology of neuropathic pain observed in the clinic (Bennett GJ and Xie YK.,1988; Field *et al.,* 1999).

Naive male Sprague Dawley rats weighing 200-250g were acclimatised to the procedure room in their home cages, with food and water available *ad libitum.* All animals underwent behavioural testing of mechanical allodynia prior to surgery in order to determine the baseline withdrawal thresholds. The average of the last two (out of three) baseline paw-withdrawal thresholds to stimulation with von-Frey hairs was taken as the baseline.

Under Isoflurane anaesthesia mixed with oxygen (3:1, 1L/min) the left hind leg was shaved mid-thigh level and an incision made through the skin using a scalpel. The biceps femoris muscle layer was dissected by making an initial incision using a pair of sharp scissors, which was then widened using a pair of blunt scissors. The common sciatic nerve was exposed using a pair of forceps and 4 loose ligatures of chromic gut (SMI) were tied around the sciatic nerve with 1mm spacing between each. The nerve was then returned below the muscle layer and the wound closed using absorbable sutures (Vicryl).

Behavioural testing started 19 days post-surgery with mechanical readings taken on day 19, and day 22. Animals were then ranked and randomised (based on a Latin square design) to treatment groups (n=10-11 per group) according to the percentage change (compared to pre-surgery baseline) of the mean mechanical withdrawal threshold observed on days 19 and 22.

On day 23, animals were treated orally with either Vehicle (30% aqueous hydroxypropyl-beta-cyclodextrin), 4-{5-[3-(4-Fluorophenyl)-3H-imidazo[4,5-c]pyridin-2-yl]pyridin-2-yl}morpholine 15, 50 and 150mg/kg (5mL/kg dose volume), or Pregabalin 30mg/kg (2mL/kg dose volume in water). Paw withdrawal thresholds (PWT) from each of the animals (mechanical von-Frey, mvF) were evaluated at 1 and 3 hours post dosing.

*Static mechanical (tactile) allodynia:* Measurement of withdrawal threshold was achieved using calibrated (force; g) von-Frey monofilaments (Touch-Test Sensory Evaluator; Scientific Marketing Associates) applied to the plantar surface of the hindpaw. Withdrawal threshold was determined by increasing and decreasing stimulus intensity, and estimated using the Dixon's up-down method (Dixon, 1980; Chaplan *et al.,* 1994).

The animals were placed on an elevated mesh bottom platform with a 0.5 cm² grid to provide access to the ventral side of the hind paws. An inverted plexiglass container was placed on top of each rat and testing was performed after an initial 15-20minute acclimatisation/habituation period. The von-Frey filaments were placed perpendicular to the plantar surface of the ipsilateral hindpaw, from below the mesh floor. The monofilaments were held at the position for approximately 8s with enough force to cause a slight bend of the filament. Only immediate sharp withdrawal responses from the stimulus (or flinching) were considered to represent a positive response.

Mechanical allodynia (von Frey) data were analysed using two-way repeated measures ANOVA with 'treatment' as a between subjects effect and 'day' as a within subjects effect. Post-hoc analysis using planned pair-wise comparisons using 2-way repeated measures ANOVA (Clark *et al.,* 2012 InVivostat).

### RESULTS

Ligation of the sciatic nerve resulted in the development of a stable and robust neuropathic pain as measured by a reduction in the von-Frey mechanical threshold. The gold-standard, Pregabalin (30mg/kg p.o.), increased the withdrawal threshold to a degree that was comparable with those animals that had undergone sham surgery. 4-{5-[3-(4-Fluorophenyl)-3H-imidazo[4,5-c]pyridin-2-yl]pyridin-2-yl}morpholine (15, 50 and 150mg/kg p.o.) tested at 1 and 3 hours post systemic dosing, increased withdrawal threshold in a dose and temporally dependent fashion, with the dose of 150mg/kg reaching statistical significance vs the vehicle group at 1h and 3h and the dose of 50mg/kg reaching statistical significance at 3h (see Figure 9).

Dixon WJ. Efficient analysis of experimental observations. Ann Rev Pharmacol Toxicol. 1980 20, 441-62.
Bennett GJ, Xie YK. A peripheral mononeuropathy in rat that produces disorders of pain sensation like those seen in man. Pain. 1988 33(1):87-107
Field MJ, Bramwell S, Hughes J, Singh L. Detection of static and dynamic components of mechanical allodynia in rat models of neuropathic pain: are they signalled by distinct primary sensory neurones? Pain. 1999 Nov;83(2):303-11.
Chaplan SR, Bach FW, Pogrel JW, Chung JM, Yaksh TL. Quantitative assessment of tactile allodynia in the rat paw. J Neurosci Methods. 1994 Jul;53(1):55-63.
Clark RA, Shoaib M, Hewitt KN, Stanford SC, Bate ST. A comparison of InVivoStat with other statistical software packages for analysis of data generated from animal experiments. J Psychopharmacology 2012 26(8) 1136-1142.

### VAP-1 Inhibition assay

(3S)-Tetrahydrofuran-3-yl (4S)-4-isopropyl-1,4,6,7-tetrahydro-5H-imidazo[4,5-c]pyridine-5-carboxylate mesylate salt (Compound 1), LJP1207, (S)-carbidopa, 1-(4-{5-[3-(4-Fluorophenyl)-3H-imidazo[4,5-c]pyridin-2-yl]pyridin-2-yl}piperazin-1-yl)ethan-1-one (Compound 2), 1-{5-[3-(4-Fluorophenyl)-3H-imidazo[4,5-c]pyridin-2-yl]pyridin-2-yl}-4-methanesulfonylpiperazine (Compound 3), and
4-{5-[3-(4-Fluorophenyl)-3H-imidazo[4,5-c]pyridin-2-yl]pyridin-2-yl}morpholine (Compound 4) are inhibitors of VAP-1 (see Table 1).

This assay is performed at room temperature with purified recombinantly expressed human VAP-1 (SSAO). Enzyme was prepared essentially as described in Ohman et al. (Protein Expression and Purification 46 (2006) 321-331). The enzyme activity is assayed with benzylamine as substrate by measuring either benzaldehyde production, using 14C-labeled substrate, or by utilizing the production of hydrogen peroxide in a horseradish peroxidise (HRP) coupled reaction. Briefly, test compounds are dissolved in dimethyl sulfoxide (DMSO) to a concentration of 10 mM. Dose-response measurements are assayed by either creating 1:10 serial dilutions in DMSO to produce a 7 point curve or by making 1:3 serial dilutions in DMSO to produce 11 point curves. The top concentrations are adjusted depending on the potency of the compounds and subsequent dilution in reaction buffer yielded a final DMSO concentration ≤ 2%.

Hydrogen peroxide detection: In a horseradish peroxidise (HRP) coupled reaction, hydrogen peroxide oxidation of 10- acetyl-3,7-dihydroxyphenoxazine produces resorufin, which is a highly fluorescent compound (Zhout and Panchuk-Voloshina. Analytical Biochemistry 253 (1997) 169-174; AmplexR Red Hydrogen Peroxide/peroxidise Assay kit, Invitrogen A22188). Enzyme and compounds in 50 mM sodium phosphate, pH 7.4 are set to pre-incubate in flat-bottomed microtiter plates for approximately 15 minutes before initiating the reaction by addition of a mixture of HRP, benzylamine and Amplex reagent. Benzylamine concentration is fixed at a concentration corresponding to the Michaelis constant, determined using standard procedures. Fluorescence intensity is then measured at several time points during 1 - 2 hours, exciting at 544 nm and reading the emission at 590 nm. For the human SSAO assay final concentrations of the reagents in the assay wells are: SSAO enzyme 1 mg/ml, benzylamine 100 µM, Amplex reagent 20 µM, HRP 0.1 U/mL and varying concentrations of test compound. The inhibition is measured as % decrease of the signal compared to a control without inhibitor (only diluted DMSO). The background signal from a sample containing no SSAO enzyme is subtracted from all data points. Data is fitted to a four parameter logistic model and IC50 values are calculated, for example by using the GraphPad Prism 4 or XLfit 4 programs.

**Table 1**

| **Compound** | **Human VAP-1 IC50** |
|---|---|
| Compound 1 | 37nM |
| LJP1207 | 34nM |
| (S)-Carbidopa | 142nM |
| 1-(4-{5-[3-(4-Fluorophenyl)-3H-imidazo[4,5-c]pyridin-2-yl]pyridin-2-yl}piperazin-1-yl)ethan-1-one | 31 nM |
| 1-{5-[3-(4-Fluorophenyl)-3H-imidazo[4,5-c]pyridin-2-yl]pyridin-2-yl}-4-methanesulfonylpiperazine | 4nM |
| 4-{5-[3-(4-Fluorophenyl)-3H-imidazo[4,5-c]pyridin-2-yl]pyridin-2-yl}morpholine | 13nm |

The following are specific numbered embodiments of the invention.
Embodiment 1. A VAP-1 inhibitor for use in, or for use in the manufacture of a medicament for, the treatment of pain.
Embodiment 2. A VAP-1 inhibitor for use in, or for use in the manufacture of a medicament for, the treatment of pain,
   PROVIDED THAT
   the VAP-1 inhibitor is other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof.
Embodiment 3. A method for the treatment of pain, which comprises administering to a subject suffering from pain an effective amount of a VAP-1 inhibitor.
Embodiment 4. A method for the treatment of pain, which comprises administering to a subject suffering from pain an effective amount of a VAP-1 inhibitor,
   PROVIDED THAT
   the VAP-1 inhibitor is other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof.
Embodiment 5. A pharmaceutical composition for use in the treatment of pain, which comprises: a VAP-1 inhibitor; and a pharmaceutically acceptable carrier, excipient, or diluent.
Embodiment 6. A pharmaceutical composition for use in the treatment of pain, which comprises: a VAP-1 inhibitor other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof; and a pharmaceutically acceptable carrier, excipient, or diluent.
Embodiment 7. A VAP-1 inhibitor for use according to embodiment 1 or 2, or a method according to embodiment 3 or 4, or a pharmaceutical composition for use according to embodiment 5 or 6, wherein the VAP-1 inhibitor has the structure of any one of the specific Examples of VAP-1 inhibitor compounds, polypeptides or proteins disclosed herein.
Embodiment 8. A VAP-1 inhibitor for use according to embodiment 1 or 2, or a method according to embodiment 3 or 4, or a pharmaceutical composition for use according to embodiment 5 or 6, wherein the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (S)-carbidopa, benserazide, LJP1207, BTT1023, RTU-1096, PXS4728 and ASP8232 or a hydrate or pharmaceutically acceptable salt thereof.
Embodiment 9. A VAP-1 inhibitor for use according to embodiment 1 or 2, or a method according to embodiment 3 or 4, or a pharmaceutical composition for use according to embodiment 5 or 6, wherein the VAP-1 inhibitor is (S)-carbidopa, or a hydrate or a pharmaceutically acceptable salt thereof.
Embodiment 10. A VAP-1 inhibitor for use according to embodiment 1 or 2, or a pharmaceutical composition for use according to embodiment 5 or 6, or any one of embodiments 7 to 9, wherein the pain is inflammatory pain.
Embodiment 11. A VAP-1 inhibitor for use according to embodiment 1 or 2, or a pharmaceutical composition for use according to embodiment 5 or 6, or any one of embodiments 7 to 9, wherein the pain is neuropathic pain.
Embodiment 12. A method according to embodiment 3 or 4, or any one of embodiments 7 to 9, wherein the pain is inflammatory pain.
Embodiment 13. A method according to embodiment 3 or 4, or any one of embodiments 7 to 9, wherein the pain is neuropathic pain.
Embodiment 14. A combined preparation for use in, or for use in the manufacture of a medicament for, the treatment of pain, which comprises: a VAP-1 inhibitor and a steroid.
Embodiment 15. A method for the treatment of pain, which comprises administering to a subject suffering from pain an effective amount of a VAP-1 inhibitor and an effective amount of a steroid.
Embodiment 16. A pharmaceutical composition for use in the treatment of pain, which comprises: a VAP-1 inhibitor; a steroid; and a pharmaceutically acceptable carrier, excipient, or diluent.
Embodiment 17. A combined preparation for use according to embodiment 14, or a method according to embodiment 15, or a pharmaceutical for use composition according to embodiment 16, wherein the VAP-1 inhibitor has the structure of any one of the specific Examples of VAP-1 inhibitor compounds, polypeptides or proteins disclosed herein.
Embodiment 18. A combined preparation for use according to embodiment 14, or a method according to embodiment 15, or a pharmaceutical composition for use according to embodiment 16, wherein the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (*S*)-carbidopa, benserazide, LJP1207, BTT1023, RTU-1096, PXS4728 and ASP8232, or a hydrate or a pharmaceutically acceptable salt thereof, and combinations thereof.
Embodiment 19. A combined preparation for use according to embodiment 14, or a method according to embodiment 15, or a pharmaceutical composition for use according to embodiment 16, wherein the VAP-1 inhibitor is (*S*)-carbidopa, or a hydrate or a pharmaceutically acceptable salt thereof.
Embodiment 20. A combined preparation for use according to embodiment 14, or a method according to embodiment 15, or a pharmaceutical composition for use according to embodiment 16, wherein the pain is inflammatory pain.
Embodiment 21. A combined preparation for use according to embodiment 14, or a method according to embodiment 15, or a pharmaceutical composition for use according to embodiment 16, wherein the pain is neuropathic pain.
Embodiment 22. A combined preparation for use according to embodiment 14, or a method according to embodiment 15, or a pharmaceutical composition for use according to embodiment 16, wherein the steroid is a glucocorticoid.
Embodiment 23. A combined preparation for use according to embodiment 14, or a method according to embodiment 15, or a pharmaceutical composition for use according to embodiment 16, wherein the steroid is selected from any one of prednisone, prednisolone, methyl prednisolone, triamcinolone, dexamethasone, hydrocortisone, deflazacort, betamethasone and budenoside, and combinations thereof.
Embodiment 24. A combined preparation, which comprises: (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof; and a steroid.
Embodiment 25. A method of treating pain comprising administering to a subject suffering from pain an effective amount of (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof, and an effective amount of a steroid.
Embodiment 26. A pharmaceutical composition, which comprises: (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof; a steroid; and a pharmaceutically acceptable carrier, excipient, or diluent.
Embodiment 27. Use of a combined preparation according to embodiment 24, or a pharmaceutical composition according to embodiment 26, in the manufacture of a medicament for the treatment of pain.
Embodiment 28. A combined preparation according to embodiment 24, or a pharmaceutical composition according to embodiment 26, for use as a medicament.
Embodiment 29. A combined preparation according to embodiment 24, or a pharmaceutical composition according to embodiment 26, for use in the treatment of pain.
Embodiment 30. A combined preparation according to any one of embodiments 24, 28, and 29 or a pharmaceutical composition according to any of embodiments 26 to 29, or a use according to embodiment 27, or method according to embodiment 25, wherein the steroid is a glucocorticoid.
Embodiment 31. A combined preparation, pharmaceutical composition, use, or method according to embodiment 30, wherein the steroid is selected from any one of prednisone, prednisolone, methyl prednisolone, triamcinolone, dexamethasone, hydrocortisone, deflazacort, betamethasone and budenoside.
Embodiment 32. A combined preparation, pharmaceutical composition, use, or method according to any preceding embodiment, wherein the pharmaceutically acceptable salt is the mesylate salt.
Embodiment 33. A combined preparation, pharmaceutical composition, use, or method according to any preceding embodiment, wherein the pharmaceutically acceptable salt is the sulphate salt, or a hydrate thereof.
Embodiment 34. A method of treatment according to any preceding embodiment, wherein the treatment is treatment in a human subject.

## Claims

1. A VAP-1 inhibitor for use in the treatment of pain,
PROVIDED THAT
the VAP-1 inhibitor is other than (3S)-Tetrahydrofuran-3-yl (4*S*)-4-isopropyl-1,4,6,7-tetrahydro-5H-imidazo[4,5-c]pyridine-5-carboxylate or a hydrate or a pharmaceutically acceptable salt thereof.

2. A pharmaceutical composition for use in the treatment of pain, which comprises: a VAP-1 inhibitor other than (3S)-Tetrahydrofuran-3-yl (4*S*)-4-isopropyl-1,4,6,7-tetrahydro-5H-imidazo[4,5-c]-pyridine-5-carboxylate or a hydrate or a pharmaceutically acceptable salt thereof; and a pharmaceutically acceptable carrier, excipient, or diluent.

3. The VAP-1 inhibitor for use according to claim 1, or the pharmaceutical composition for use according to claim 2, wherein the VAP-1 inhibitor has the structure of any one of the specific Examples of VAP-1 inhibitor compounds, polypeptides or proteins disclosed herein.

4. The VAP-1 inhibitor for use according to claim 1, or the pharmaceutical composition for use according to claim 2, wherein the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (S)-carbidopa, benserazide, LJP1207, BTT1023, RTU-1096, PXS4728 and ASP8232 or a hydrate or pharmaceutically acceptable salt thereof.

5. The VAP-1 inhibitor for use according to claim 1, or the pharmaceutical composition for use according to claim 2, wherein the VAP-1 inhibitor is (S)-carbidopa, or a hydrate or a pharmaceutically acceptable salt thereof.

6. The VAP-1 inhibitor for use according to claim 1, or the pharmaceutical composition for use according to claim 2, or any one of claims 3 to 5, wherein the pain is
(a) inflammatory pain; or
(b) neuropathic pain.

7. A combined preparation for use in the treatment of pain, which comprises: a VAP-1 inhibitor and a steroid,
wherein the VAP-1 inhibitor is a VAP-1 inhibitor other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof.

8. A pharmaceutical composition for use in the treatment of pain, which comprises: a VAP-1 inhibitor; a steroid; and a pharmaceutically acceptable carrier, excipient, or diluent,
wherein the VAP-1 inhibitor is a VAP-1 inhibitor other than (3S)-Tetrahydrofuran-3-yl (4S)-4-isopropyl-1,4,6,7-tetrahydro-5H-imidazo[4,5-c]pyridine-5-carboxylate or a hydrate or a pharmaceutically acceptable salt thereof.

9. The combined preparation for use according to claim 7, or the pharmaceutical composition for use according to claim 8, wherein the VAP-1 inhibitor has the structure of any one of the specific Examples of VAP-1 inhibitor compounds, polypeptides or proteins disclosed herein.

10. The combined preparation for use according to claim 7, or a pharmaceutical composition for use according to claim 8, wherein the VAP-1 inhibitor is a compound selected from 1-(4-{5-[3-(4-Fluorophenyl)-3H-imidazo[4,5-c]pyridin-2-yl]pyridin-2-yl}piperazin-1-yl)ethan-1-one, 1-{5-[3-(4-Fluorophenyl)-3H-imidazo[4,5-c]pyridin-2-yl]pyridin-2-yl}-4-methanesulfonylpiperazine, 4-{5-[3-(4-Fluorophenyl)-3H-imidazo[4,5-c]pyridin-2-yl]pyridin-2-yl}morpholine, (S)-carbidopa, benserazide, LJP1207, BTT1023, RTU-1096, PXS4728 and ASP8232, or a hydrate or a pharmaceutically acceptable salt thereof, and combinations thereof.

11. The combined preparation for use according to claim 7, or the pharmaceutical composition for use according to claim 8, wherein the VAP-1 inhibitor is (S)-carbidopa, or a hydrate or a pharmaceutically acceptable salt thereof.

12. The combined preparation for use according to claim 7, or the pharmaceutical composition for use according to claim 8, wherein the pain is
(a) inflammatory pain; or
(b) neuropathic pain.

13. The combined preparation for use according to claim 7, or the pharmaceutical composition for use according to claim 8, wherein the steroid is a glucocorticoid.

14. The combined preparation for use according to claim 7, or the pharmaceutical composition for use according to claim 8, wherein the steroid is selected from any one of prednisone, prednisolone, methyl prednisolone, triamcinolone, dexamethasone, hydrocortisone, deflazacort, betamethasone and budenoside, and combinations thereof.
